# FASCICULE DE BREVET EUROPEEN

(11) **EP 1 858 898 B1**
(45) Date de publication et mention de la délivrance du brevet: **26.08.2009**
(21) Numéro de dépôt: 06726018.2
(22) Date de dépôt: 03.03.2006
(51) Int. Cl.: C07D 495/04, A61K 31/4162, A61P 35/00

(54) **HYDRAZINOCARBONYL-THIENO[2,3-C]PYRAZOLES, PROCEDE DE PREPARATION, COMPOSITIONS LES CONTENANT ET UTILISATION**
HYDRAZINOCARBONYLTHIENO[2,3-C]PYRAZOLE, HERSTELLUNGSVERFAHREN, ZUSAMMENSETZUNGEN DIE SIE ENTHALTEN, UND ANWENDUNG
HYDRAZINOCARBONYL-THIENO[2,3-C]PYRAZOLES, PREPARATION METHOD, COMPOSITIONS CONTAINING SAME AND USE

(30) Priorité: 04.03.2005 FR 0502199
(43) Date de publication de la demande: 28.11.2007
(73) Titulaire: Aventis Pharma S.A., 92160 Antony (FR)
(72) Inventeur: BARBERIS, Claude, 94440 Santeny (FR); CARRY, Jean-Christophe c/o sanofi-aventis, Département Brevets, 75013 Paris (FR); DOERFLINGER, Gilles c/o sanofi-aventis, Département Brevets, 75013 Paris (FR); BARBALAT-DAMOUR, Dominique, 94130 Orly (FR); CLERC, François-Frédéric, 92160 Antony (FR); MINOUX, Hervé c/o sanofi-aventis, Département Brevets, 75013 Paris (FR)
(74) Mandataire: Le Pennec, Magali
(86) Numéro de dépôt international: PCT/FR2006/000480
(87) Numéro de publication internationale: WO 2006/092510

(56) Documents cités:
- WO-A-20/04013146

## Description

La présente invention concerne notamment des hydrazinocarbonyl-thiéno[2,3-c]pyrazoles substitués, leur procédé de préparation, des compositions les contenant, et leur utilisation comme médicament.

Plus particulièrement, et selon un premier aspect, l'invention concerne des hydrazinocarbonyl-thiéno[2,3-c]pyrazoles substitués, utiles comme agents anticancéreux.

Des 1H-thiéno[2,3-c]pyrazoles sont connus de WO 04/013146 et de WO 03/101968. Ces produits sont présentés comme inhibiteurs de nombreuses protéines kinases. Toutefois, l'administration de tels produits à des patients peut induire des effets secondaires importants, en raison de leur large spectre d'action. Ainsi, l'obtention d'inhibiteurs spécifiques d'une sélection de protéines, notamment de kinases, est recherchée.

Contre toute attente, il a été trouvé qu'il est possible d'obtenir des inhibiteurs de la kinase Aurora 2 (Aurora A) et de quelques autres kinases utiles en oncologie, par des hydrazinocarbonyl-1 H-thiéno[2,3-c]pyrazoles substitués.

Ces produits répondent à la formule générale (I) suivante : 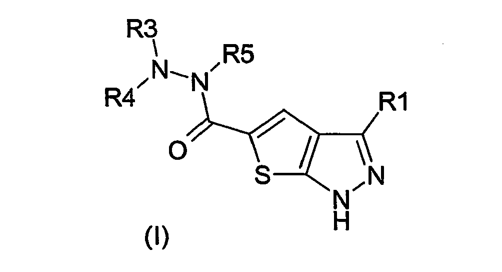 dans laquelle :
(i) R1 est indépendamment sélectionné dans le groupe constitué par - NHCO(R2), -NHCONH(R2), -NHCOO(R2), dans lequel R2 est indépendamment sélectionné dans le groupe constitué par -H, -(C₁-C₂₄)alkyle, (C₃-C₉)cycloalkyle, -(C₃-C₉)cycloalkylène, hétérocycloalkyle, hétérocycloalkylène, aryle, hétéroaryle, -(C₁-C₆)alkyl-aryle, -(C₁-C₆)alkyl-hétéroaryle, -aryl-(C₁-C₆)alkyle, - hétéroaryl-(C1-C6)alkyle, éventuellement substitués ;
(ii) Chacun des R3, R4, et R5 est indépendamment sélectionné dans le groupe constitué par H, -(C₁-C₆)alkyle, -(C₁-C₆)alkyl-aryle, -(C₁-C₆)alkyl-hétéroaryle, -aryle, -hétéroaryle, éventuellement substitués, ou bien [(R3 et R4) ou (R3 et R5)] sont liés entre eux pour former un hétérocycle mono ou bicyclique, saturé ou insaturé, comprenant de 2 à 10 chaînons carbonés et de 1 à 5 hétéroatomes choisis parmi N, O, et S, éventuellement substitué, ou pour former un groupement N=CH-aryle, le dit aryle étant éventuellement substitué.

Dans le cadre de l'invention, et sauf mention différente dans le texte, on entend par :
- halogène : un fluor, un chlore, un brome ou un iode.
- alkyle : un substituant aliphatique saturé linéaire ou ramifié, comprenant de 1 à 12 atomes de carbone. A titre d'exemples, on peut citer les groupes méthyle, éthyle, propyle, isopropyle, butyle, isobutyle, tertbutyle, pentyle, 1-méthylbutyle, 2-méthylbutyle, 3-méthylbutyle, 1,1-diméthylpropyle, 1,2-diméthylpropyle, 2,2-diméthyl-propyle, 1-éthylpropyle, hexyle, 1-méthylpentyle, 2-méthylpentyle, 1-éthyl-butyle, 2-éthylbutyle, 3,3-diméthylbutyle, heptyle, 1-éthylpentyle, etc ;
- alkylène : un substituant alkyle tel que défini ci-dessus, qui est divalent. A titre d'exemple, on peut citer les groupes méthylène (-CH₂-)
ou diméthylène (-CH₂-CH₂-), éthylènyle, 1-méthyléthylènyle, prop-1-ènyle, prop-2-ènyle, Z-1-méthylprop-1-ènyle, E-1-méthylprop-1-ènyle, Z-1,2-diméthyl-prop-1-ènyle, E-1,2-diméthylprop-1-ènyle, but-1,3-diényle, 1-méthylidènyl-prop-2-ènyle, Z-2-méthylbut-1,3-diényle, E-2-méthylbut-1,3-diényle, 2-méthyl-1-méthylidènylprop-2-ènyle, undéc-1-ènyle et undéc-10-ènyle, etc ;
- aryle : un substituant aromatique mono- ou polycyclique ayant de 6 à 14 atomes de carbone. A titre d'exemples, on peut citer les groupes phényle, napht-1-yle, napht-2-yle, anthracen-9-yl, 1,2,3,4-tétrahydronapht-5-yle , 1,2,3,4-tétrahydronapht-6-yle, etc ;
- Cycloalkyle : un substituant alkyle tel que défini ci-dessus, qui comprend de 3 à 12 atomes de carbone et qui est cyclique. A titre d'exemples, on peut citer les substituants cyclopropyle, cyclobutyle, cyclopentyle, cyclopentènyle, cyclopentadiényle, cyclohexyle, cyclohexènyle, cycloheptyle; bicyclo[2.2.1 ]heptyle, cyclooctyle, bicyclo[2.2.2]octyle, adamantyle, perhydronapthyle, etc ;
- hétéroatome : un atome d'azote, d'oxygène ou de soufre ;
- Hétéroaryle : un substituant aromatique mono- ou polycyclique comprenant de 5-12 chaînons et comprenant de 1 à 4 hétéroatomes tels que précédemment définis. A titre d'exemples, on peut citer les substituants pyrrol-1-yle, pyrrol2-yle, pyrrol3-yle, furyle, thienyle, imidazolyle, pyrazolyle, oxazolyle, thiazolyle, isoxazolyle, isothiazolyle, 1,2,4-triazolyle, oxadiazolyle, thiadiazolyle, tétrazolyle, pyridyle, pyrimidyle, pyrazinyle, 1,3,5-triazinyle, indolyle, benzo[b]furyle, benzo[b]thiényle, indazolyle, benzimidazolyle, azaindolyle, quinoléyle, isoquinoléyle, carbazolyle , acridyle, etc ;
- hétérocyclyle : un substituant hydrocarboné cyclique saturé ou partiellement insaturé comprenant 5 à 13 chaînons et comprenant 1 à 4 hétéroatomes tels que précédemment définis. De préférence, le substituant hydrocarboné cyclique saturé ou partiellement insaturé sera monocyclique et comportera 4 ou 5 atomes de carbone et 1 à 3 hétéroatomes. A titre d'exemples, on peut citer les substituants piperidinyle, azetidinyle, piperazinyle, morpholinyle, oxazepinyle, pyrrolidinyle, diazepinyle, etc ;
- alcoxy : un substituant de formule -O-alkyle où le groupe alkyle est tel que précédemment défini.
- substitué : un substituant différent de H, par exemple halogène, alkyle, aryle; hétéroaryle, cycloalkyle, hétérocyclyle, alkylène, alkynyle, OH, O-alkyle, O-alkylène, O-aryle, O-hétéroaryle, SH, S-alkyle, S-aryle, S(O₂)H, S(O₂)-alkyle, S(O₂)-arylₑ, SO₃H, SO₃-alkyle, SO₃-aryle, CHO, C(O)-alkyle, C(O)-aryle, C(O)OH, C(O)O-alkyle, C(O)O-aryle, OC(O)-alkyle, OC(O)-aryle, C(O)NH₂, C(O)NH-aryle, C(O)NHR7, C(O)NR7R8, -(C₁-C₃)alkyle-aryle, -(C₁-C₃)alkyle-hétéroaryle, -(C₁-C₃)alkyle-hétérocyclyle, -(C₁-C₃)alkyle-cycloalkyle, NR7R8, où les cycles sont éventuellement substitués par un ou plusieurs substituants du groupe halogène, (C₁-C₃)alkyle, alcoxy, et où R7 et R8 sont indépendamment sélectionnés dans le groupe constitué par H, alkyle, aryle, hétéroaryle, cycloalkyle, hétérocyclyle, alkyle-N[(C₁-C₆)alkyle]₂, -C(O)-alkyle, -C(O)-aryle. De préférence dans le groupe R2 le terme « substitué » désigne un substituant halogène, (C₁-C₆)alkyle, aryle, hétéroaryle, cycloalkyle, hétérocyclyle, alkylène, alkynyle, OH, O-alkyle, O-alkylène, NR7R8, où les cycles sont éventuellement substitués par un ou plusieurs substituants du groupe halogène, (C₁-C₃)alkyle, alcoxy, et où R7 et R8 sont indépendamment sélectionnés dans le groupe constitué par hydrogène, (C₀-C₃)alkyle, phényle, hétéroaryle, cycloalkyle, hétérocyclyle, (C₁-C₃)alkyle-N[(C₀-C₃)alkyle]₂. De préférence lorsque [(R3 et R4) ou (R3 et R5)] sont liés entre eux pour former un cycle ou un groupement N=CH-aryle, le terme « substitué » désigne un ou plusieurs substituants du groupe halogène, -(C₁-C₆)alkyle, -(C₀-C₆)alkyle-alcoxy
- C₀ : une liaison covalente ou -H.
- Par convention pour -(C₁-C₆)alkyl-aryle : le rattachement au reste de la molécule se fait au niveau du groupe alkyl. Inversement pour -Aryl-(C₁-C₆)-alkyle, le rattachement se fait au niveau du groupement aryle.
- Par protéines JNK on entend les protéines JNK1, 2 et 3.

Parmi les valeurs préférées de R1 on choisit NHCO(R2) ; dans lequel R2 est, de préférence, choisi parmi aryle, hétéroaryle et -aryl-(C₁-C₆)alkyle, éventuellement substitué.

L'invention a tout particulièrement pour objet les produits de formule (I) répondant à la formule (I'): 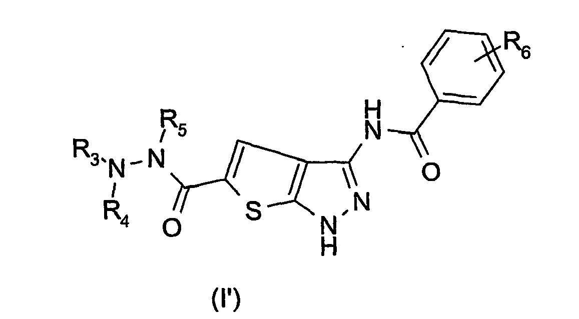 dans laquelle :
- Chacun des groupements R3, R4, et R5 sont tels que précédemment définis et,
- R6 est choisi parmi le groupe constitué par halogène, (C₁-C₃)alkyle-NR₇R₈, (C₁-C₆)alcoxy, (C₀-C₃)alkyle-hétérocycle, (C₀-C₃)alkyle-aryle, (C₀-C₃)alkyle-hétéroaryle, (C₀-C₃)alkyle-cylcoalkyle, où les cycles sont éventuellement substitués par un ou plusieurs substituants (C₁-C₃)alkyle, halogène, alcoxy, et où R₇ et R₈ sont indépendamment sélectionnés dans le groupe constitué par -H, (C₀-C₃)alkyle, aryle, (C₁-C₃)alkyle-N-[(C₀-C₃)alkyle]₂.

Plus particulièrement le groupement R6 est en position 3 ou 4 sur le phényle auquel il se rattache.

Plus particulièrement, R6 selon la présente invention est -(C₀-C₃)alkyle-hétérocycle, le dit hétérocycle étant lui même éventuellement substitué par un ou plusieurs substituants (C₁-C₃)alkyle, halogène, alcoxy.

Plus particulièrement, R3 est avantageusement choisi parmi aryle, hétéroaryle, -(C₁-C₆)alkyl-aryle ou -(C₁-C₆)alkyl-hétéroaryle, éventuellement substitué, de préférence parmi phényle, pyridyle, indolyle, benzimidazolyle, pyrazolyle, et pyrrolyle, éventuellement substitué.

Plus particulièrement R4 est avantageusement choisi parmi H ou alkyle, notamment -(C₁-C₆)alkyle. Tout particulièrement R4 est H, méthyle, ou éthyle.

Plus particulièrement R5 est avantageusement choisi parmi H ou alkyle, notamment -(C₁-C₆)alkyle.

Plus particuliérement, R3 et R4 forment avec l'azote auquel ils sont rattachés, un hétérocycle de 5 à 6 chaînons renfermant en outre le cas échéant un atome de N, O, ou S, éventuellement substitué, tout particulièrement piperidine ou pyrrolidine, éventuellement substitué.

Plus généralement, et dans le contexte de l'invention, un aryle préféré est phényle, et un hétéroaryle préféré est choisi parmi pyridyle, indolyle, benzimidazolyle, pyrazolyle, thiényl et pyrrolyle, éventuellement substitué.

Des produits illustratifs de l'invention selon son premier aspect peuvent être choisis parmi :
5-(N'-Phényl-hydrazinocarbonyl)-1H-thiéno[2,3-c]pyrazol-3-yl]-4-méthoxy-benzamide,
5-(N'-Méthyl-N'-phényl-hydrazinocarbonyl)-1H-thiéno[2,3-c]pyrazol-3-yl]-4-méthoxy-benzamide,
N-[5-(N'-Cyclohexyl-hydrafinocarbonyl)-1H-thiéno[2,3-c]pyrazol-3-yl]-4-méthoxy-benzamide,
N-[5-(N'-Benzyl-N'-phényl-hydrazinocarbonyl)-1H-thiéno[2,3-c]pyrazol-3-yl]-4-méthoxy-benzamide,
N-{5-[N'-(2-Ethyl-phényl)-hydrazinocarbonyl]-1H-thiéno[2,3-c]pyrazol-3-yl}-4-méthoxy-benzamide,
N-{5-[N'-(2-Fluoro-phényl)-hydrazinocarbonyl]-1H-thiéno[2,3-c]pyrazol-3-yl}-4-méthoxy-benzamide,
4-Méthoxy-N-[5,(N'-*o*-tolyl-hydrazinocarbonyl)-1H-thiéno[2,3-c]pyrazol-3-yl]-benzamide,
N-[5-(N'-Méthyl-N'-phényl-hydrazinocarbonyl)-1H-thiéno[2,3-c]pyrazol-3-yl]-4-morpholin-4-ylméthyl-benzamide,
4-Bromo-N-[5-(N'-méthyl-N'-phényl-hydrazinocarbonyl)-1H-thiéno[2,3-c]pyrazol-3-yl]-benzamide,
N-[5-(N'-Méthyl-N'-phényl-hydrazinocarbonyl)-1H-thiéno[2,3-c]pyrazol-3-yl]-4-(4-méthyl-pipérazin-1-ylméthyl)-benzamide,
4-(3,5-Diméthyl-pipérazin-1-ylméthyl)-N-[5-(N'-méthyl-N'-phényl-hydrazinocarbonyl)-1H-thiéno[2,3-c]pyrazol-3-yl]-benzamide,
4-(3,5-Diméthyl-pipérazin-1-ylméthyl)-N-[5-(N'-(4-chloro-phényl)-N'-méthyl-hydrazinocarbonyl)-1H-thiéno[2,3-c]pyrazol-3-yl]-benzamide,
4-(4-Méthyl-perhydro-1,4-diazepin-1-ylméthyl)-N-[5-(N'-méthyl-N'-phényl-hydrazinocarbonyl)-1H-thiéno[2,3-c]pyrazol-3-yl]-benzamide,
4-(4-Méthyl-perhydro-1,4-diazepin-1-ylméthyl)-N-[5-(N'-(4-chloro-phényl)-N'-méthyl-hydrazinocarbonyl)-1H-thiéno[2,3-c]pyrazol-3-yl]-benzamide,
N-[5-(N'-Méthyl-N'-phényl-hydrazinocarbonyl)-1H-thiéno[2,3-c]pyrazol-3-yl]-4-pipérazin-1-ylméthyl-benzamide,
N-{5-[N'-(4-Chloro-phényl)-N'-méthyl-hydrazinocarbonyl]-1H-thiéno[2,3-c]pyrazol-3-yl}-4-pipérazin-1-ylméthyl-benzamide,
N-[5-(N'-Méthyl-N'-phényl-hydrazinocarbonyl)-1H-thiéno[2,3-c]pyrazol-3-yl]-4-(3-méthyl-piperazin-1-ylméthyl)-benzamide,
N-{5-[N'-(4-Chloro-phényl)-N'-méthyl-hydrazinocarbonyl]-1H-thiéno[2,3-c]pyrazol-3-yl}-4-(3-méthyl-pipérazin-1-ylméthyl)-benzamide,
4-Diéthylaminométhyl-N-[5-(N'-méthyl-N'-phényl-hydrazinocarbonyl)-1H-thiéno[2,3-c]pyrazol-3-yl]-benzamide; trifluoro-acetate,
N-{5-[N'-(4-Chloro-phényl)-N'-méthyl-hydrazinocarbonyl]-1H-thiéno[2,3-c]pyrazol-3-yl}-4-diéthylaminométhyl-benzamide,
N-[5-(N'-Méthyl-N'-phényl-hydrazinocarbonyl)-1H-thiéno[2,3-c]pyrazol-3-yl]-3-(4-méthyl-pipérazin-1-ylméthyl)-benzamide,
N-[5-(N'-(4-Chloro-phényl)-N'-méthyl-hydrazinocarbonyl)-1H-thiéno[2,3-c]pyrazol-3-yl]-3-(4-méthyl-pipérazin-1-ylméthyl)-benzamide,
N-[5-(N'-Méthyl-N'-phényl-hydrazinocarbonyl)-1 H-thiéno[2,3-c]pyrazol-3-yl]-4-pipéridin-1-ylméthyl-benzamide,
N-[5-(N'-(4-Chloro-phényl)-N'-méthyl-hydrazinocarbonyl)-1 H-thiéno[2,3-c]pyrazol-3-yl]-4-pipéridin-1-ylméthyl-benzamide,
N-[5-(N'-Méthyl-N'-phényl-hydrazinocarbonyl)-1 H-thiéno[2,3-c]pyrazol-3-yl]-4-pyrrolidin-1-ylméthyl-benzamide,
N-[5-(N'-(4-Chloro-phényl)-N'-méthyl-hydrazinocarbonyl)-1 H-thiéno[2,3-c]pyrazol-3-yl]-4-pyrrolidin-1-ylméthyl-benzamide,
4-Azétidin-1-ylméthyl-N-[5-(N'-méthyl-N'-phényl-hydrazinocarbonyl)-1 H-thiéno[2,3-c]pyrazol-3-yl]-benzamide,
4-{[(2-Diméthylamino-éthyl)-méthyl-amino]-méthyl}-N-[5-(N'-méthyl-N'-phényl-hydrazinocarbonyl)-1 H-thiéno[2,3-c]pyrazol-3-yl]-benzamide,
N-{5-[N'-(4-Chloro-phényl)-N'-méthyl-hydrazinocarbonyl]-1 H-thiéno[2,3-c]pyrazol-3-yl}-4-{[(2-diméthylamino-éthyl)-méthyl-amino]-méthyl}-benzamide,
N-[5-(N'-Méthyl-N'-phényl-hydrazinocarbonyl)-1 H-thiéno[2,3-c]pyrazol-3-yl]-4-perhydro-1,4-oxazepin-4-ylméthyl-benzamide,
N-{5-[N'-(4-Chloro-phényl)-N'-méthyl-hydrazinocarbonyl]-1 H-thiéno[2,3-c]pyrazol-3-yl}-4-perhydro-1,4-oxazepin-4-ylméthyl-benzamide,
N-[5-(N'-Méthyl-N'-phényl-hydrazinocarbonyl)-1 H-thiéno[2,3-c]pyrazol-3-yl]-3-morpholin-4-ylméthyl-benzamide,
N-{5-[N'-(4-Chloro-phényl)-N'-méthyl-hydrazinocarbonyl]-1 H-thiéno[2,3-c]pyrazol-3-yl}-3-morpholin-4-ylméthyl-benzamide,
4-[(2-Diéthylamino-éthylamino)-méthyl]-N-[5-(N'-méthyl-N'-phényl-hydrazinocarbonyl)-1 H-thiéno[2,3-c]pyrazol-3-yl]-benzamide,
N-{5-[N'-(4-Chloro-phényl)-N'-méthyl-hydrazinocarbonyl]-1 H-thiéno[2,3-c]pyrazol-3-yl}-4-[(2-diéthylamino-éthylamino)-méthyl]-benzamide,
4-[(Méthyl-phényl-amino)-méthyl]-N-[5-(N'-méthyl-N'-phényl-hydrazinocarbonyl)-1 H-thiéno[2,3-c]pyrazol-3-yl]-benzamide,
4-[(Diisopropylamino)-méthyl]-N-[5-(N'-méthyl-N'-phényl-hydrazinocarbonyl)-1 H-thiéno[2,3-c]pyrazol-3-yl]-benzamide; trifluoroacétate,
N-[5-(N'-Benzyl-hydrazinocarbonyl)-1H-thiéno[2,3-c]pyrazol-3-yl]-4-méthoxy-benzamide,
4-Méthoxy-N-[5-(N'-pyridin-2-yl-hydrazinocarbonyl)-1H-thiéno[2,3-c]pyrazol-3-yl]-benzamide,
N-{5-[N'-(4-Chloro-phényl)-N'-méthyl-hydrazinocarbonyl]-1 H-thiéno[2,3-c]pyrazol-3-yl}-4-(4-méthyl-pipérazin-1-yl)-benzamide,
N-[5-(N'-Benzyl-N'-méthyl-hydrazinocarbonyl)-1H-thiéno[2,3-c]pyrazol-3-yl]-4-(4-méthyl-pipérazin-1-yl)-benzamide,
N-(Pipéridin-1-yl)-3-[4-(4-méthyl-pipérazin-1-yl)-benzoylamino]-1H-thiéno[2,3-c]pyrazole-5-carboxamide,
N-[5-(N'-Benzyl-hydrazinocarbonyl)-1H-thiéno[2,3-c]pyrazol-3-yl]-4-(4-méthyl-pipérazin-1-yl)-benzamide,
N-[5-(N'-Ethyl-N'-phényl-hydrazinocarbonyl)-1H-thiéno[2,3-c]pyrazol-3-yl]-4-(4-méthyl-pipérazin-1-yl)-benzamide,
N-[5-(N'-Benzyl-N'-phényl-hydrazinocarbonyl)-1H-thiéno[2,3-c]pyrazol-3-yl]-4-(4-méthyl-pipérazin-1-yl)-benzamide,
N-[5-(N'-Méthyl-N'-phényl-hydrazinocarbonyl)-1H-thiéno[2,3-c]pyrazol-3-yl]-4-(4-méthyl-pipérazin-1-yl)-benzamide,
N-{5-[N'-(4-Chloro-phényl)-N'-cyclobutylméthyl-hydrazinocarbonyl]-1H-thiéno[2,3-c]pyrazol-3-yl}-4-(4-méthyl-pipérazin-1-yl)-benzamide,
N-{5-[N'-Ethyl-N'-(4-fluoro-phényl)-hydrazinocarbonyl]-1H-thiéno[2,3-c]pyrazol-3-yl}-4-(4-méthyl-pipérazin-1-yl)-benzamide,
N-{(5-[N'-(4-Fluoro-phényl)-N'-(2-méthoxy-éthyl)-hydrazinocarbonyl]-1H-thiéno[2,3-c]pyrazol-3-yl}-4-(4-méthyl-pipérazin-1-yl)-benzamide,
N-[5-(N'-Ethyl-N'-*o*-tolyl-hydrazinocarbonyl)-1H-thiéno[2,3-c]pyrazol-3-yl]-4-(4-méthyl-pipérazin-1-yl)-benzamide,
N-[5-(N'-Ethyl-N'-*m*-tolyl-hydrazinocarbonyl)-1H-thiéno[2,3-c]pyrazol-3-yl]-4-(4-méthyl-pipérazin-1-yl)-benzamide,
N-[5-(N'-Ethyl-N'-*m*-tolyl-hydrazinocarbonyl)-1H-thiéno[2,3-c]pyrazol-3-yl]-4-(4-méthyl-pipérazin-1-yl)-benzamide,
N-{5-[N'-(4-Fluoro-phényl)-N'-isobutyl-hydrazinocarbonyl]-1H-thiéno[2,3-c]pyrazol-3-yl}-4-(4-méthyl-pipérazin-1-yl)-benzamide,
N-{5-[N'-(3-Bromo-phényl)-N'-éthyl-hydrazinocarbonyl]-1H-thiéno[2,3-c]pyrazol-3-yl}-4-(4-méthyl-pipérazin-1-yl)-benzamide,
6-(4-Méthyl-[1,4]diazepan-1-yl)-N-[5-(N'-méthyl-N'-phényl-hydrazinocarbonyl)-1 H-thiéno[2,3-c]pyrazol-3-yl]-nicotinamide,
N-[5-(Benzylidène-hydrazinocarbonyl)-1H-thiéno[2,3-c]pyrazol-3-yl]-4-(4-méthyl-pipérazin-1-yl)-benzamide,
N-[5-(N'-Méthyl-N'-phényl-hydrazinocarbonyl)-1H-thiéno[2,3-c]pyrazol-3-yl]-4-(2-morpholin-4-yl-éthoxy)-benzamide,
4-(4-méthyl-[1,4]diazepan-1-yl)-N-[5-(N'-méthyl-N'-phényl-hydrazinocarbonyl)-1 H-thiéno[2,3-c]pyrazol-3-yl]-benzamide,

Les produits conformes à l'invention peuvent se présenter sous forme non chirale, racémique, enrichie en un stéréo-isomère ou enrichie en un énantiomère ; et sont éventuellement salifiés.

Selon un second aspect, l'invention a pour objet un procédé de préparation des produits selon son premier aspect.

En particulier l'invention concerne un procédé de préparation d'un produit de formule générale (I) suivante : 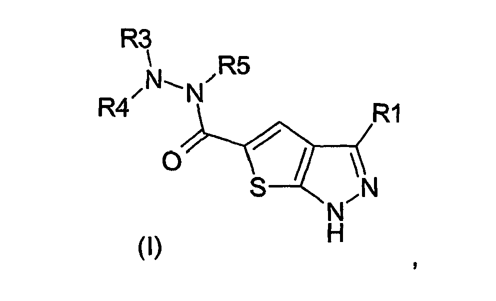 dans laquelle R1 est NHCO(R2), et dans lequel R3, R4 et R5 sont tels que définis précédemment, ledit produit de formule générale (I) étant obtenu par :
(i) couplage entre (i-a) un acide de formule générale (X) suivante : 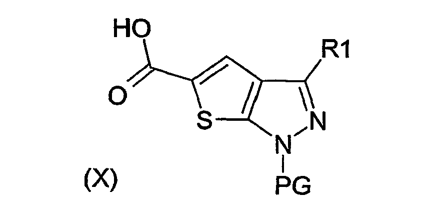 dans laquelle R1 est tel que défini précédemment, et dans lequel PG est un groupe protecteur de la fonction NH libre intracyclique du noyau thiéno[2,3-c]pyrazole, et
   (i-b) une hydrazine (R3)(R4)N-NH(R5) dans laquelle R3, R4 et R5 sont tels que définis précédemment, en présence d'un agent de couplage et en présence d'une base telle qu'une amine tertiaire ou un carbonate d'un métal alcalin ; puis
(ii) clivage de PG.

Le produit de formule générale (X) peut être obtenu par saponification de la fonction ester du noyau thiophène d'un produit de formule générale (IX): 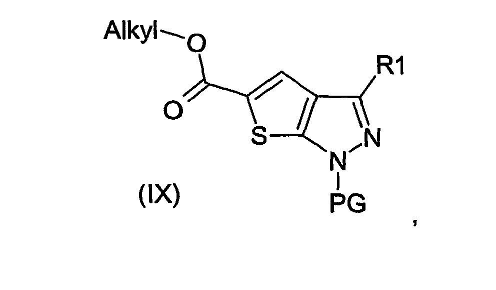 dans lequel R1 est tel que défini précédemment.

Le produit de formule générale (IX) peut être obtenu par couplage entre :
(i) un produit de formule générale (IIa) suivante : 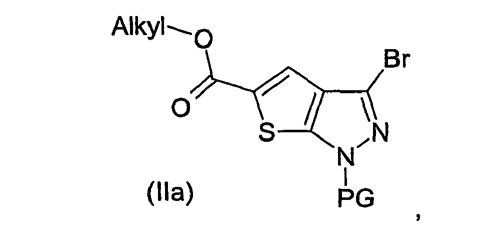 dans laquelle Alkyl est tel que défini précédemment, et dans laquelle PG est un groupe protecteur de la fonction NH libre intracyclique du noyau thiéno[2,3-c]pyrazole, et
(ii) un produit de formule générale (R2)CONH₂, en présence :
   - d'un catalyseur tel que l'iodure de cuivre (I),
   - d'une amine telle que le *trans*-1,2-diaminocyclohexane, le *trans*-1,2-bis(méthylamino)cyclohexane ou, de préférence le N,N'-diméthyl-1,2-diaminoéthane, et
   - d'une base telle que le phosphate tripotassique ou le carbonate de césium.

Le produit de formule générale (IIa) peut être obtenu par réaction entre un mercaptoacétate d'alkyle Alkyl-OCO-CH₂-SH, en présence d'une base telle que le carbonate de sodium et un composé (IIIa) : 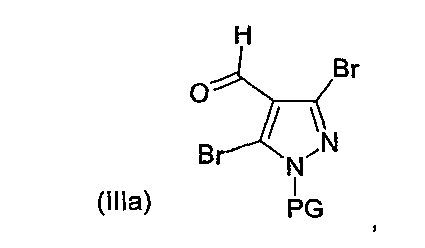 dans lequel PG est tel que défini précédemment :
Le produit de formule générale (IIIa) peut être obtenu par (i) formylation du 3,4,5-tribromo-pyrazole pour l'obtention de 3,5-dibromo-4-formyl-pyrazole (III), puis (ii) protection de la fonction amine intracyclique de (III) par l'introduction du groupe protecteur PG.

Le groupe protecteur PG peut être introduit via :
(i) réaction avec de l'éthylvinyl éther en présence d'un acide tel que l'acide chlorhydrique, pour l'obtention d'un groupe PG = 1-éthoxy-éthyle, au sein d'un solvant inerte tel que le toluène, ou ;
(ii) réaction avec du di-*tert*-butyldicarbonate en présence d'une base telle que la triéthylamine, la pyridine, ou la N,N-diméthylamino-pyridine, pour l'obtention d'un groupe PG = *tert*-butyloxycarbonyle, au sein d'un solvant inerte tel que le dichlorométhane.

Selon un troisième aspect, l'invention concerne une composition pharmaceutique comprenant un produit selon son premier aspect, en combinaison avec un excipient pharmaceutiquement acceptable.

Selon un quatrième aspect, l'invention a pour objet l'utilisation d'un produit selon son premier aspect, pour la fabrication d'un médicament utile pour traiter un état pathologique, en particulier le cancer.

Selon une variante du quatrième aspect, l'invention a pour objet l'utilisation d'un produit selon son premier aspect, pour la fabrication d'un médicament utile pour traiter un état pathologique choisi parmi le psoriasis, le glaucome, les leucémies, les maladies liées au système nerveux central, les inflammations, et les maladies liées à une dérégulation des protéines JNK.

Les composés de formule (I) peuvent être préparés à partir des composés de formule générale (II), selon le schéma de synthèse général suivant:

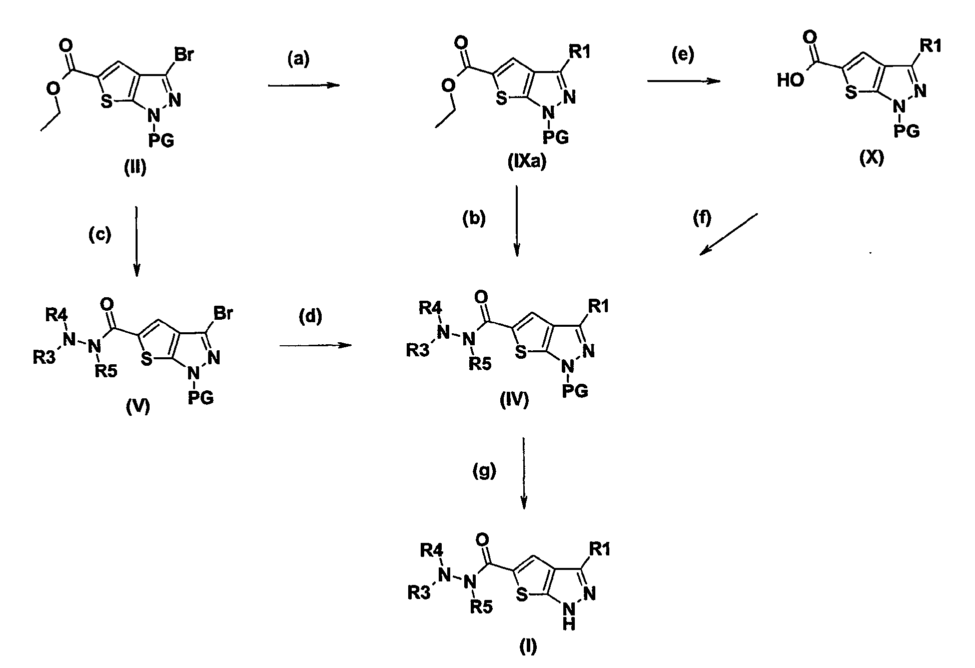

Les réactions (a) et (d) peuvent s'effectuer lorsque R1 représente NHCO(R2) où R2 a la même signification que précédemment en présence d'un amide de type (R2)CONH₂, d'iodure de cuivre (I), d'une amine telle que le *trans*-1,2-diaminocyclohexane, le *trans*-1,2-bis(méthylamino)cyclohexane ou, - et c'est un des aspects de l'invention -, le N,N'-diméthyl-1,2-diaminoéthane, et d'une base telle que le phosphate tripotassique ou le carbonate de césium au sein d'un solvant inerte tel que le dioxanne à une température comprise entre 20°C et la température d'ébullition du milieu réactionnel, selon les méthodes générales décrites par S. L. BUCHWALD et al., J. Am. Chem. Soc., 2002, 124, 7421; J. Am. Chem. Soc., 2001, 123, 7727.

Les réactions (b) et (c) peuvent s'effectuer en présence d'un dérivé de type (R3)(R4)NN(R5)H où R3, R4, R5 ont la même signification que précédemment et de triméthylaluminium au sein d'un solvant tel que le toluène ou le diméthylformamide à une température comprise entre 0°C et la température d'ébullition du milieu réactionnel.

La réaction (e) s'effectue généralement selon les méthodes habituelles qui n'affectent pas le reste de la molécule, notamment par applications des méthodes décrites par T. W. Greene et P. G. M. Wuts, Protective Groups in Organic Synthesis (2 ème éd.), A. Wiley - Interscience Publication (1991), ou par Mc Omie, Protective Groups in Organic Chemistry, Plenum Press (1973) ou par Bradford P. Mundy et Michael G. Ellerd, Name Reactions and Reagents in Organic Synthesis, A. Wiley - Interscience Publication (1988). La réaction (e) peut s'effectuer par exemple en milieu basique, en présence d'hydroxyde de potassium ou d'hydroxyde de sodium, au sein d'un solvant inerte tel qu'un mélange de tétrahydrofuranne, d'eau et d'un alcool (éthanol ou méthanol de préférence) ou bien d'un alcool seul (éthanol ou méthanol de préférence), à une température comprise entre 20°C et la température d'ébullition du milieu réactionnel, de préférence à la température de reflux du milieu réactionnel.

La réaction (f) s'effectue de préférence en présence d'un dérivé de type R3R4NNR5H où R3, R4, R5 ont la même signification que précédemment et d'un agent d'activation du type tetrafluoroborate de O-(1 H-benzotriazol-1-yl)-N,N,N',N'-tetraméthyluronium (TBTU) par exemple, en présence d'une base (triéthylamine ou diisopropyléthylamine par exemple) au sein d'un solvant inerte (acétonitrile ou diméthylformamide par exemple) à une température comprise entre 0°C et la température d'ébullition du milieu, ou selon les méthodes bien connues de couplage de la chimie peptidique (M. BODANSZKY et coll., Principles of Peptide Synthesis, Spinger-Verlag, New York, NY, 1984, 9-58) ou de la formation d'un amide. Alternativement, la réaction (f) peut s'effectuer généralement selon les méthodes habituelles qui n'affectent pas le reste de la molécule, notamment par applications des méthodes décrites par Bradford P. Mundy et Michael G. Ellerd, Name Reactions and Reagents in Organic Synthesis, A. Wiley - Interscience Publication (1988). Par exemple la réaction (f) peut s'effectuer sous atmosphère inerte (par exemple sous azote ou sous argon) en présence de chlorure d'oxalyle, au sein d'un solvant inerte tel que du dichlorométhane, à une température comprise entre 20°C et la température d'ébullition du milieu réactionnel, de préférence à une température voisine de 20°C ou bien en présence de chlorure de sulfinyle, au sein d'un solvant inerte tel que du chloroforme, à une température comprise entre 20°C et la température d'ébullition du milieu réactionnel, de préférence à la température de reflux du milieu réactionnel, suivi d'une réaction en présence d'un dérivé de type R3R4NNR5H où R3, R4, R5 ont la même signification que précédemment et d'une base telle que la triéthylamine ou la pyridine, au sein d'un solvant inerte tel que le diméthylformamide, à une température comprise entre 20°C et la température d'ébullition du milieu réactionnel.

La réaction de déprotection (g) peut s'effectuer (lorsque PG représente un groupement 1-éthoxy-éthyle) en présence d'un acide minéral tel que l'acide chlorhydrique, au sein d'un solvant tel que le tétrahydrofuranne ou l'eau, à une température comprise entre 20°C et la température d'ébullition du milieu réactionnel, ou encore selon les méthodes bien connues de déprotection de la fonction amine (T. W. GREENE et coll. dans Protective Groups in Organic Synthesis, third edition, 1999, Wiley-Interscience.

Les composés de formule générale (IIa) peuvent être préparés à partir du 3,4,5-tribromo-pyrazole, selon le schéma de synthèse général suivant:

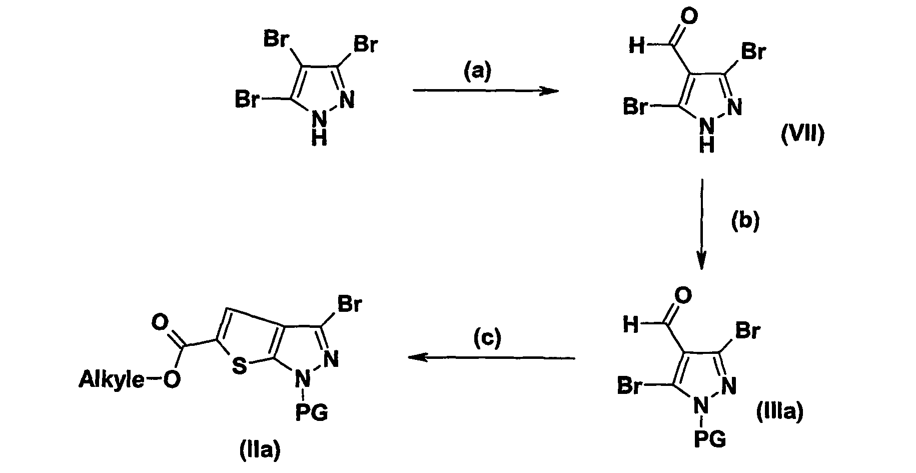

La réaction (a) peut s'effectuer en présence d'un organolithien tel que le n-butyllithium, en présence de diméthylformamide, au sein d'un solvant inerte tel que le diéthyl éther ou le tétrahydrofuranne à une température comprise entre -78°C et la température ambiante.

La réaction de protection (b) peut s'effectuer en présence d'éthylvinyl éther, en présence d'une quantité catalytique d'un acide tel que l'acide chlorhydrique, au sein d'un solvant inerte tel que le toluène à une température comprise entre 20°C et la température d'ébullition du milieu réactionnel ou selon les méthodes bien connues de protection de la fonction amine (T. W. GREENE et coll. dans Protective Groups in Organic Synthesis, third edition, 1999, Wiley-Interscience).

La réaction (c) peut s'effectuer en présence de mercaptoacétate d'éthyle, en présence d'une base telle que le carbonate de sodium, au sein d'un solvant inerte tel qu'un alcool (éthanol de préférence) à une température comprise entre 20°C et la température d'ébullition du milieu réactionnel.

Alternativement, lorsque R1 représente NHCO(R2) où R2 a la même signification que précédemment, les composés de formule (I) peuvent être préparés à partir des composés de formule générale (XIV), selon le schéma de synthèse général suivant:

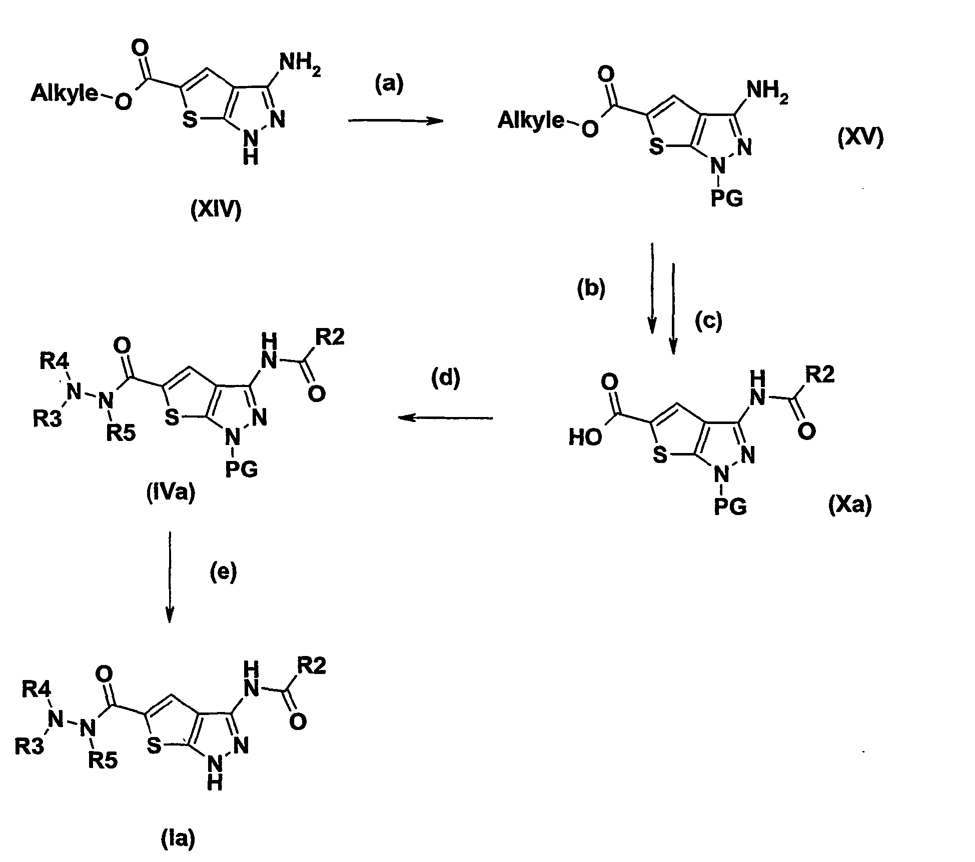

La réaction de protection (a) peut s'effectuer (lorsque PG représente un groupement *tert*-butyloxycarbonyle) à l'aide de di-*tert-*butyldicarbonate en présence d'une base telle que la triéthylamine ou la pyridine et éventuellement en présence de N,N-diméthylamino-pyridine, au sein d'un solvant inerte (dichlorométhane par exemple) à une température comprise entre -10°C et la température d'ébullition du milieu réactionnel, ou bien (lorsque PG représente un groupement 1-éthoxy-éthyle) en présence d'éthylvinyl éther, en présence d'une quantité catalytique d'un acide tel que l'acide chlorhydrique, au sein d'un solvant inerte tel que le toluène à une température comprise entre 20°C et la température d'ébullition du milieu réactionnel, ou selon les méthodes bien connues de protection de la fonction amine (T. W. GREENE et coll. dans Protective Groups in Organic Synthesis, third edition, 1999, Wiley-Interscience).

La réaction (b) peut s'effectuer:
- à l'aide d'un chlorure d'acide (R2)C(O)CI où R2 a la même signification que précédemment en présence d'une base comme la triéthylamine, la pyridine, la diisopropyléthylamine, le carbonate de potassium ou de sodium, au sein d'un solvant inerte (diméthylformamide, tetrahydrofuranne par exemple) ou dans la base organique elle-même à une température comprise entre 0°C et la température d'ébullition du milieu réactionnel (G. DAIDONE et coll, Heterocycles, 1996, 43(11), 2385).
- à l'aide d'un anhydride ((R2)CO)₂O où R2 a la même signification que précédemment au sein d'un solvant inerte (diméthylformamide, tétrahydrofuranne, dichlorométhane par exemple) ou dans l'anhydride lui-même à une température comprise entre 0°C et la température d'ébullition du milieu réactionnel (F. ALBERICIO, Synth. Commun., 2001, 31(2), 225, G. PROCTER, Tetrahedron, 1995, 51 (47), 12837).
- à l'aide d'un acide (R2)C(O)OH où R2 a la même signification que précédemment en présence d'un agent d'activation tel que l'hexafluorophosphate de O-(7-aza-benzotriazol-1-yl)-N,N,N',N'-tétraméthyluronium (HATU) en présence d'une base (pyridine, diisopropyléthylamine ou triéthylamine par exemple) au sein d'un solvant inerte (diméthylformamide par exemple) à une température comprise entre 0°C et la température d'ébullition du milieu réactionnel, ou selon les méthodes bien connues de couplage de la chimie peptidique (M. BODANSZKY et coll., Principles of Peptide Synthesis, Spinger-Verleg, New York, NY, 1984, 9-58) ou de la formation d'un amide.

La réaction (c) s'effectue généralement selon les méthodes habituelles qui n'affectent pas le reste de la molécule, notamment par applications des méthodes décrites par T. W. Greene et P. G. M. Wuts, Protective Groups in Organic Synthesis (2 ème éd.), A. Wiley - Interscience Publication (1991), ou par Mc Omie, Protective Groups in Organic Chemistry, Plenum Press (1973) ou par Bradford P. Mundy et Michael G. Ellerd, Name Reactions and Reagents in Organic Synthesis, A. Wiley - Interscience Publication (1988). La réaction (c) peut s'effectuer par exemple en milieu basique, en présence d'hydroxyde de potassium ou d'hydroxyde de sodium, au sein d'un solvant inerte tel qu'un mélange de tétrahydrofuranne, d'eau et d'un alcool (éthanol ou méthanol de préférence) ou bien d'un alcool seul (éthanol ou méthanol de préférence), à une température comprise entre 20°C et la température d'ébullition du milieu réactionnel, de préférence à la température de reflux du milieu réactionnel.

La réaction (d) s'effectue de préférence en présence d'un dérivé de type R3R4NNR5H où R3, R4, R5 ont la même signification que précédemment et d'un agent d'activation du type tetrafluoroborate de O-(1 H-benzotriazol-1-yl)-N,N,N',N'-tetraméthyluronium (TBTU) par exemple, en présence d'une base (triéthylamine ou diisopropyléthylamine par exemple) au sein d'un solvant inerte (acétonitrile ou diméthylformamide par exemple) à une température comprise entre 0°C et la température d'ébullition du milieu, ou selon les méthodes bien connues de couplage de la chimie peptidique (M. BODANSZKY et coll., Principles of Peptide Synthesis, Spinger-Verlag, New York, NY, 1984, 9-58) ou de la formation d'un amide. Alternativement, la réaction (d) peut s'effectuer généralement selon les méthodes habituelles qui n'affectent pas le reste de la molécule, notamment par applications des méthodes décrites par Bradford P. Mundy et Michael G. Ellerd, Name Reactions and Reagents in Organic Synthesis, A. Wiley - Interscience Publication (1988). Par exemple la réaction (d) peut s'effectuer sous atmosphère inerte (par exemple sous azote ou sous argon) en présence de chlorure d'oxalyle, au sein d'un solvant inerte tel que du dichlorométhane, à une température comprise entre 20°C et la température d'ébullition du milieu réactionnel, de préférence à une température voisine de 20°C ou bien en présence de chlorure de sulfinyle, au sein d'un solvant inerte tel que du chloroforme, à une température comprise entre 20°C et la température d'ébullition du milieu réactionnel, de préférence à la température de reflux du milieu réactionnel, suivi d'une réaction en présence d'un dérivé de type R3R4NNR5H où R3, R4, R5 ont la même signification que précédemment et d'une base telle que la triéthylamine ou la pyridine, au sein d'un solvant inerte tel que le diméthylformamide, à une température comprise entre 20°C et la température d'ébullition du milieu réactionnel.

La réaction de déprotection (e) peut s'effectuer (lorsque PG représente un groupement *tert*-butyloxycarbonyle) en présence d'iodotriméthylsilane, ou en milieu acide (acide trifluoroacétique, ou acide chlorhydrique dans un solvant tel que le dichlorométhane ou le dioxanne par exemple) à une température comprise entre 0°C et la température d'ébullition du milieu réactionnel, ou en milieu basique (carbonate de potassium au sein d'un solvant tel qu'un alcool (méthanol de préférence) à une température comprise entre 0°C et la température d'ébullition du milieu réactionnel et éventuellement sous irradiation par des micro-ondes), ou en milieu neutre dans un solvant tel qu'un alcool (méthanol de préférence) sous irradiation par des micro-ondes; ou bien (lorsque PG représente un groupement 1-éthoxy-éthyle) en présence d'un acide minéral tel que l'acide chlorhydrique, au sein d'un solvant tel que le tétrahydrofuranne ou l'eau, à une température comprise entre 20°C et la température d'ébullition du milieu réactionnel, ou encore selon les méthodes bien connues de déprotection de la fonction amine (T. W. GREENE et coll. dans Protective Groups in Organic Synthesis, third edition, 1999, Wiley-Interscience.

Les composés de formule générale (XVa) peuvent être préparés à partir du 3-amino-4-cyano-5-méthylsulfanylthiophène-2-carboxylate d'éthyle, selon le schéma de synthèse général suivant:

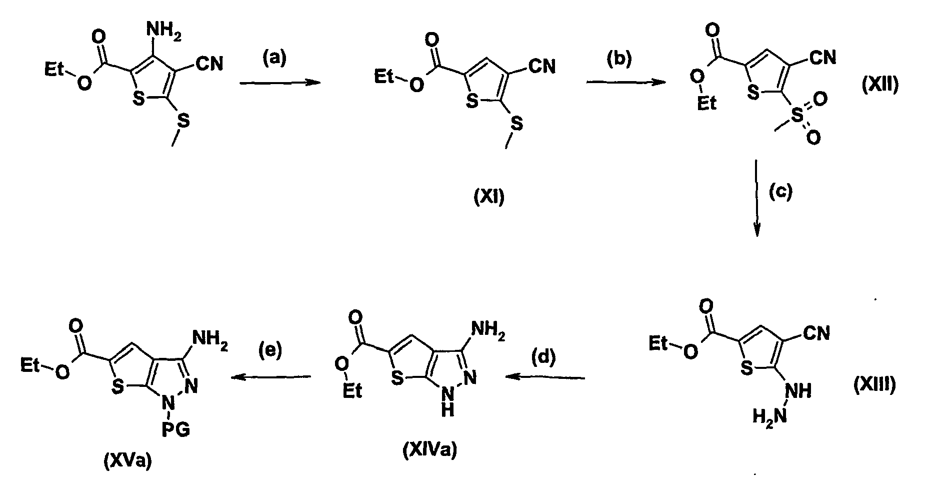

La réaction (a) peut s'effectuer en présence de nitrite d'isopentyle et de Cu(II), au sein d'un solvant inerte tel que le diméthylformamide à une température comprise entre 20°C et la température d'ébullition du milieu réactionnel.

La réaction d'oxydation (b) peut s'effectuer en présence d'acide 3-chloro-peroxybenzoïque, au sein d'un solvant inerte tel que le dichlorométhane à une température comprise entre -20°C et la température ambiante.

La réaction (c) peut s'effectuer en présence d'hydrazine, au sein d'un solvant inerte tel qu'un alcool (éthanol de préférence) à une température comprise entre 20°C et la température d'ébullition du milieu réactionnel.

La réaction de cyclisation (d) peut s'effectuer en présence d'un acide minéral tel que l'acide chlorhydrique concentré ou l'acide sulfurique concentré, au sein d'un solvant inerte tel qu'un alcool (éthanol de préférence) à une température comprise entre 20°C et la température d'ébullition du milieu réactionnel.

La réaction de protection (e) peut s'effectuer (lorsque PG représente un groupement *tert*-butyloxycarbonyle) à l'aide de di-*tert*-butyldicarbonate en présence d'une base telle que la triéthylamine ou la pyridine et éventuellement en présence de N,N-diméthylamino-pyridine, au sein d'un solvant inerte (dichlorométhane par exemple) à une température comprise entre -10°C et la température d'ébullition du milieu réactionnel, ou bien (lorsque PG représente un groupement 1-éthoxy-éthyle) en présence d'éthylvinyl éther, en présence d'une quantité catalytique d'un acide tel que l'acide chlorhydrique, au sein d'un solvant inerte tel que le toluène à une température comprise entre 20°C et la température d'ébullition du milieu réactionnel, ou selon les méthodes bien connues de protection de la fonction amine (T. W. GREENE et coll. dans Protective Groups in Organic Synthesis, third edition, 1999, Wiley-Interscience).

La synthèse du 3-amino-4-cyano-5-méthylsulfanyl-thiophène-2-carboxylate d'éthyle est décrite dans Synthesis 2003, 735

Alternativement, lorsque R1 représente NHCO(R2) où R2 a la même signification que précédemment, les composés de formule (I) peuvent être préparés à partir des composés de formule générale (XIV), selon le schéma de synthèse général suivant:

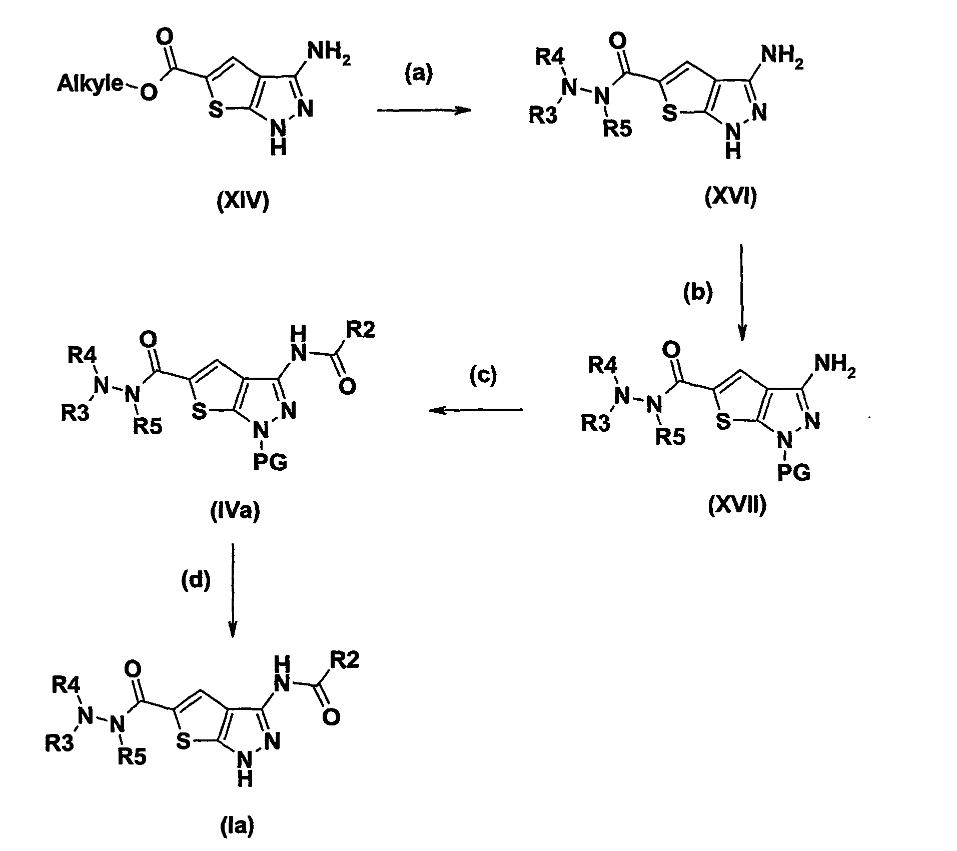

La réaction (a) peut s'effectuer en présence d'un dérivé de type (R3)(R4)NN(R5)H où R3, R4, R5 ont la même signification que précédemment et de triméthylaluminium au sein d'un solvant tel que le toluène ou le diméthylformamide à une température comprise entre 0°C et la température d'ébullition du milieu réactionnel.

La réaction de protection (b) peut s'effectuer (lorsque PG représente un groupement *tert*-butyloxycarbonyle) à l'aide de di-*tert*-butyldicarbonate en présence d'une base telle que la triéthylamine ou la pyridine et éventuellement en présence de N,N-diméthylamino-pyridine, au sein d'un solvant inerte (dichlorométhane par exemple) à une température comprise entre -10°C et la température d'ébullition du milieu réactionnel, ou bien (lorsque PG représente un groupement 1-éthoxy-éthyle) en présence d'éthylvinyl éther, en présence d'une quantité catalytique d'un acide tel que l'acide chlorhydrique, au sein d'un solvant inerte tel que le toluène à une température comprise entre 20°C et la température d'ébullition du milieu réactionnel, ou selon les méthodes bien connues de protection de la fonction amine (T. W. GREENE et coll. dans Protective Groups in Organic Synthesis, third edition, 1999, Wiley-Interscience).

La réaction (c) peut s'effectuer:
- à l'aide d'un chlorure d'acide (R2)C(O)CI où R2 a la même signification que précédemment en présence d'une base comme la triéthylamine, la pyridine, la diisopropyléthylamine, le carbonate de potassium ou de sodium, au sein d'un solvant inerte (diméthylformamide, tetrahydrofuranne par exemple) ou dans la base organique elle-même à une température comprise entre 0°C et la température d'ébullition du milieu réactionnel (G. DAIDONE et coll, Heterocycles, 1996, 43(11), 2385).
- à l'aide d'un anhydride ((R2)CO)₂O où R2 a la même signification que précédemment au sein d'un solvant inerte (diméthylformamide, tétrahydrofuranne, dichlorométhane par exemple) ou dans l'anhydride lui-même à une température comprise entre 0°C et la température d'ébullition du milieu réactionnel (F. ALBERICIO, Synth. Commun., 2001, 31(2), 225, G. PROCTER, Tetrahedron, 1995, 51 (47), 12837).
- à l'aide d'un acide (R2)C(O)OH où R2 a la même signification que précédemment en présence d'un agent d'activation tel que l'hexafluorophosphate de O-(7-aza-benzotriazol-1-yl)-N,N,N',N'-tétraméthyluronium (HATU) en présence d'une base (pyridine, diisopropyléthylamine ou triéthylamine par exemple) au sein d'un solvant inerte (diméthylformamide par exemple) à une température comprise entre 0°C et la température d'ébullition du milieu réactionnel, ou selon les méthodes bien connues de couplage de la chimie peptidique (M. BODANSZKY et coll., Principles of Peptide Synthesis, Spinger-Verleg, New York, NY, 1984, 9-58) ou de la formation d'un amide.

La réaction de déprotection (d) peut s'effectuer (lorsque PG représente un groupement *tert*-butyloxycarbonyle) en présence d'iodotriméthylsilane, ou en milieu acide (acide trifluoroacétique, ou acide chlorhydrique dans un solvant tel que le dichlorométhane ou le dioxanne par exemple) à une température comprise entre 0°C et la température d'ébullition du milieu réactionnel, ou en milieu basique (carbonate de potassium au sein d'un solvant tel qu'un alcool (méthanol de préférence) à une température comprise entre 0°C et la température d'ébullition du milieu réactionnel et éventuellement sous irradiation par des micro-ondes), ou en milieu neutre dans un solvant tel qu'un alcool (méthanol de préférence) sous irradiation par des micro-ondes; ou bien (lorsque PG représente un groupement 1-éthoxy-éthyle) en présence d'un acide minéral tel que l'acide chlorhydrique, au sein d'un solvant tel que le tétrahydrofuranne ou l'eau, à une température comprise entre 20°C et la température d'ébullition du milieu réactionnel, ou encore selon les méthodes bien connues de déprotection de la fonction amine (T. W. GREENE et coll. dans Protective Groups in Organic Synthesis, third edition, 1999, Wiley-Interscience.

Alternativement, lorsque R1 représente NHCO(R2) où R2 a la même signification que précédemment, les composés de formule (XVI) peuvent être préparés à partir des composés de formule générale (XI), selon le schéma de synthèse général suivant:

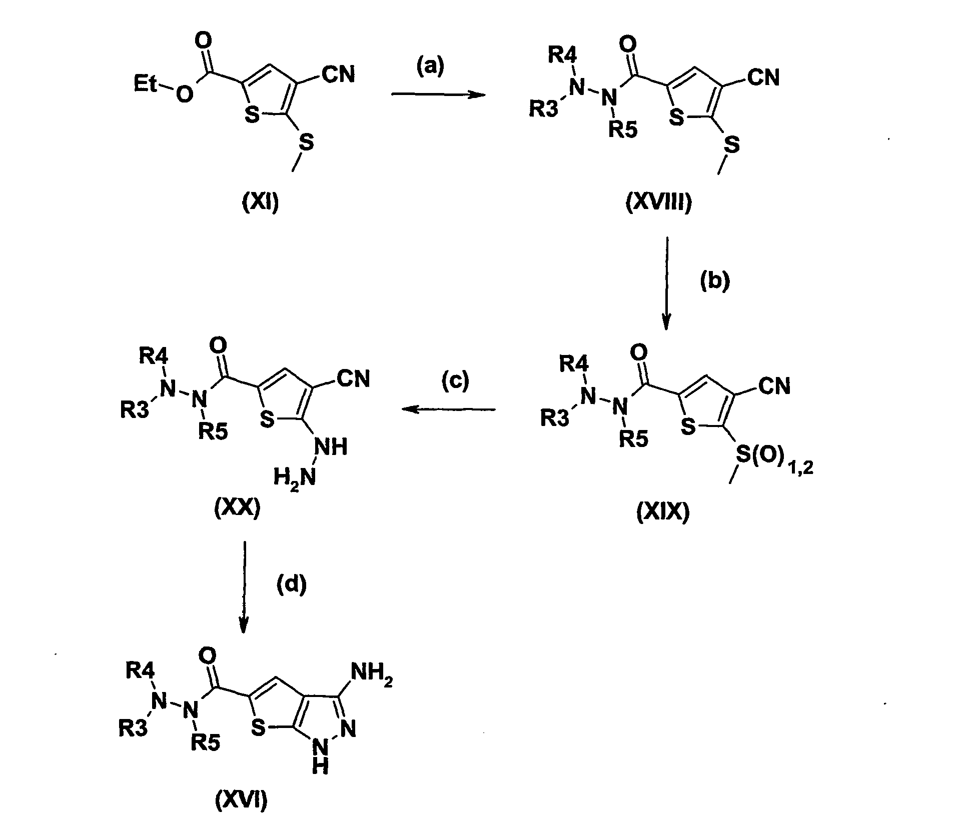

La réaction (a) peut s'effectuer en présence d'un dérivé de type (R3)(R4)NN(R5)H où R3, R4, R5 ont la même signification que précédemment et de triméthylaluminium au sein d'un solvant tel que le toluène ou le diméthylformamide à une température comprise entre 0°C et la température d'ébullition du milieu réactionnel.

La réaction d'oxydation (b) peut s'effectuer en présence d'oxone^{®} (peroxymonosulfate de potassium) ou d'acide 3-chloro-peroxybenzoïque, au sein d'un solvant inerte tel qu'un mélange de tetrahydrofuranne ou de diméthylformamide et d'eau dans le premier cas ou le dichlorométhane dans le deuxième cas, à une température comprise entre -20°C et la température ambiante.

La réaction (c) peut s'effectuer en présence d'hydrazine, au sein d'un solvant inerte tel qu'un alcool (éthanol de préférence) à une température comprise entre 20°C et la température d'ébullition du milieu réactionnel.

La réaction de cyclisation (d) peut s'effectuer en présence d'un acide minéral tel que l'acide chlorhydrique concentré ou l'acide sulfurique concentré, au sein d'un solvant inerte tel qu'un alcool (éthanol de préférence) à une température comprise entre 20°C et la température d'ébullition du milieu réactionnel.

Les composés de formule (Ib) dans lesquels R1 représente NHCO(R2) où R2 représente un groupement phényle substitué par un groupement de type - CH2NR6R7, R6 et R7 étant indépendamment sélectionnés dans le groupe constitué par -H, (C1-C6)alkyle, aryle, hétéroaryle, -(C1-C6)alkyl-aryle, -(C1-C6)alkyl-hétéroaryle, éventuellement substitués, ou bien (R6 et R7) étant liés entre eux pour former un hétérocycle mono ou bicyclique, saturé ou insaturé, comprenant de 2 à 10 chaînons et de 1 à 5 hétéroatomes choisis parmi N, O, et S, peuvent être préparés à partir des composés de formule générale (IVb), selon le schéma de synthèse général suivant:

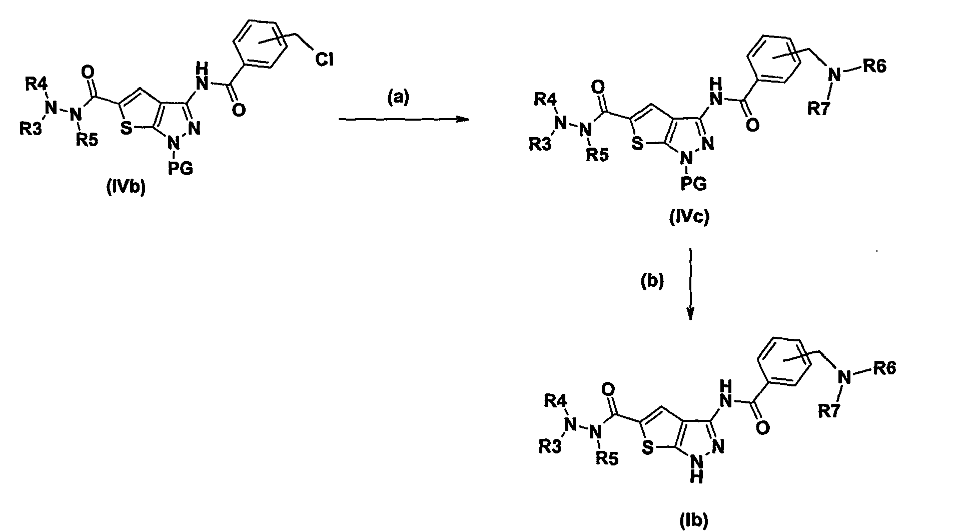

La réaction (a) peut s'effectuer en présence d'une amine de type HNR6R7, en présence d'un sel tel que l'iodure de tetrabutylammonium au sein d'un solvant inerte tel que le diméthylformamide, à une température comprise entre 20°C et la température d'ébullition du milieu réactionnel.

La réaction de déprotection (b) peut s'effectuer (lorsque PG représente un groupement *tert*-butyloxycarbonyle) en présence d'iodotriméthylsilane, ou en milieu acide (acide trifluoroacétique, ou acide chlorhydrique dans un solvant tel que le dichlorométhane ou le dioxanne par exemple) à une température comprise entre 0°C et la température d'ébullition du milieu réactionnel, ou en milieu basique (carbonate de potassium au sein d'un solvant tel qu'un alcool (méthanol de préférence) à une température comprise entre 0°C et la température d'ébullition du milieu réactionnel et éventuellement sous irradiation par des micro-ondes), ou en milieu neutre dans un solvant tel qu'un alcool (méthanol de préférence) sous irradiation par des micro-ondes; ou bien (lorsque PG représente un groupement 1-éthoxy-éthyle) en présence d'un acide minéral tel que l'acide chlorhydrique, au sein d'un solvant tel que le tétrahydrofuranne ou l'eau, à une température comprise entre 20°C et la température d'ébullition du milieu réactionnel, ou encore selon les méthodes bien connues de déprotection de la fonction amine (T. W. GREENE et coll. dans Protective Groups in Organic Synthesis, third edition, 1999, Wiley-Interscience.

Les dérivés de type (R3)(R4)NN(R5)H dans lesquels R3 représente un groupement Aryle et plus particulièrement un groupement phényle substitué, R4 représente un groupement un groupement alkyle, R5 représente un atome d'hydrogène, peuvent être préparés à partir des composés de formule générale (XXI), selon le schéma de synthèse général suivant:

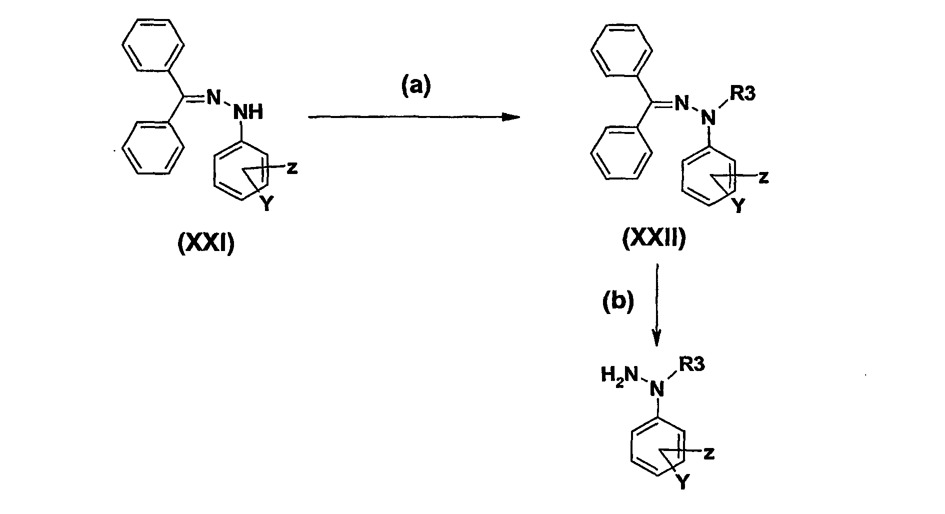

La réaction (a) peut s'effectuer en présence d'une base telle qu'un organolithien, en présence d'un halogénure d'alkyle, au sein d'un solvant inerte tel que le tetrahydrofuranne, à une température comprise entre -80°C 25°C.

La réaction de clivage (b) peut s'effectuer en présence d'un acide minéral tel que l'acide chlorhydrique, au sein d'un solvant inerte tel que le tetrahydrofuranne, à une température comprise entre 25°C et la température d'ébullition du milieu réactionnel.

Les composés de formule générale (XXI) dans lesquels Y et Z représentent indépendemment et plus particulièrement un atome d'hydrogène, ou un groupement halogène ou alkyle, peuvent être préparés à partir des composés de formule générale (XXIII), selon le schéma de synthèse général suivant:

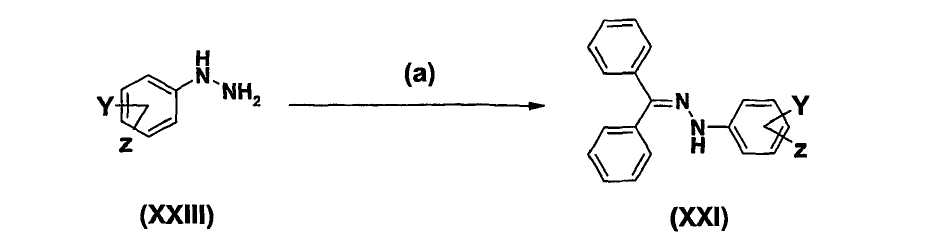

La réaction (a) peut s'effectuer en présence de benzophénone, en présence d'un acide minéral tel que l'acide sulfurique ou l'acide chlorhydrique, au sein d'un solvant inerte tel qu'un alcool, l'éthanol de préférence, à une température comprise entre 25°C et la température d'ébullition du milieu réactionnel.

Alternativement, les composés de formule générale (XXI) dans lesquels Y et Z représentent indépendemment et plus particulièrement un atome d'hydrogène, ou un groupement halogène ou alkyle, peuvent être préparés à partir des composés de formule générale (XXIV), selon le schéma de synthèse général suivant:

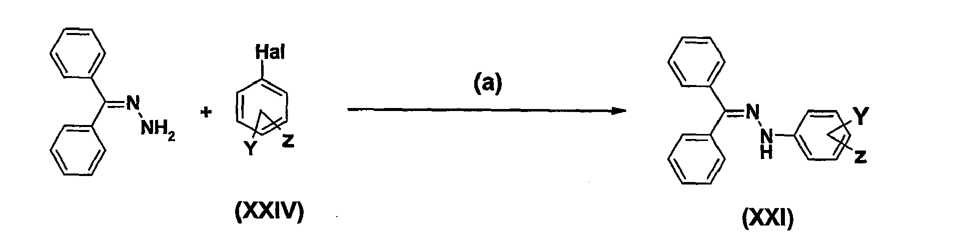

La réaction (a) peut s'effectuer en présence de benzhydrylidène-hydrazine, d'un composé de formule générale (XXIV) dans lequel Y et Z représentent indépendemment et plus particulièrement un atome d'hydrogène, ou un groupement halogène ou alkyle et où Hal représente plus particulièrement un atome de brome ou de chlore, d'un dérivé de palladium (II) tel que le diacétate de palladium (II), d'un ligand phosphine, d'une base telle que la soude, au sein d'un solvant inerte tel qu'un alcool (alcool *tert*-amylique de préférence) à une température comprise entre 20°C et la température d'ébullition du milieu réactionnel, selon les méthodes générales décrites par J.-M. CAMPAGNE et al., Tetrahedron, 2002, 58, 2041.

Il est entendu pour l'homme du métier que, pour la mise en oeuvre des procédés selon l'invention décrits précédemment, il peut être nécessaire d'introduire des groupements protecteurs des fonctions amine et carboxyle afin d'éviter des réactions secondaires. Ces groupes sont ceux qui permettent d'être éliminés sans toucher au reste de la molécule. Comme exemples de groupes protecteurs de la fonction amine, on peut citer le 1-éthoxy-éthyle qui peut être éliminé en présence en présence d'un acide minéral tel que l'acide chlorhydrique (au sein d'un solvant tel que le tétrahydrofuranne ou l'eau par exemple), le carbamate de *tert*-butyle qui peut être éliminé au moyen d'iodotriméthylsilane ou en milieu acide (acide trifluoroacétique, ou acide chlorhydrique dans un solvant tel que le dichlorométhane ou le dioxanne par exemple), le carbamate de benzyle qui peut être éliminé en présence d'hydrogène ou en présence d'un mélange d'un thiol (benzènethiol par exemple) et d'un acide de Lewis (éthérate de trifluorure de bore par exemple), l'acétyle qui peut être éliminé en milieu acide (acide chlorhydrique par exemple), le benzoyle qui peut être éliminé en milieu acide (acide chlorhydrique par exemple), le 2-triméthylsilanyl-éthoxyméthyle qui peut être éliminé en présence de fluorure de tétrabutylammonium ou en milieu acide par exemple (acide chlorhydrique par exemple). Comme groupes protecteurs de la fonction carboxyle, on peut citer les esters (méthoxyméthylester, benzylester, méthylester par exemple) qui peuvent être éliminé par les méthodes décrites par T. W. GREENE et coll. dans Protective Groups in Organic Synthesis, third edition, 1999, Wiley-Interscience.

Les composés de formule (I) sont isolés et peuvent être purifiés par les méthodes connues habituelles, par exemple par cristallisation, chromatographie ou extraction.

Les énantiomères, diastéréoisomères des composés de formule (I) font également partie de l'invention.

Les composés de formule (I) comportant un reste basique peuvent être éventuellement transformés en sels d'addition avec un sel minéral ou organique par action d'un tel acide au sein d'un solvant organique tel un alcool, une cétone, un éther ou un solvant chloré.

Les composés de formule (I) comportant un reste acide peuvent être éventuellement transformés en sels métalliques ou en sels d'addition avec des bases azotées selon les méthodes connues en soi. Ces sels peuvent être obtenus par action d'une base métallique (alcaline ou alcalinoterreuse par exemple), de l'ammoniac, d'une amine ou d'un sel d'amine sur un composé de formule (I), dans un solvant. Le sel formé est séparé par les méthodes habituelles.

Ces sels font également partie de l'invention.

Lorsqu'un produit selon l'invention présente au moins une fonction basique libre, des sels pharmaceutiquement acceptables peuvent être préparés par réaction entre ledit produit et un acide minéral ou organique. Des sels pharmaceutiquement acceptables incluent les chlorures, nitrates, sulfates, hydrogénosulfates, pyrosulfates, bisulfates, sulfites, bisulfites, phosphates, monohydrogénophosphates, dihydrogénophosphates, métaphosphates, pyrophosphates, acétates, propionates, acrylates, 4-hydroxybutyrates, caprylates, caproates, décanoates, oxalates, malonates, succinates, glutarates, adipates, pimélates, maléates, fumarates, citrates, tartrates, lactates, phénylacétates, mandélates, sébacates, subérates, benzoates, phtalates, méthanesulfonates, propanesulfonates, xylènesulfonates, salicylates, cinnamates, glutamates, aspartates, glucuronates, galacturonates.

Lorsqu'un produit selon l'invention présente au moins une fonction acide libre, des sels pharmaceutiquement acceptables peuvent être préparés par réaction entre ledit produit et une base minérale ou organique. Des bases pharmaceutiquement acceptables incluent des hydroxydes de cations de métaux alcalins ou alcalino-terreux tels que Li, Na, K, Mg, Ca, des composés aminés basiques tels qu'ammoniac, arginine, lysine histidine, pipéridine, morpholine, pipérazine, triéthylamine.

L'invention est également décrite par les exemples suivants, donnés à titre d'illustration de l'invention.

### Exemple 1: Préparation du 5-(N'-phényl-hydrazinocarbonyl)-1H-thiéno[2,3-c]pyrazol-3-yl]-4-méthoxy-benzamide.

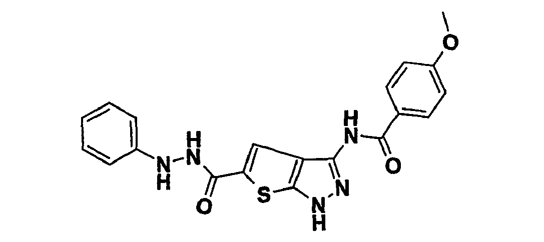

Dans un ballon de 20 mL sous argon sont introduits successivement l'acide 1-(1-éthoxy-éthyl)-3-(4-méthoxy-benzoylamino)-1 H-thiéno[2,3-c]pyrazole-5-carboxylique (0,3 g, 0,77 mmol), le tetrafluoroborate de O-(1H-benzotriazol-1-yl)-N,N,N',N'-tetraméthyluronium (TBTU) (0,247 g, 0,77 mmol, 1 éq), le diméthylformamide (3,85 mL) et la phénylhydrazine (0,151 mL, 1,54 mmol, 2 éq). Au mélange réactionnel est ajouté la triéthylamine (0,322 mL, 2,31 mmol, 3 éq). Le mélange est agité à une température voisine de 60°C pendant 15 heures. Le solvant est ensuite évaporé sous vide et l'huile résiduelle est diluée avec 15 mL d'acétate d'éthyle et 10 mL d'eau. La phase aqueuse est isolée puis acidifiée à pH 4-5 avant d'être extraite avec de l'acétate d'éthyle (3 x 15 mL). Les phases organiques réunies sont séchées sur sulfate de magnésium et concentrées à sec sous pression réduite. Le résidu est purifié par flash-chromatographie sur gel de silice avec comme éluant un mélange 1 : 1 acétate d'éthyle /heptane. Le produit obtenu sous forme d'huile (0,310 g, 84%, Rf = 0,47 (1 :9 MeOH/dichlorométhane)) est placé sans autre purification dans 5 mL de tetrahydrofuranne et 2,5 mL d'acide chlorhydrique 2,5 N. Le mélange réactionnel est alors chauffé à une température voisine de 50°C pendant 2 heures puis est refroidi à température ambiante. Au mélange sont alors ajoutés 15 mL d'eau. Un précipité se forme. Le tetrahydrofuranne est évaporé et la phase aqueuse est neutralisée avec NaOH 5N. Le précipité formé est filtré puis lavé avec de l'eau. Le solide beige est séché dans une étuve à une température voisine de 60 °C sous vide. Le solide sec est ensuite dissous dans l'acétone et précipité avec de l'heptane. Le 5-(N'-phényl-hydrazinocarbonyl)-1 H-thiéno[2,3-c]pyrazol-3-yl]-4-méthoxy-benzamide ainsi obtenu (86 mg, 33%) possède une pureté de 95 %. Rdt global = 28%. RMN ¹H (400 MHz, (CD₃)₂SO d₆, δ en ppm) : 3,85 (s, 3H) ; 6,72 (t large, J = 7,5 Hz, 1 H) ; 6,76 (d large, J = 8,0 Hz, 2H) ; 7,05 (d large, J = 8,0 Hz, 2H) ; 7,15 (t large, J = 7,5 Hz, 2H) ; 7,85 (s large, 1 H) ; 8,09 (d large, J = 8,0 Hz, 2H) ; 8,19 (s large, 1 H) ; 10,45 (m large, 1 H) ; 11,0 (m large, 1 H) ; 12,9 (m large, 1 H) . LC/MS: m = 407, ES m/z = 408 MH⁺

### Acide 1-(1-Ethoxy-éthyl)-3-(4-méthoxy-benzoylamino)-1 H-thiéno[2,3-c]pyrazol-5-carboxylique:

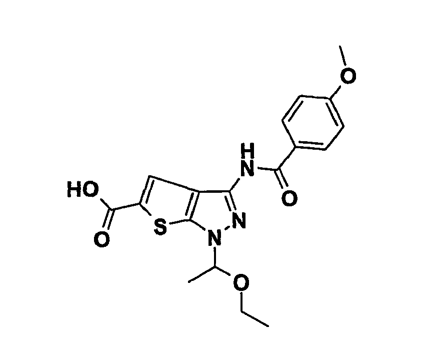

Dans un ballon de 500 mL sous argon sont introduits du 3-bromo-1-(1-éthoxy-éthyl)-1H-thiéno[2,3-c]pyrazole-5-carboxylate d'éthyle (10 g, 28 mmol), du 4-méthoxy-benzamide (7,39 g, 48 mmol, 1,7 éq), de l'iodure de cuivre (I) (0,548 g, 2,88 mmol, 0,1 éq), du phosphate tripotassique (18,34 g, 86 mmol, 3 éq) préalablement pulvérisé, et 180 mL de dioxanne anhydre préalablement dégazé à l'argon. A cette suspension est ajouté à la seringue 0,306 mL (2,88 mmol, 0,1 éq) de N,N'-diméthyléthylènediamine. Le mélange réactionnel est alors chauffé à une température voisine de 110°C pour une période de 24 heures. Alors, le dioxanne est évaporé sous vide et le résidu est dilué avec de l'acétate d'éthyle (300 mL) et 150 mL d'eau. La phase organique est lavée 1 fois avec 100 mL d'eau, puis elle est séchée sur sulfate de magnésium et concentrée à sec sous pression réduite. Le résidu est purifié par chromatographie sur gel de silice avec comme éluant un mélange acétate d'éthyle/heptane (1: 1). L'huile jaune pâle obtenue après purification correspond au 1-(1-éthoxy-éthyl)-3-(4-méthoxy-benzoylamino)-1H-thiéno[2,3-c]pyrazole-5-carboxylate d'éthyle (7,41 g, 64%). Les phases aqueuses réunies sont acidifiées avec de l'acide chlorhydrique 5N jusqu'à un pH=5-6. Un fort précipité apparaît, celui-ci est extrait avec (2 x 100 mL) d'acétate d'éthyle. La phase organique est à son tour séchée sur sulfate de magnésium et concentrée à sec sous pression réduite. Le solide blanc obtenu après évaporation est trituré dans un mélange heptane/éther diéthylique (1 :1). On obtient après filtration, l'acide 1-(1-éthoxy-éthyl)-3-(4-méthoxy-benzoylamino)-1H-thiéno[2,3-c]pyrazole-5-carboxylique sous forme d'un solide blanc (1,27g, 11 %). Le 1-(1-éthoxy-éthyl)-3-(4-méthoxy-benzoylamino)-1 H-thiéno[2,3-c]pyrazole-5-carboxylate d'éthyle est soumis à une saponification en utilisant 50 mL de tetrahydrofuranne et 20 mL d'hydroxyde de sodium 5N à une température voisine de 50 °C pendant 5 heures. Le tetrahydrofuranne est alors évaporé, la phase aqueuse est diluée avec 150 mL d'eau et le pH est ajusté à 3-4 avec de l'acide chlorhydrique 5N. Le précipité blanc obtenu est filtré et lavé avec de l'eau, puis séché sous vide à une température voisine de 60 °C. On obtient ainsi 7,04 g d'acide 1-(1-éthoxy-éthyl)-3-(4-méthoxy-benzoylamino)-1H-thiéno[2,3-c]pyrazole-5-carboxylique soit 98% qui est utilisé directement dans l'étape suivante.

### 3-Bromo-1-(1-éthoxy-éthyl)-1 H-thiéno[2,3-c]pyrazole-5-carboxylate d'éthyle:

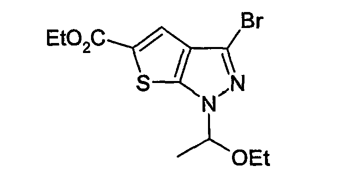

Dans 50 mL d'éthanol sous atmosphère d'argon à une température voisine de 20°C, on introduit sous agitation 1,19 g (3,81 mmol) de 3,5-dibromo-1-(1-éthoxy-éthyl)-1H-pyrazole-4-carboxaldéhyde, puis l'on ajoute 0,4 g (3,81 mmol) de carbonate de sodium et 0,42 mL (3,81 mmol) de 2-mercaptoacétate d'éthyle. Le mélange réactionnel est ensuite porté à reflux pendant 2 heures, puis il est concentré à sec sous pression réduite (2,7 kPa). Le résidu est repris dans 60 mL d'eau et 60 mL de dichlorométhane et décanté. La phase organique est séchée sur sulfate de magnésium puis concentrée à sec sous pression réduite (2,7 kPa). On obtient ainsi 1,17 g de 3-bromo-1-(1-éthoxy-éthyl)-1 H-thiéno[2,3-c]pyrazole-5-carboxylate d'éthyle sous forme d'une huile jaune claire qui cristallise donnant des cristaux crèmes fondant à 69°C.

### 3,5-Dibromo-1-(1-éthoxy-éthyl)-1 H-pyrazole-4-carboxaldéhyde:

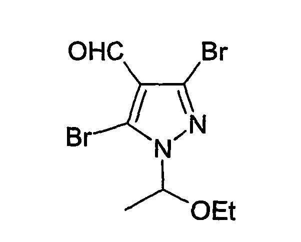

Dans 30 mL de toluène à une température voisine de 20°C, on introduit sous agitation 1 g (3,94 mmol) de 3,5-dibromo-1H-pyrazole-4-carboxaldéhyde, 1,5 mL (16 mmol) d'éthylvinyl éther et 3 gouttes d'acide chlorhydrique concentré 12N. Le mélange réactionnel est agité pendant 15 heures à une température voisine de 20°C, puis l'on coule 1,5 mL (16 mmol) d'éthylvinyl éther et l'agitation est poursuivie pendant 15 heures à une température voisine de 20°C. Le mélange réactionnel est ensuite dilué par 20 mL de toluène et lavé par 2 fois 30 mL d'une solution saturée d'hydrogénocarbonate de sodium. La phase organique est séchée sur sulfate de magnésium et concentrée à sec sous pression réduite (2,7 kPa) pour donner 1,19 g de 3,5-dibromo-1-(1-éthoxy-éthyl)-1H-pyrazole-4-carboxaldéhyde sous forme d'une huile jaune.
Spectre R.M.N. ¹H (300 MHz, (CD₃)₂SO d6, δ en ppm) : 1,10 (t, J = 7,5 Hz : 3H); 1,64 (d, J = 6,5 Hz: 3H); 3,31 (mt: 1H); 3,51 (mt: 1H); 5,88 (q, J = 6,5 Hz : 1H) ; 9,73 (s : 1H).

### 3,5-Dibromo-1 H-pyrazole-4-carboxaldéhyde:

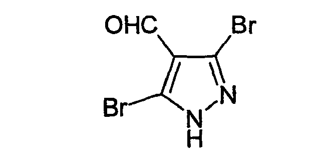

Dans 1500 mL d'éther diéthylique à une température voisine de 20°C et sous atmosphère d'argon, on introduit sous agitation 81,7 g (0,268 mol) de 3,4,5-tribromopyrazole. Le mélange est refroidi à une température voisine de - 78°C, puis sont coulés goutte à goutte 335 mL (0,536 mol) d'une solution de *n*-butyllithium 1,6 M dans l'hexane en 3h15. Le mélange réactionnel est agité pendant 1,5 heures à une température voisine de -75°C, puis on coule au goutte à goutte 100 mL (1,34 mol) de diméthylformamide en maintenant la température inférieure à -70°C. On laisse encore agiter pendant 2 heures à une température voisine de -75°C, puis pendant 15 heures à une température voisine de 20°C. Le milieu réactionnel est ensuite refroidi dans un bain glace-eau et on coule 1000 mL d'eau. Après décantation, la phase aqueuse est extraite par 500 mL d'éther diéthylique et par 3 fois 500 mL d'acétate d'éthyle. La phase aqueuse est ensuite acidifiée par une solution d'acide citrique à pH=3 (on observe la formation d'un précipité) et extraite par 2 fois 1000 mL d'éther diéthylique. La phase organique est séchée sur sulfate de magnésium, concentrée à sec sous pression réduite (2,7 kPa) et le solide jaune obtenu est repris par 300 mL d'eau et agité pendant 2 heures à une température voisine de 20°C. Le mélange est ensuite filtré et le solide est lavé par 2 fois 50 mL d'eau, séché sous hotte ventilée, puis séché sous pression réduite (2,7 kPa) à une température voisine de 40°C. On obtient ainsi 51,3 g de 3,5-dibromo-1 H-pyrazole-4-carboxaldéhyde sous forme d'une poudre jaune fondant à 173°C.

### Exemple 2: Préparation du 5-(N"-méthyl-N'-phényl-hydrazinocarbonyl)-1H-thiéno[2,3-c]pyrazol-3-yl]-4-méthoxy-benzamide.

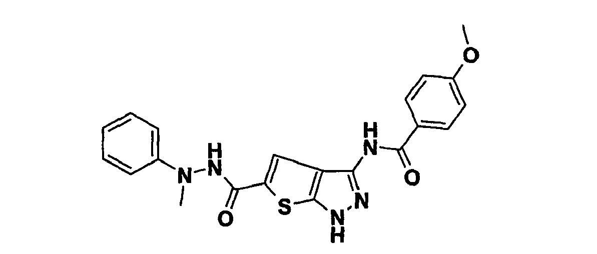

Le 5-(N'-méthyl-N'-phényl-hydrazinocarbonyl)-1 H-thiéno[2,3-c]pyrazol-3-yl]-4-méthoxy-benzamide est préparé selon l'exemple 1. Rdt = 36%; RMN ¹H (300 MHz, (CD₃)₂SO d₆, δ en ppm) : 3,17 (s, 3H) ; 3,85 (s, 3H) ; 6,77 (t large, J = 7,5 Hz, 1H) ; 6,82 (d large, J = 8,5 Hz, 2H) ; 7,07 (d large, J = 8,0 Hz, 2H) ; 7,22 (t large, J = 8,0 Hz, 2H) ; 8,07 (d large, J = 8,5 Hz, 2H) ; 8,17 (s large, 1 H) ; 10,75 (m large, 1 H) ; 11,0 (m étalé, 1 H) ; 12,9 (m étalé, 1 H) .LC/MS m = 421, IE m/z = 421 M⁺-, m/z = 300(M - C₈H₇O₂)⁺, m/z = 135C₈H₇O₂ ⁺

### Exemple 3: Préparation du N-[5-(N'-Cyclohexyl-hydrazinocarbonyl)-1H-thiéno[2,3-c]pyrazol-3-yl]-4-méthoxy-benzamide.

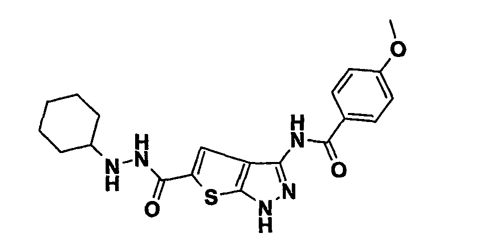

Le N-[5-(N'-cyclohexyl-hydrazinocarbonyl)-1 H-thiéno[2,3-c]pyrazol-3-yl]-4-méthoxy-benzamide est préparé selon l'exemple 1. Rdt = 11%. RMN ¹H (400 MHz, (CD₃)₂SO d₆, δ en ppm) : 1,01 à 1,26 (m, 5H) ; 1,55 (m, 1 H) ; de 1,63 à 1,90 (m, 4H) ; 2,73 (m, 1 H) ; 3,85 (s, 3H) ; 4,84 (m, 1 H) ; 7,06 (d large, J = 8,5 Hz, 2H) ; 8,02 (s large, 1H); 8,07 (d large, J = 8,5 Hz, 2H) ; 10,05 (s large, 1 H) ; 10,9 (s large, 1 H) ; 12,85 (s large, 1H). LC/MS : m = 413 ; ES m/z = 414 MH⁺

### Exemple 4: Préparation du N-[5-(N'-Benzyl-N'-phényl-hydrazinocarbonyl)-1 H-thiéno[2,3-c]pyrazol-3-yl]-4-méthoxy-benzamide.

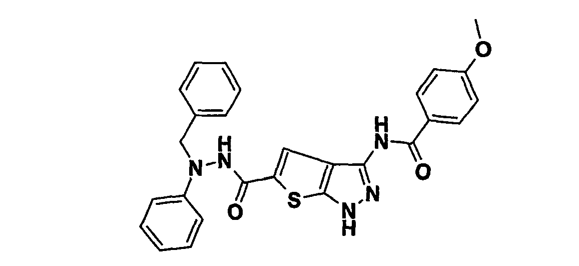

Le N-[5-(N'-benzyl-N'-phényl-hydrazinocarbonyl)-1 H-thiéno[2,3-c]pyrazol-3-yl]-4-méthoxy-benzamide est préparé selon l'exemple 1. Rdt = 7%. SM: ES m/z=498 MH+. RMN ¹H (400 MHz, (CD₃)₂SO d₆, δ en ppm) : 3,85 (s, 3H) ; 4,75 (s large, 2H) ; 6,75 (t large, J = 7,5 Hz, 1 H) ; 6,92 (d large, J = 8,5 Hz, 2H) ; 7,07 (d large, J = 8,5 Hz, 2H) ; de 7,15 à 7,28 (m, 3H) ; 7,34 (t large, J = 7,5 Hz, 2H) ; 7,49 (d large, J = 7,5 Hz, 2H) ; 8,07 (d large, J = 8,5 Hz, 2H) ; 8,17 (s large, 1 H) ; 10,85 (s large, 1 H) ; 11,05 (m étalé, 1 H) ; de 12,75 à 13,1 (m très étalé, 1 H). LC/MS : m = 497 ; ES m/z = 498 MH⁺

### Exemple 5 : Préparation du N-{5-[N'-(2-Ethyl-phényl)-hydrazinocarbonyl]-1 H-thiéno[2,3-c]pyrazol-3-yl}-4-méthoxy-benzamide.

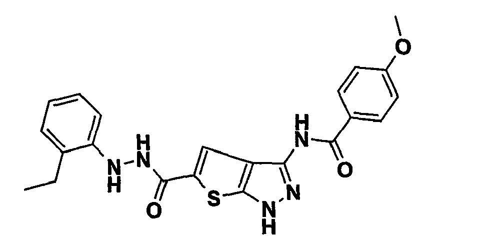

Le N-{5-[N'-(2-éthyl-phényl)-hydrazinocarbonyl]-1H-thiéno[2,3-c]pyrazol-3-yl}-4-méthoxy-benzamide est préparé selon l'exemple 1. Rdt = 60%. SM: ES m/z=436 MH+. RMN ¹H (400 MHz, (CD₃)₂SO d₆, δ en ppm) : 1,19 (t, J = 7,5 Hz, 3H) ; 2,59 (q, J = 7,5 Hz, 2H) ; 3,85 (s, 3H) ; de 6,68 à 6,77 (m, 2H) ; de 6,98 à 7,11 (m, 4H) ; 7,26 (s large, 1H) ; 8,08 (d large, J = 8,5 Hz, 2H) ; 8,19 (s large, 1 H) ; 10,5 (m étalé, 1 H) ; de 10,7 à 11,3 (m très étalé, 1 H) ; de 12,5 à 13,2 (m très étalé, 1 H). LC/MS : m = 435, ES m/z = 436 MH⁺

### Exemple 6 : Préparation du N-{5-[N'-(2-Fluoro-phényl)-hydrazinocarbonyl]-1 H-thiéno[2,3-c]pyrazol-3-yl}-4-méthoxy-benzamide.

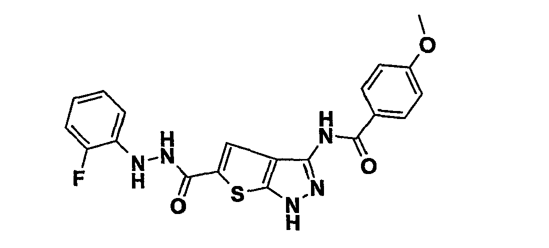

Le N-{5-[N'-(2-fluoro-phényl)-hydrazinocarbonyl]-1 H-thiéno[2,3-c]pyrazol-3-yl}-4-méthoxy-benzamide est préparé selon l'exemple 1. Rdt = 65%. RMN ¹H (400 MHz, (CD₃)₂SO d₆, δ en ppm) : 3,85 (s, 3H) ; 6,73 (m, 1 H) ; 6,82 (t large, J = 8,0 Hz, 1 H) ; de 6,96 à 7,13 (m, 4H) ; 7,78 (s large, 1 H) ; 8,09 (d large, J = 8,5 Hz, 2H) ; 8,21 (s large, 1 H) ; 10,5 (s large, 1H) ; 11,0 (s large, 1 H) ; 12,9 (s large, 1 H). LC/MS : m = 425, IC m/z = 426 MH⁺

### Exemple 7: Préparation du 4-Méthoxy-N-[5-(N'-o-tolyl-hydrazinocarbonyl)-1H-thiéno[2,3-c]pyrazol-3-yl]-benzamide.

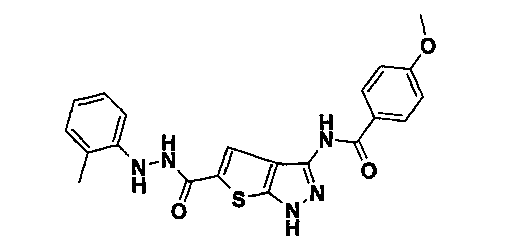

Le 4-méthoxy-N-[5-(N'-*o*-tolyl-hydrazinocarbonyl)-1H-thiéno[2,3-c]pyrazol-3-yl]-benzamide est préparé selon l'exemple 1. Rdt = 70%. RMN ¹H (400 MHz, (CD₃)₂SO d₆, δ en ppm) : 2,19 (s, 3H) ; 3,85 (s, 3H) ; de 6,63 à 6,74 (m, 2H) ; 7,02 (m large, 2H) ; 7,08 (d large, J = 8,5 Hz, 2H) ; 7,25 (s large, 1 H) ; 8,08 (d large, J = 8,5 Hz, 2H) ; 8,19 (s large, 1 H) ; 10,5 (m étalé, 1H) ; de 10,7 à 11,5 (m très étalé, 1H) ; de 12,3 à 13,3 (m très étalé, 1H). LC/MS : m = 421, ES m/z = 422 MH⁺

### Exemple 8: Préparation du N-[5-(N'-Méthyl-N'-phényl-hydrazinocarbonyl)-1 H-thiéno[2,3-c]pyrazol-3-yl]-4-morpholin-4-ylméthyl-benzamide

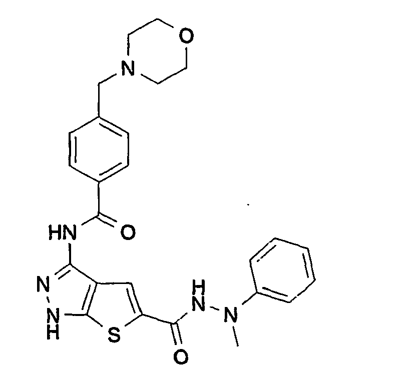

A une solution de 0,09 g (154 µmol) de 5-(N'-méthyl-N'-phényl-hydrazinocarbonyl)-3-(4-morpholin-4-ylméthyl-benzoylamino)-thiéno[2,3-c]pyrazole-1-carboxylate de *tert*-butyle dans 5 mL de méthanol dans un tube pour four à micro-ondes, sont ajoutés 21 mg (154 µmol) de carbonate de potassium. Le tube est bouché et le mélange réactionnel est irradié à une température voisine de 180°C pendant 3 minutes. Le mélange réactionnel est ensuite concentré à sec sous vide (0,6 kPa) à une température voisine de 30°C. Le résidu est purifié par chromatographie sur une cartouche de gel de silice de 25 g (0,015-0,040 mm) en éluant avec un mélange dichlorométhane/ méthanol (99/1 en volumes, puis 98/2 et enfin 97/3) à un débit de 10 mL/min. Les fractions contenant le produit attendu sont réunies puis concentrées à sec sous pression réduite (1,0 kPa) à une température voisine de 30°C. Le résidu est repris dans 10 mL d'éther diisopropylique, trituré, puis reconcentré à sec. Le solide obtenu est séché à l'étuve sous vide (0,01 kPa) à une température voisine de 60°C. On obtient ainsi 40 mg de N-[5-(N'-méthyl-N'-phényl-hydrazinocarbonyl)-1 H-thiéno[2,3-c]pyrazol-3-yl]-4-morpholin-4-ylméthyl-benzamide sous forme d'un solide blanc cassé fondant à 242°C. SM-EI : 490(+)=M(+).

### 5-(N'-Méthyl-N'-phényl-hydrazinocarbonyl)-3-(4-morpholin-4-ylméthyl-benzoylamino)-thiéno[2,3-c]pyrazole-1-carboxylate de tert-butyle:

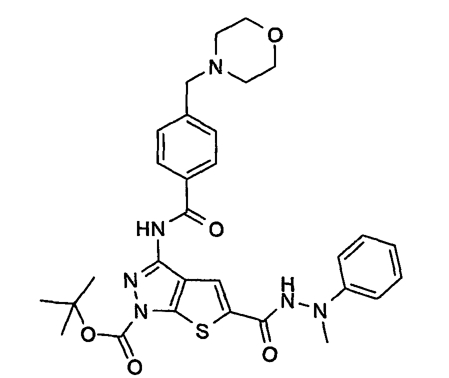

A une solution de 0,27 g (0,70 mmol) de 3-amino-5-(N'-méthyl-N'-phényl-hydrazinocarbonyl)-thiéno[2,3-c]pyrazole-1-carboxylate de *tert*-butyle dans 20 mL de tetrahydrofuranne sous argon, sont ajoutés 193 mg (1,39 mmol) de carbonate de potassium, suivis de 240 mg (0,87 mmol) de chlorure de 4-morpholin-4-ylméthyl-benzoyle; chlorhydrate. Le mélange réactionnel est agité à une température voisine de 25°C pendant 2 heures, puis à reflux pendant 4 heures. Le mélange réactionnel est ensuite refroidi à une température voisine de 25°C, puis traité avec 40 mL d'eau glacée. Le mélange est extrait successivement avec 60 mL, puis 2 fois 40 mL d'acétate d'éthyle. Les phases organiques sont réunies, lavées 2 fois avec 40 mL de saumure saturée, séchées sur sulfate de magnésium, filtrées, puis concentrées à sec sous vide (2 kPa) à une température voisine de 40°C. Le résidu est purifié par chromatographie sur une cartouche de gel de silice de 25 g (0,015-0,040 mm) en éluant avec successivement avec du dichlorométhane pur, puis par des mélanges dichlorométhane/ méthanol (99/1 en volumes, puis 98,5/1,5, 98/2 et enfin 97/3) à un débit de 10 mL/min. Les fractions contenant le produit attendu sont réunies puis concentrées à sec sous pression réduite (0,6 kPa) à une température voisine de 30°C. On obtient ainsi 0,1 g de N-[5-(N'-méthyl-N'-phényl-hydrazinocarbonyl)-1 H-thiéno[2,3-c]pyrazol-3-yl]-4-morpholin-4-ylméthyl-benzamide sous forme d'un solide blanc cassé fondant à 190°C.
Le chlorure de 4-morpholin-4-ylméthyl-benzoyle; chlorhydrate peut être préparé selon les brevets US 4,623,486 A (1986) et WO 9008128 A1.

### 3-Amino-5-(N'-méthyl-N'-phényl-hydrazinocarbonyl)-thiéno[2,3-c]pyrazole-1-carboxylate de tert-butyle:

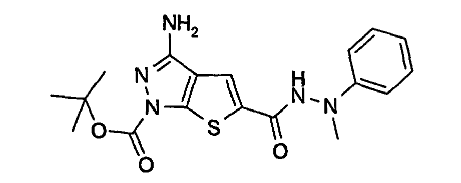

A une suspension de 1,19 g (4,14 mmol) de N'-méthyl-N'-phényl-(3-amino-1 H-thiéno[2,3-c]pyrazole)-5-carbo-hydrazide dans 35 mL de dichlorométhane sous argon, est ajouté 0,58 mL (4,14 mmol) de triéthylamine. Le mélange réactionnel est agité à une température voisine de 25°C pendant 15 minutes, puis 61 mg (0,50 mmol) de 4-diméthylamino-pyridine sont ajoutés. Le mélange réactionnel est ensuite refroidi à une température voisine de 0°C, et une solution de 0,90 g (4,14 mmol) de di-*tert-*butyl-carbonate dans 15 mL de dichlorométhane est ajoutée au goutte à goutte. Le mélange réactionnel est ensuite agité à une température voisine de 0°C pendant une heure, puis à une température voisine de 25°C pendant 3 heures. Il est alors traité avec 50 mL d'eau, agité à une température voisine de 25°C, puis décanté. La phase aqueuse est extraite deux fois avec 30 mL de dichlorométhane. Les phases organiques sont réunies, lavées à l'eau jusqu'à pH neutre (2 fois 50 mL), séchées sur sulfate de magnésium, filtrées, puis concentrées à sec sous vide (2 kPa) à une température voisine de 30°C. Le résidu est purifié par chromatographie sur une cartouche de gel de silice de 70 g (0,015-0,040 mm) en éluant avec un mélange dichlorométhane/ méthanol (98/2 en volumes), à un débit de 20 mL/min. Les fractions contenant le produit attendu sont réunies puis concentrées à sec sous pression réduite (2 kPa) à une température voisine de 30°C. On obtient ainsi 0,38 g de 3-amino-5-(N'-méthyl-N'-phényl-hydrazinocarbonyl)-thiéno[2,3-c]pyrazole-1-carboxylate de *tert*-butyle sous forme d'un solide beige fondant à 195°C.

### N'-Méthyl-N'-phényl-(3-amino-1 H-thiéno[2,3-c]pyrazole)-5-carbohydrazide:

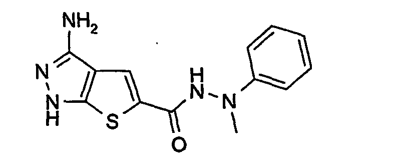

A une solution de 3,9 mL (32,0 mmol) de 1-méthyl-1-phényl-hydrazine dans 180 mL de toluène à une température voisine de 25°C et sous argon, on ajoute au goutte à goutte 17,3 mL (34,5 mmol) de solution de triméthylaluminium 2M dans le toluène en maintenant la température inférieure ou égale à 25°C. Le mélange réactionnel est agité à une température voisine de 25°C pendant 1 heure, puis 2,7 g (11,8 mmol) de 3-amino-1H-thiéno[2,3-c]pyrazole)-5-carboxylate d'éthyle sont ajoutés par spatulées. Le mélange réactionnel est chauffé à reflux pendant 16 heures, puis il est refroidi à une température voisine de 25°C et traité avec 200 mL d'une solution d'acide citrique à 10%. Le mélange est agité à une température voisine de 25°C pendant 30 minutes, puis 200 mL d'acétate d'éthyle sont ajoutés et le mélange est décanté. La phase aqueuse est extraite deux fois avec 150 mL d'acétate d'éthyle. Les extraits organiques sont réunis, lavés à l'eau jusqu'à un pH voisin de 6, séchés sur sulfate de magnésium, filtrés puis concentrés à sec sous vide (2 kPa) à une température voisine de 30°C. Le résidu est purifié par chromatographie sur une colonne de gel de silice de 200 g (0,020-0,040 mm) préalablement neutralisée par élution d'un mélange de dichlorométhane et de triéthylamine (1 volume mort), suivi par élution avec du dichlorométhane pur (4 volumes morts). Les produits sont élués successivement avec des mélanges dichlorométhane/ méthanol (95/5 puis 90/10 en volumes). Les fractions contenant le produit attendu sont réunies puis concentrées à sec sous pression réduite (2 kPa) à une température voisine de 40°C. Le résidu est repris dans 40 mL d'éther diisopropylique, trituré, puis filtré sur verre fritté. Le solide obtenu est séché sous vide. On obtient ainsi 1,5 g de N'-méthyl-N'-phényl-(3-amino-1 H-thiéno[2,3-c]pyrazole)-5-carbo-hydrazide sous forme d'une poudre beige fondant à 186°C.

### 3-Amino-1H-thiéno[2,3-c]pyrazole)-5-carboxylate d'éthyle:

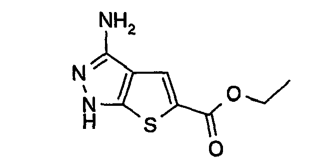

Une suspension de 16,4 g (0,063 mol) de 4-cyano-5-méthylsulfonyl-thiophène-2-carboxylate d'éthyle dans 160 mL d'éthanol absolu est chauffée à reflux jusqu'à dissolution, puis une solution de 6,2 mL (0,126 mol) d'hydrate d'hydrazine dans 40 mL d'éthanol absolu sont ajoutés au goutte à goutte. Le mélange réactionnel est agité à reflux pendant 2 heures, puis 4,6 mL (0,094 mol) d'hydrate d'hydrazine sont ajoutés au goutte à goutte et l'agitation à reflux est poursuivie pendant deux heures. Après refroidissement à une température voisine de 25°C, le mélange est concentré à sec sous vide (2 kPa) à une température voisine de 40°C. Le résidu est repris dans 200 mL d'éthanol et une solution de 13,2 mL (0,158 mol) d'acide chlorhydrique 12 N est additionnée sous agitation à une température voisine de 25°C. Le mélange réactionnel est agité à une température voisine de 25°C pendant 4 heures, puis il est versé lentement dans 500 mL d'une solution saturée d'hydrogénocarbonate de sodium préalablement refroidie à 10°C. Après agitation pendant 30 minutes, le mélange est extrait 3 fois avec 600 mL d'acétate d'éthyle. Les extraits organiques sont réunis, lavés 2 fois avec 300 mL de saumure saturée, séchés sur sulfate de magnésium, filtrés et concentrés à sec sous vide (2 kPa) à une température voisine de 40°C. Le résidu est purifié par chromatographie sur une colonne de gel de silice de 500 g (0,020-0,045 mm) en éluant avec de l'acétate d'éthyle pur. Les fractions contenant le produit attendu sont réunies puis concentrées à sec sous pression réduite (2 kPa) à une température voisine de 40°C. On obtient ainsi 3,4 g de 3-amino-1H-thiéno[2,3-c]pyrazole)-5-carboxylate d'éthyle sous forme d'une poudre marron qui est directement utilisée dans l'étape suivante. SM-EI : 211 (+)=M(+).

### 4-Cyano-5-méthylsulfonyl-thiophène-2-carboxylate d'éthyle:

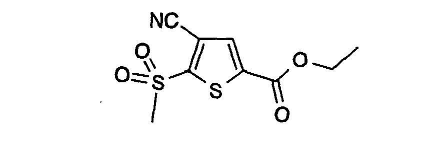

A une solution de 42,5 g (0,187 mol) de 4-cyano-5-méthylsulfanyl-thiophène-2-carboxylate d'éthyle dans 500 mL de dichlorométhane maintenue à une température voisine de -5°C, est ajoutée au goutte à goutte en 2 heures une solution de 92,3 g (0,374 mol) d'acide 3-chloro-peroxybenzoïque dans 1 L de dichlorométhane en maintenant une température voisine de -5°C. Le mélange réactionnel est agité à une température voisine de -5°C pendant 1 heure, puis il est agité une température voisine de 25°C pendant 18 heures. Il est ensuite refroidi dans un bain de glace et traité avec 750 mL d'une solution saturée d'hydrogénocarbonate de sodium et 250 mL de dichlorométhane. Après 30 minutes d'agitation, le mélange est filtré sur verre fritté et le solide est lavé 2 fois avec 100 mL de dichlorométhane. Après décantation du filtrat, la phase aqueuse est extraite avec 500 mL de dichlorométhane. Les extraits organiques sont réunis, lavés 2 fois avec 1 L de saumure saturée, séchés sur sulfate de sodium, filtrés puis concentrés à sec sous vide (1,5 kPa) à une température voisine de 45°C. Le résidu est recristallisé dans 200 mL d'éthanol. Le précipité est filtré sur verre fritté, lavé avec 100 mL d'éthanol, essoré, puis séché dans un dessicateur sous vide (2 kPa). On obtient ainsi 37 g de 4-cyano-5-méthylsulfonyl-thiophène-2-carboxylate d'éthyle sous forme d'une poudre cristalline jaune pâle fondant à 110°C.

### 4-Cyano-5-méthylsulfanyl-thiophène-2-carboxylate d'éthyle:

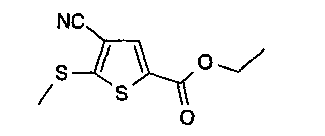

Une solution de 50g (0,21 mol) de 3-amino-4-cyano-5-méthylsulfanyl-thiophène-2-carboxylate d'éthyle dans 500 mL de diméthylformamide sous atmosphère d'argon est chauffée à une température voisine de 60°C, puis on introduit sous agitation une solution de 71,1 mL (0,45 mol) d'isopentylnitrite dans 100 mL de diméthylformamide en maintenant la température entre 65 et 70°C. Le mélange réactionnel est ensuite agité à une température voisine de 60°C pendant 2,5 heures, puis il est refroidi à une température voisine de 25°C et agité pendant 1 heure. Le mélange est ensuite traité avec 1 L d'eau glacée puis il est agité à une température voisine de 0°C pendant 1 heure. Le précipité obtenu est filtré puis lavé successivement 3 fois avec 200 mL d'eau et 3 fois avec 200 mL d'éther de pétrole. On obtient ainsi, après séchage sous pression réduite, 40,5 g (86%) de 4-cyano-5-méthylsulfanyl-thiophène-2-carboxylate d'éthyle sous forme d'une poudre cristalline jaune fondant à 100°C. SM-CI(NH3) : 245(+)=(M+NH4)(+).

### 3-Amino-4-cyano-5-méthylsulfanyl-thiophène-2-carboxylate d'éthyle:

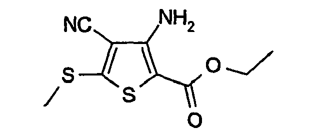

Le 3-amino-4-cyano-5-méthylsulfanyl-thiophène-2-carboxylate d'éthyle peut être préparé par adaptation de la méthode décrite dans Synthesis 2003, 735 à partir de 89,5g de 2-[bis-(méthylsulfanyl)méthylène]malononitrile et en remplaçant le carbonate de potassium par la triéthylamine. On obtient, après séchage, 120,5g (95%) de 3-amino-4-cyano-5-méthylsulfanyl-thiophène-2-carboxylate d'éthyle brut sous forme d'une poudre rose fondant à 149°C.

### 2-[bis-(Méthylsulfanyl)méthylène]malononitrile:

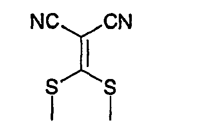

Le 2-[bis-(méthylsulfanyl)méthylène]malononitrile peut-être préparé selon J. Med. Chem. 2003, 46, 1229 ou bien selon Synth. Commun. 2003, 33, 3989.

### Exemple 9: Préparation du 4-Bromo-N-[5-(N'-méthyl-N'-phényl-hydrazinocarbonyl)-1 H-thiéno[2,3-c]pyrazol-3-yl]-benzamide.

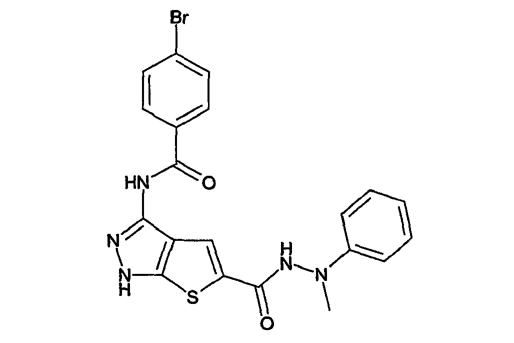

A une solution de 0,04 g (70 µmol) de 3-(4-bromo-benzoylamino)-5-(N'-méthyl-N'-phényl-hydrazinocarbonyl)-thiéno[2,3-c]pyrazole-1-carboxylate de *tert*-butyle dans 3 mL de méthanol dans un tube pour four à micro-ondes, sont ajoutés 14,5 mg (105 µmol) de carbonate de potassium. Le tube est bouché et le mélange réactionnel est irradié à une température voisine de 180°C pendant 3 minutes. Le mélange réactionnel est ensuite concentré à sec sous vide (2 kPa) à une température voisine de 35°C. Le résidu est repris dans le dichlorométhane, puis dans un mélange de 1 mL de méthanol, 4 mL d'eau et 10 mL d'acétate d'éthyle. Après décantation, la phase organique est lavée 2 fois avec 3 mL d'eau, séchée sur sulfate de magnésium, filtrée, puis concentrée à sec sous vide (2 kPa) à une température voisine de 35°C. Le résidu est repris dans l'éther diisopropylique, filtré sur verre fritté, essoré puis séché dans une étuve sous vide (0,01 kPa) à une température voisine de 40°C. Le résidu est purifié par chromatographie sur une colonne de gel de silice de 15 g (0,015-0,040 mm) en éluant avec un mélange dichlorométhane/ méthanol (99/1 en volumes, puis 98/2 et enfin 97/3) à un débit de 20 mL/min. Les fractions contenant le produit attendu sont réunies puis concentrées à sec sous pression réduite (2 kPa) à une température voisine de 35°C. On obtient ainsi 18 mg de 4-bromo-N-[5-(N'-méthyl-N'-phényl-hydrazinocarbonyl)-1H-thiéno[2,3-c]pyrazol-3-yl]-benzamide sous forme d'un solide jaune pâle fondant à 172°C. LC-MS-DAD-ELSD : 468(-)/...=(M-H)(-)/... ; 470(+)/...=(M+H)(+)/... (1 Br present).

### 3-(4-Bromo-benzoylamino)-5-(N'-méthyl-N'-phényl-hydrazinocarbonyl)-thiéno[2,3-c]pyrazole-1-carboxylate de tert-butyle:

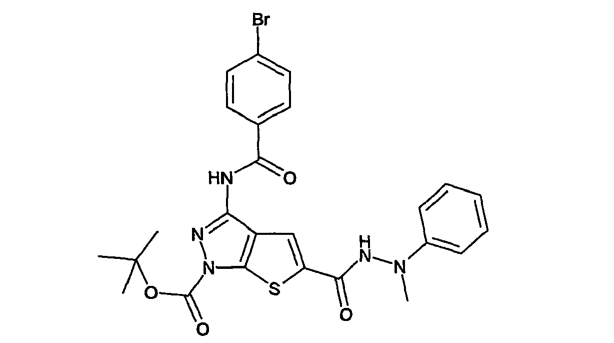

A une solution de 0,27 g (0,70 mmol) de 3-amino-5-(N'-méthyl-N'-phényl-hydrazinocarbonyl)-thiéno[2,3-c]pyrazole-1-carboxylate de *tert*-butyle dans 20 mL de tetrahydrofuranne sous argon, sont ajoutés 169 mg (1,22 mmol) de carbonate de potassium, suivis de 191 mg (0,87 mmol) de chlorure de 4-bromo-benzoyle. Le mélange réactionnel est agité à reflux pendant 2 heures. Le mélange réactionnel est ensuite refroidi à une température voisine de 25°C, puis traité avec 40 mL d'eau glacée. Le mélange est extrait successivement avec 60 mL, puis 2 fois 40 mL d'acétate d'éthyle. Les phases organiques sont réunies, lavées 2 fois avec 40 mL de saumure saturée, séchées sur sulfate de magnésium, filtrées, puis concentrées à sec sous vide (2 kPa) à une température voisine de 35°C. Le résidu est purifié par chromatographie sur une cartouche de gel de silice de 25 g (0,015-0,040 mm) en éluant successivement avec des mélanges dichlorométhane/ méthanol (99/1 en volumes, puis 98/2) à un débit de 13 mL/min. Les fractions contenant le produit attendu sont réunies puis concentrées à sec sous pression réduite (2 kPa) à une température voisine de 35°C. On obtient ainsi 0,04 g de 3-(4-bromo-benzoylamino)-5-(N'-méthyl-N'-phényl-hydrazinocarbonyl)-thiéno[2,3-c]pyrazole-1-carboxylate de *tert*-butyle sous forme d'un solide blanc fondant à 192°C.
Le 3-amino-5-(N'-méthyl-N'-phényl-hydrazinocarbonyl)-thiéno[2,3-c]pyrazole-1-carboxylate de *tert*-butyle est décrit à l'exemple 8.

### Exemple 10: Préparation du N-(5-(N'-Méthyl-N'-phényl-hydrazinocarbonyl)-1 H-thiéno[2,3-c]pyrazol-3-yl]-4-(4-méthyl-pipérazin-1-ylméthyl)-benzamide.

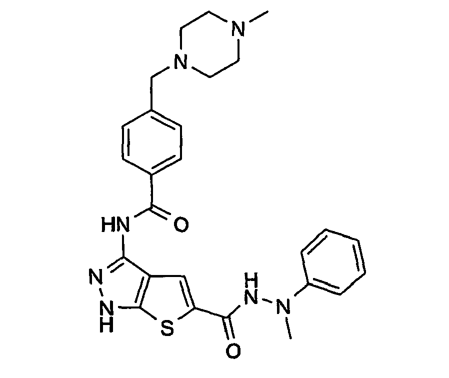

Une solution de 0,14 g (232 µmol) de 5-(N'-méthyl-N'-phényl-hydrazinocarbonyl)-3-[4-(4-méthyl-pipérazin-1-ylméthyl)-benzoylamino]-thiéno[2,3-c]pyrazole-1-carboxylate de *tert*-butyle dans 8 mL de méthanol est introduite dans un tube pour four à micro-ondes. Le tube est bouché et le mélange réactionnel est irradié à une température voisine de 100°C pendant 20 minutes, puis 2 fois 15 minutes additionnelles. Le mélange réactionnel est ensuite concentré à sec sous vide (2 kPa) à une température voisine de 35°C. Le résidu est repris dans 15 mL d'éther diisopropylique, trituré, filtré sur verre fritté, lavé 2 fois avec 5 mL d'éther diisopropylique, essoré puis séché dans une étuve sous vide (0,01 kPa) à une température voisine de 60°C. On obtient ainsi 78 mg de N-[5-(N'-méthyl-N'-phényl-hydrazinocarbonyl)-1H-thiéno[2,3-c]pyrazol-3-yl]-4-(4-méthyl-pipérazin-1-ylméthyl)-benzamide sous forme d'un solide blanc cassé fondant à 246°C. LC-MS-DAD-ELSD : 504(+)=(M+H)(+) ; 502(-)=(M-H)(-).

### 5-(N'-Méthyl-N'-phényl-hydrazinocarbonyl)-3-[4-(4-méthyl-pipérazin-1-ylméthyl)-benzoylamino]-thiéno[2,3-c]pyrazole-1-carboxylate de tert-butyle:

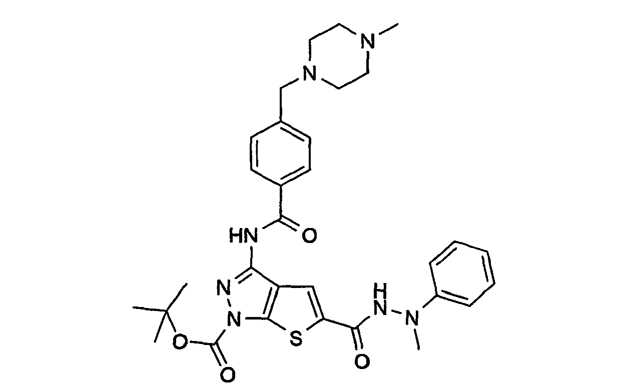

A une solution de 0,30 g (0,77 mmol) de 3-amino-5-(N'-méthyl-N'-phényl-hydrazinocarbonyl)-thiéno[2,3-c]pyrazole-1-carboxylate de *tert*-butyle dans 16 mL de pyridine sous argon, sont ajoutés 391 mg (1,55 mmol) de chlorure de 4-(4-méthyl-pipérazin-1-ylméthyl)-benzoyle; chlorhydrate. Le mélange réactionnel est agité à une température voisine de 25°C pendant 16 heures, puis 294 mg (1,16 mmol) de chlorure de 4-(4-méthyl-pipérazin-1-ylméthyl)-benzoyle sont rajoutés et l'agitation est poursuivie pendant 5 heures. Le mélange réactionnel est ensuite refroidi à une température voisine de 10°C, puis traité avec 50 mL d'eau. Le mélange est extrait 2 fois avec 100 mL d'acétate d'éthyle. Les phases organiques sont réunies, lavées à l'eau jusqu'à pH neutre, séchées sur sulfate de magnésium, filtrées, puis concentrées à sec sous vide (3 kPa) à une température voisine de 30°C. Le résidu est purifié par chromatographie sur une cartouche de gel de silice de 25 g (0,015-0,040 mm) en éluant successivement avec des mélanges dichlorométhane/ méthanol (98/2 en volumes, puis 97/3, puis 96/4 et enfin 95/5) à un débit de 15 mL/min. Les fractions contenant le produit attendu sont réunies puis concentrées à sec sous pression réduite (3 kPa) à une température voisine de 30°C. On obtient ainsi 0,14 g de 5-(N'-méthyl-N'-phényl-hydrazinocarbonyl)-3-[4-(4-méthyl-pipérazin-1-ylméthyl)-benzoylamino]-thiéno[2,3-c]pyrazole-1-carboxylate de *tert*-butyle sous forme d'un solide blanc cassé fondant à 218°C.
Le 3-amino-5-(N'-méthyl-N'-phényl-hydrazinocarbonyl)-thiéno[2,3-c]pyrazole-1-carboxylate de *tert*-butyle est décrit à l'exemple 8. Le chlorure de 4-(4-méthyl-pipérazin-1-ylméthyl)-benzoyle peut être préparé selon le brevet WO 03066613 A1.

### Exemples 11a et 11b: Préparation du 4-(3,5-Diméthyl-pipérazin-1-ylméthyl)-N-[5-(N'-méthyl-N'-phényl-hydrazinocarbonyl)-1H-thiéno[2,3-c]pyrazol-3-yl]-benzamide et du 4-(3,5-Diméthyl-pipérazin-1-ylméthyl)-N-[5-(N'-(4-chloro-phényl)-N'-méthyl-hydrazinocarbonyl)-1H-thiéno[2,3-c]pyrazol-3-yl]-benzamide

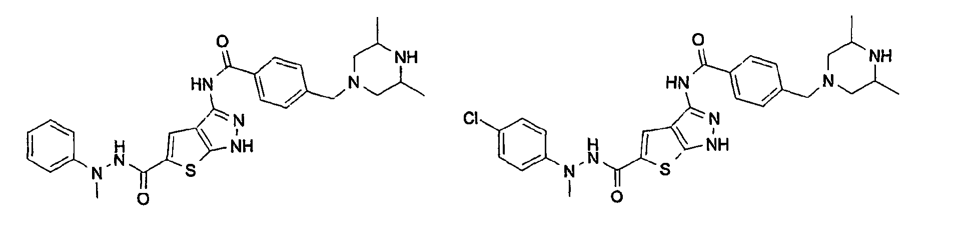

A une solution de 290 mg (0,54 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle dans 6 mL de diméthylformamide sous argon, sont ajoutés 40 mg (108 µmol) d' iodure de tetrabutylammonium, suivis de 184 mg (1,61 mmol) de *cis*-2,6-diméthylpipérazine. Le mélange réactionnel est agité à une température voisine de 25°C pendant 3 heures, puis il est coulé dans un mélange de glace, d'acétate d'éthyle et de n-heptane. Le mélange est filtré sur verre fritté, essoré et séché à l'air. Le solide obtenu est dissous dans 5 mL de méthanol et la solution est introduite dans un tube pour four à micro-ondes. Le tube est bouché et le mélange réactionnel est irradié à une température voisine de 100°C pendant 3 fois 10 minutes. Le mélange réactionnel est ensuite concentré à sec sous vide (2 kPa) à une température voisine de 40°C. Le résidu est purifié par LC/MS préparative (conditions B'). On obtient ainsi 130 mg de 4-(3,5-diméthyl-pipérazin-1-ylméthyl)-N-[5-(N'-méthyl-N'-phényl-hydrazinocarbonyl)-1 H-thiéno[2,3-c]pyrazol-3-yl]-benzamide sous forme d'une poudre jaune fondant entre 192 et 202°C. LC-MS-DAD-ELSD : 518(+)=(M+H)(+).
On isole de la même façon 49 mg de 4-(3,5-diméthyl-pipérazin-1-ylméthyl)-N-[5-(N'-(4-chloro-phényl)-N'-méthyl-hydrazinocarbonyl)-1H-thiéno[2,3-c]pyrazol-3-yl]-benzamide sous forme d'une poudre jaune pâle fondant à 212°C. RMN ¹H (300 MHz, (CD₃)₂SO d₆, δ en ppm): Il s'agit du sel de la structure attendue avec 1,18 (d, J = 6,5 Hz, 6H) ; 2,11 (t large, J = 12,5Hz, 2H) ; 3,00 (d large, J = 12,5Hz, 2H) ; 3,17 (s, 3H) ; 3,33 (m large, 2H) ; 3,73 (m partiellement masqué, 2H) ; 6,82 (d, J = 9,0 Hz, 2H) ; 7,25 (d, J = 9,0 Hz, 2H) ; 7,50 (d, J = 8,0 Hz, 2H) ; 8,08 (d, J = 8,0 Hz, 2H) ; 8,16 (s, 1H) ; 8,20 (m étalé partiellement masqué, 2H) ; 8,92 (m étalé, 1H) ; 10,8 (s, 1 H) ; 11,2 (s, 1H).

### 3-(4-Chlorométhyl-benzoylamino)-5-(N'-méthyl-N'-phényl-hydrazinocarbonyl)-thiéno[2,3-c]pyrazole-1-carboxylate de tert-butyle et 3-(4-Chlorométhyl-benzoylamino)-5-(N'-(4-chloro-phényl)-N'-méthyl-hydrazinocarbonyl)-thiéno[2,3-c]pyrazole-1-carboxylate de tert-butyle:

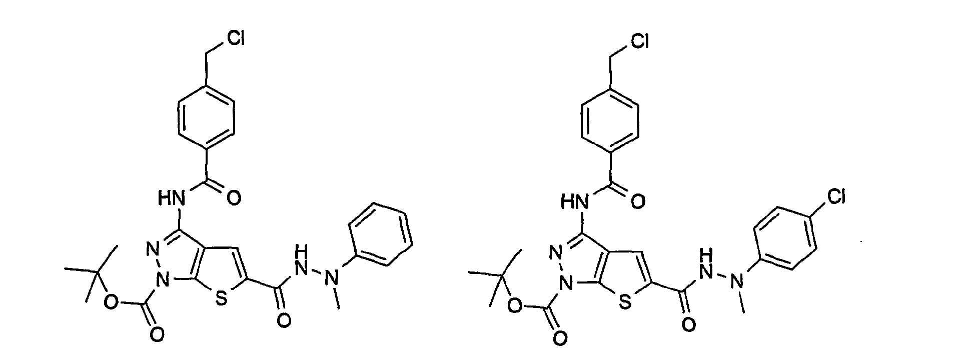

A une solution de 0,39 g (1,0 mmol) d'un mélange environ 70:30 de 3-amino-5-(N'-méthyl-N'-phényl-hydrazinocarbonyl)-thiéno[2,3-c]pyrazole-1-carboxylate de *tert*-butyle et de 3-amino-5-(N'-(4-chloro-phényl)-N'-méthyl-hydrazinocarbonyl)-thiéno[2,3-c]pyrazole-1-carboxylate de *tert*-butyle dans 25 mL de tetrahydrofuranne sous argon, sont ajoutés 276 mg (2,0 mmol) de carbonate de potassium, suivis de 2,0 mL (2,0 mmol) d'une solution 1 M de chlorure de 4-(chlorométhyl)benzoyle dans le tetrahydrofuranne. Le mélange réactionnel est agité à reflux pendant 2 heures, puis il est ensuite refroidi à une température voisine de 25°C et agité pendant 16 heures. Il est ensuite coulé dans l'eau glacée et extrait 2 fois avec 25 mL d'acétate d'éthyle. Les phases organiques sont réunies, lavées avec de la saumure saturée, séchées sur sulfate de magnésium, filtrées, puis concentrées à sec sous vide (2 kPa) à une température voisine de 40°C. Le résidu est purifié par chromatographie sur une cartouche de gel de silice de 25 g (0,015-0,040 mm) en éluant avec un mélange dichlorométhane/ méthanol (98/2 en volumes) à un débit de 10 mL/min. Les fractions contenant le produit attendu sont réunies puis concentrées à sec sous pression réduite (2 kPa) à une température voisine de 40°C. Le résidu est repurifié par chromatographie sur une cartouche de gel de silice de 90 g (0,015-0,040 mm) en éluant avec un mélange dichlorométhane/ méthanol (98/2 en volumes) à un débit de 10 mL/min. Les fractions contenant le produit attendu sont réunies puis concentrées à sec sous pression réduite (2 kPa) à une température voisine de 40°C. On obtient ainsi 422 mg d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle sous forme d'une mousse orangée. LC-MS-DAD-ELSD et SM-EI-CI : mélange structure attendue (environ 70% ;M.W=539/..) et analogue attendu dichoré (environ 30% ; M.W=573/...). RMN ¹H (400 MHz, (CD₃)₂SO d₆, δ en ppm): mélange de conformères environ 80% - 20% avec 1,55 (s, 1,8H) ; 1,64 (s, 7,2H) ; 3,18 (s, 2,4H) ; 3,20 (s, 0,6H) ; 4,85 (s, 2H) ; 6,78 (t, J = 7,5 Hz, 0,8H) ; 6,83 (d, J = 8,0 Hz, 1,6H) ; 6,97 (t, J = 8,0 Hz, 0,2H) ; 7,03 (d, J = 8,0 Hz, 0,4H) ; 7,22 (t, J = 8,0 Hz, 1,6H) ; 7,32 (t, J = 8,0 Hz, 0,4H) ; 7,60 (d, J = 8,0 Hz, 2H) ; 8,11 (d, J = 8,0 Hz, 2H) ; 8,27 (s, 0,2H) ; 8,32 (s, 0,8H) ; 9,90 (s, 0,2H) ; 10,95 (s, 0,8H) ; 11,7 (s, 0,2H) ; 11,8 (s, 0,8H) .

### 3-Amino-5-(N'-méthyl-N'-phényl-hydrazinocarbonyl)-thiéno[2,3-c]pyrazole-1-carboxylate de tert-butyle et 3-Amino-5-(N'-(4-chloro-phényl)-N'méthyl-hydrazinocarbonyl)-thiéno[2,3-c]pyrazole-1-carboxylate de tert-butyle:

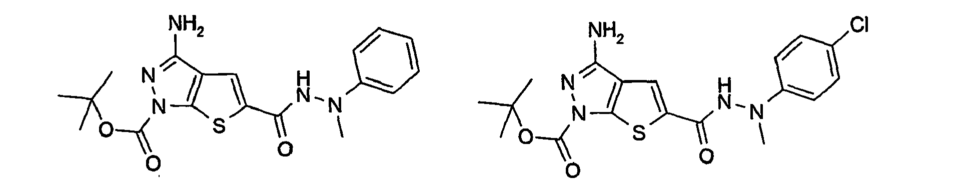

A une suspension de 14 g (48,7 mmol) d'un mélange de N'-méthyl-N'-phényl-(3-amino-1H-thiéno[2,3-c]pyrazole)-5-carbo-hydrazide (majoritaire) et de 3-amino-5-(N'-(4-chloro-phényl)-N'-méthyl-hydrazinocarbonyl)-thiéno[2,3-c]pyrazole-1-carboxylate de *tert*-butyle (minoritaire) dans 500 mL de dichlorométhane sous argon, sont ajoutés 1,19 g (9,74 mmol) de 4-(diméthylamino)-pyridine puis 7,4 mL (53,6 mmol) de triéthylamine. Le mélange réactionnel est ensuite refroidi à une température voisine de 0°C, puis une solution de 8,5 g (39,0 mmol) de di-*tert*-butyl-dicarbonate dans 200 mL de dichlorométhane est ajoutée au goutte à goutte en 1 heure. Le mélange réactionnel est ensuite agité à une température voisine de 0°C pendant une heure, puis il est réchauffé à température voisine de 25°C et concentré à sec sous vide (2 kPa) à une température voisine de 40°C. Le résidu est purifié par chromatographie sur une colonne de gel de silice de 500 g (0,020-0,040 mm) en éluant avec du dichlorométhane pur, puis avec un mélange dichlorométhane/ méthanol (99/1 en volumes). Les fractions contenant le produit attendu sont réunies puis concentrées à sec sous pression réduite (2 kPa) à une température voisine de 40°C. Le résidu est repurifié par chromatographie sur une cartouchee de gel de silice de 400 g (0,020-0,040 mm) en éluant avec du un mélange dichlorométhane/ tetrahydrofuranne (98/2 en volumes). Les fractions contenant le produit attendu sont réunies puis concentrées à sec sous pression réduite (2 kPa) à une température voisine de 40°C. On obtient ainsi 7,0 g d'un mélange environ 70:30 de 3-amino-5-(N'-méthyl-N'-phényl-hydrazinocarbonyl)-thiéno[2,3-c]pyrazole-1-carboxylate de *tert*-butyle et de 3-amino-5-(N'-(4-chloro-phényl)-N'-méthyl-hydrazinocarbonyl)-thiéno[2,3-c]pyrazole-1-carboxylate de *tert-*butyle sous forme d'un solide beige. LC-MS-DAD-ELSD :mélange contenant la structure attendue (pureté estimée 70%) : 388(+)=(M+H)(+).

### N'-Méthyl-N'-phényl-(3-amino-1H-thiéno[2,3-c]pyrazole)-5-carbo-hydrazide:

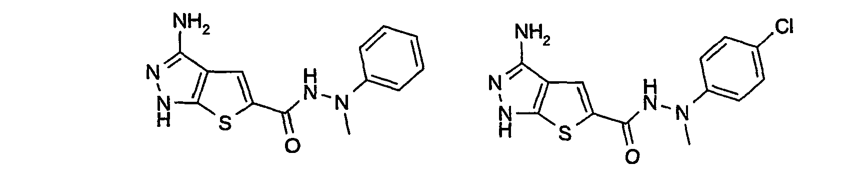

A une solution de 29,6 g (45,2 mmol) de mélange de N'-méthyl-N'-phényl-(4-cyano-5-méthanesulfinyl-thiophène)-2-carbo-hydrazide (majoritaire), de N'-méthyl-N'-phényl-(4-cyano-5-méthanesulfonyl-thiophène)-2-carbo-hydrazide (minoritaire), de N'-(4-chloro-phényl)-N'-méthyl-(4-cyano-5-méthanesulfinyl-thiophène)-2-carbo-hydrazide (minoritaire) et de N'-(4-chloro-phényl)-N'-méthyl-(4-cyano-5-méthanesulfonyl-thiophène)-2-carbo-hydrazide (minoritaire) dans 900 mL d'éthanol sous argon, sont ajoutés 650 mL (650 mmol) d'une solution 1 M d'hydrazine dans le tetrahydrofuranne en 20 minutes. Le mélange réactionnel est chauffé à une température voisine de 50°C pendant 16 heures, puis il est refroidi à une température voisine de 25°C et concentré à sec sous vide (2 kPa) à une température voisine de 40°C. Le résidu est repris dans 1 L d'éthanol et une solution de 20 mL (240 mmol) d'acide chlorhydrique 12N est additionnée sous agitation à une température voisine de 25°C. Le mélange réactionnel est agité à une température voisine de 25°C pendant 1,75 heures, puis 20 mL (240 mmol) d'acide chlorhydrique 12 N sont additionnés. Le mélange réactionnel est agité à une température voisine de 25°C pendant 1,5 heures, puis il est filtré et le solide est lavé deux fois avec 200 mL d'éthanol. Le filtrat est concentré à sec sous vide (2 kPa) à une température voisine de 40°C. Le résidu est purifié par chromatographie sur une colonne de gel de silice de 500 g (0,020-0,040 mm) en éluant avec un mélange dichlorométhane/ méthanol (90/10 en volumes), puis par un mélange dichlorométhane/ méthanol/ ammoniaque 28% (77/20/3 en volumes). Les fractions contenant le produit attendu sont réunies puis concentrées à sec sous pression réduite (2 kPa) à une température voisine de 40°C. Le résidu est repris dans 200 mL d'acétate d'éthyle et recristallisé. Le solide obtenu est filtré, essoré et séché dans un dessicateur sous vide. Les liqueurs mères sont concentrées à sec sous vide (2 kPa) à une température voisine de 40°C. Le résidu est purifié par chromatographie sur une colonne de gel de silice (0,020-0,040 mm) en éluant avec un mélange dichlorométhane/ méthanol/ ammoniaque 28% (77/20/3 en volumes). Les fractions contenant le produit attendu sont réunies puis concentrées à sec sous pression réduite (2 kPa) à une température voisine de 40°C. Le résidu est repris dans l'acétate d'éthyle, trituré, puis reconcentré à sec dans les mêmes conditions. On obtient ainsi au total 14 g de N'-méthyl-N'-phényl-(3-amino-1H-thiéno[2,3-c]pyrazole)-5-carbo-hydrazide (majoritaire) et de N'-(4-chloro-phényl)-N'-méthyl-(3-amino-1H-thiéno[2,3-c]pyrazole)-5-carbo-hydrazide (minoritaire) sous forme d'une mousse brune qui est directement utilisée dans l'étape suivante.

### N'-Méthyl-N'-phényl-(4-cyano-5-méthanesulfinyl-thiophène)-2-carbo-hydrazide ; N'-Méthyl-N'-phényl-(4-cyano-5-méthanesulfonyl-thiophène)-2-carbo-hydrazide; N'-(4-chloro-phényl)-N'-Méthyl-(4-cyano-5-méthanesulfinyl-thiophène)-2-carbo-hydrazide ; N'-(4-chloro-phényl)-N'-Méthyl-(4-cyano-5-méthanesulfonyl-thiophène)-2-carbo-hydrazide:

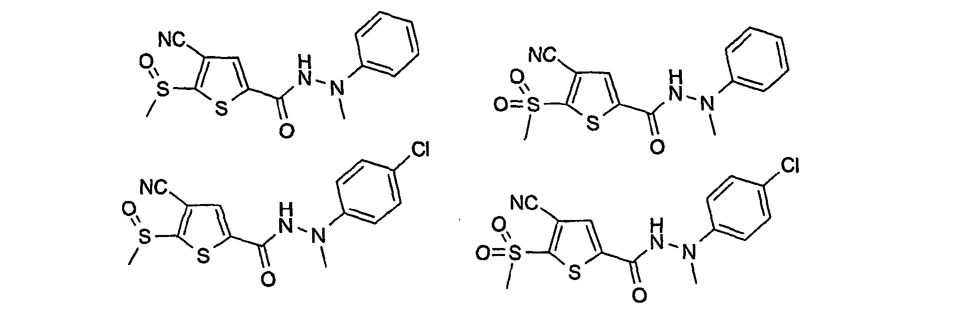

A une solution de 27,4 g (90,3 mmol) de N'-méthyl-N'-phényl-(4-cyano-5-méthylsulfanyl-thiophène)-2-carbo-hydrazide dans 1,0 L de tetrahydrofuranne sous argon, sont ajoutés 83,3 g (135,5 mmol) d'oxone^{®} (peroxymonosulfate de potassium) en 5 minutes, suivis de 100 mL d'eau. Le mélange réactionnel est agité à une température voisine de 25°C pendant 2,5 heures, puis il est filtré sur sulfate de magnésium. Le solide est lavé deux fois avec du tetrahydrofuranne et le filtrat est concentré à sec sous vide (2 kPa) à une température voisine de 40°C. Le résidu est repris dans 500 mL d'acétate d'éthyle, décanté et la phase aqueuse est extraite avec 500 mL d'acétate d'éthyle. Les phases organiques sont réunies, séchées sur sulfate de magnésium, filtrées, puis concentrées à sec sous vide (2 kPa) à une température voisine de 40°C. On obtient ainsi 29,4 g d'un mélange de N'-méthyl-N'-phényl-(4-cyano-5-méthanesulfinyl-thiophène)-2-carbo-hydrazide (majoritaire), de N'-méthyl-N'-phényl-(4-cyano-5-méthanesulfonyl-thiophène)-2-carbo-hydrazide (minoritaire), de N'-(4-chloro-phényl)-N'-méthyl-(4-cyano-5-méthanesulfinyl-thiophène)-2-carbo-hydrazide (minoritaire) et de N'-(4-chloro-phényl)-N'-méthyl-(4-cyano-5-méthanesulfonyl-thiophène)-2-carbo-hydrazide (minoritaire) sous forme d'une mousse brune qui est utilisée directement dans l'étape suivante. LC/MS: 4 pics TR=3,11 min. M+H⁺: 320.06; TR=3,33 min.
M+H⁺: 353.99 (1 CI); TR=3,53 min. M+H⁺: 336.04; TR=3,88 min. M+H⁺_{:} 369.97 (1 CI).

### N'-Méthyl-N'-phényl-(4-cyano-5-méthylsulfanyl-thiophène)-2-carbo-hydrazide:

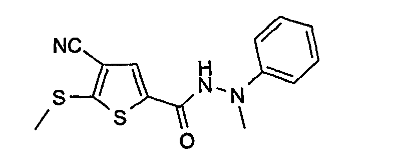

A une solution de 24,6 mL (200,6 mmol) de 1-méthyl-1-phényl-hydrazine dans 300 mL de toluène à une température voisine de 25°C et sous argon, on ajoute 100,3 mL (200,6 mmol) de solution de triméthylaluminium 2M dans le toluène, diluée dans 600 mL de toluène, en maintenant la température inférieure ou égale à 25°C. Le mélange réactionnel est agité à une température voisine de 25°C, puis 39,3 g (172,9 mmol) de 4-cyano-5-méthylsulfanyl-thiophène-2-carboxylate d'éthyle sont ajoutés par spatulées. Le mélange réactionnel est chauffé à une température voisine de 80°C pendant 4 heures, puis il est refroidi à une température voisine de 25°C et agité pendant 16 heures. Il est ensuite refroidi à une température voisine de 0°C et 1 L d'acide chlorhydrique 1 N est ajouté. La suspension résultante est agitée à une température voisine de 10°C pendant une heure, puis filtrée. Le précipité est essoré, séché sous hotte ventilée pendant 2,5 jours, puis séché dans un dessicateur sous vide (2 kPa) pendant 4 heures. Le filtrat est décanté et la phase aqueuse est extraite avec 500 mL d'acétate d'éthyle. Les phases organiques sont réunies, lavées avec 500 mL de saumure saturée, séchées sur sulfate de magnésium, filtrées puis concentrées à sec sous vide (2 kPa) à une température voisine de 40°C. On obtient ainsi 29,1 g de N'-méthyl-N'-phényl-(4-cyano-5-méthylsulfanyl-thiophène)-2-carbo-hydrazide sous forme d'une poudre ocre. SM-EI : 303(+)=M(+).
Le 4-cyano-5-méthylsulfanyl-thiophène-2-carboxylate d'éthyle est décrit à l'exemple 8.

### Exemples 12a et 12b: Préparation du 4-(4-Méthyl-perhydro-1,4-diazepin-1-ylméthyl)-N-[5-(N'-méthyl-N'-phényl-hydrazinocarbonyl)-1H-thiéno[2,3-c]pyrazol-3-yl]-benzamide et du 4-(4-Méthyl-perhydro-1,4-diazepin-1-ylméthyl)-N-[5-(N'-(4-chloro-phényl)-N'-méthyl-hydrazinocarbonyl)-1 H-thiéno[2,3-c]pyrazol-3-yl]-benzamide.

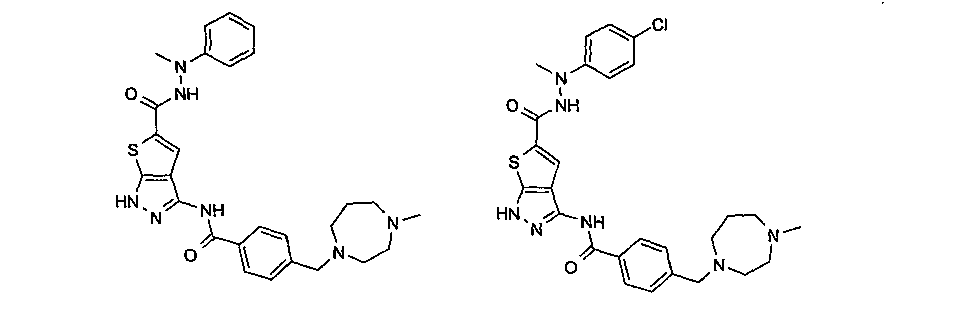

A une solution de 200 mg (0,37 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle dans 6 mL de diméthylformamide sous argon, sont ajoutés 27 mg (74 µmol) d' iodure de tetrabutylammonium, suivis de 138 µL (1,11 mmol) de N-méthyl-homopipérazine. Le mélange réactionnel est agité à une température voisine de 25°C pendant 16 heures, puis il est traité avec de la glace. Il est ensuite extrait avec de l'acétate d'éthyle puis 2 fois avec du dichlorométhane. Les extraits organiques sont réunis puis concentrés à sec sous vide (2 kPa) à une température voisine de 40°C. Le résidu est dissous dans 3 mL de méthanol et la solution est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par LC/MS préparative (1 injection: conditions B'; 3 injections: conditions B). On obtient ainsi 53 mg de 4-(4-méthyl-perhydro-1,4-diazepin-1-ylméthyl)-N-[5-(N'-méthyl-N'-phényl-hydrazinocarbonyl)-1H-thiéno[2,3-c]pyrazol-3-yl]-benzamide sous forme d'une poudre jaune. LC-MS-DAD-ELSD : 516(-)=(M-H)(-) ; 518(+)=(M+H)(+).
On isole de la même façon 20 mg de 4-(4-méthyl-perhydro-1,4-diazepin-1-ylméthyl)-N-[5-(N'-(4-chloro-phényl)-N'-méthyl-hydrazinocarbonyl)-1H-thiéno[2,3-c]pyrazol-3-yl]-benzamide sous forme d'une poudre jaune pâle fondant à 162°C. RMN ¹H (300 MHz, (CD₃)₂SO d₆, δ en ppm): Il s'agit du sel de la structure attendue avec 2,08 (m large, 2H) ; 2,84 (s, 3H) ; 3,18 (s, 3H) ; de 2,93 à 4,51 (m partiellement masqué, 10H) ; 6,82 (d, J = 9,0 Hz, 2H) ; 7,24 (d, J = 9,0 Hz, 2H) ; 7,63 (d large, J = 8,0 Hz, 2H) ; 8,14 (d, J = 8,0 Hz, 2H) ; 8,17 (s, 1 H) ; 8,22 (m étalé partiellement masqué, 1 H) ; 10,0 (m très étalé, 1H); 10,8 (s,1H); 11,3 (s, 1 H) .
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle est décrit à l'exemple 11.

### Exemples 13a et 13b: Préparation du N-[5-(N'-Méthyl-N'-phényl-hydrazinocarbonyl)-1H-thiéno[2,3-c]pyrazol-3-yl]-4-pipérazin-1-ylméthyl-benzamide et du N-{5-[N'-(4-Chloro-phényl)-N'-méthyl-hydrazinocarbonyl]-1H-thiéno[2,3-c]pyrazol-3-yl}-4-pipérazin-1-ylméthyl-benzamide.

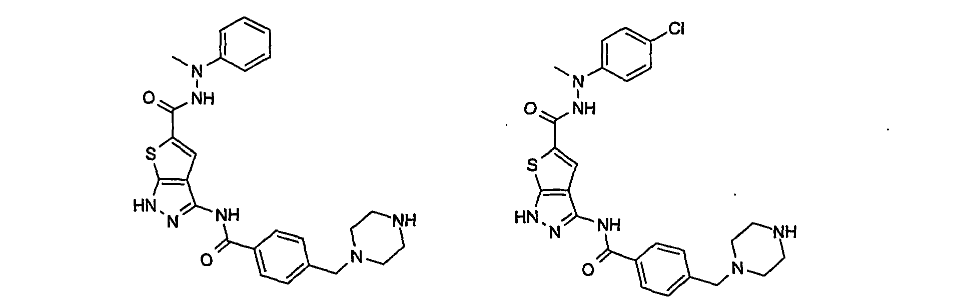

A une solution de 200 mg (0,37 mmol) d'un mélange environ 70:30 de 3-(4-chlorométhyl-benzoylamino)-5-(N'-méthyl,N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle dans 6 mL de diméthylformamide sous argon, sont ajoutés 27 mg (74 µmol) d' iodure de tetrabutylammonium, suivis de 96 mg (1,11 mmol) de pipérazine. Le mélange réactionnel est agité à une température voisine de 25°C pendant 16 heures, puis il est traité avec de la glace. Il est ensuite extrait avec de l'acétate d'éthyle puis 2 fois avec du dichlorométhane. Les extraits organiques sont réunis puis concentrés à sec sous vide (2 kPa) à une température voisine de 40°C. Le résidu est dissous dans 3 mL de méthanol et la solution est introduite dans un tube pour four à micro-ondes. Le tube est bouché et le mélange réactionnel est irradié à une température voisine de 100°C pendant 2 fois 30 minutes. Le mélange réactionnel est ensuite concentré à sec sous vide (2 kPa) à une température voisine de 40°C. Le résidu est purifié par LC/MS préparative (conditions B). On obtient ainsi 83 mg de N-[5-(N'-méthyl-N'-phényl-hydrazinocarbonyl)-1H-thiéno[2,3-c]pyrazol-3-yl]-4-pipérazin-1-ylméthyl-benzamide sous forme d'une poudre jaune fondant à 176°C. LC-MS-DAD-ELSD: 490(+)=M(+H)(+).
On isole de la même façon 34 mg de N-{5-[N'-(4-chloro-phényl)-N'-méthyl-hydrazinocarbonyl]-1H-thiéno[2,3-c]pyrazol-3-yl}-4-pipérazin-1-ylméthyl-benzamide sous forme d'une poudre orangée fondant à 180°C. LC-MS-DAD-ELSD : 524(+)/...=(M+H)(+)/...(1 Cl)
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle est décrit à l'exemple 11.

### Exemples 14a et 14b: Préparation du N-[5-(N'-Méthyl-N'-phényl-hydrazinocarbonyl)-1H-thiéno[2,3-c]pyrazol-3-yl]-4-(3-méthyl-piperazin-1-ylméthyl)-benzamide et du N-{5-[N'-(4-Chloro-phényl)-N'-méthyl-hydrazinocarbonyl]-1H-thiéno[2,3-c]pyrazol-3-yl}-4-(3-méthyl-pipérazin-1-ylméthyl)-benzamide.

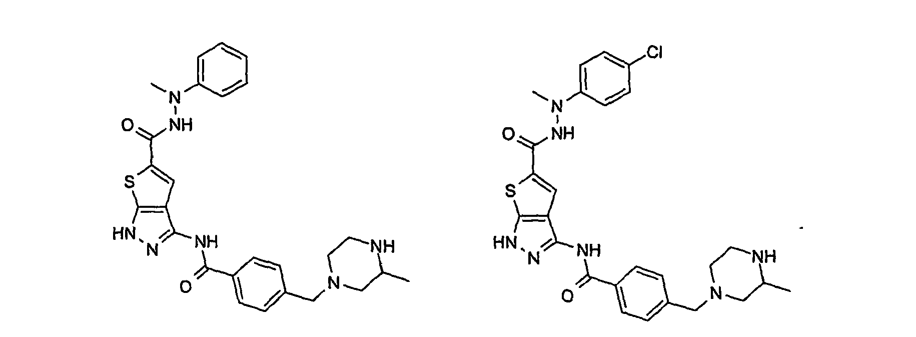

A une solution de 200 mg (0,37 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle dans 6 mL de diméthylformamide sous argon, sont ajoutés 27 mg (74 µmol) d' iodure de tetrabutylammonium, suivis de 111 mg (1,11 mmol) de 2-méthyl-pipérazine. Le mélange réactionnel est agité à une température voisine de 25°C pendant 16 heures, puis il est traité avec de la glace. Il est ensuite extrait avec de l'acétate d'éthyle puis 2 fois avec du dichlorométhane. Les extraits organiques sont réunis puis concentrés à sec sous vide (2 kPa) à une température voisine de 40°C. Le résidu est dissous dans 3 mL de méthanol et la solution est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par chromatographie sur une cartouche de gel de silice de 12 g (0,035-0,060 mm) en éluant d'abord avec un gradient d'un mélange dichlorométhane/méthanol (100/0 à 98/2 en volumes), puis avec un mélange dichlorométhane/méthanol/ammoniaque à 28% (12/3/0,5 en volumes) à un débit de 20 mL/min. Les fractions contenant le produit attendu sont réunies puis concentrées à sec sous pression réduite (2 kPa) à une température voisine de 40°C. Le résidu est purifié par LC/MS préparative (conditions B). On obtient ainsi 85 mg de N-[5-(N'-méthyl-N'-phényl-hydrazinocarbonyl)-1H-thiéno[2,3-c]pyrazol-3-yl]-4-(3-méthyl-piperazin-1-ylméthyl)-benzamide sous forme d'une poudre jaune fondant de 176 à 186°C. LC-MS-DAD-ELSD : 504(+)=(M+H)(+).
On isole de la même façon 8 mg de N-{5-[N'-(4-chloro-phényi)-N'-méthyl-hydrazinocarbonyl]-1H-thiéno[2,3-c]pyrazol-3-yl}-4-(3-méthyl-piperazin-1-ylméthyl)-benzamide sous forme d'un solide jaune pâle fondant de 189 à 195°C. LC-MS-DAD-ELSD : 538(+)/...=(M+H)(+)/...(1 Cl)
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle est décrit à l'exemple 11.

### Exemples 15a et 15b: Préparation du 4-Diéthylaminométhyl-N-[5-(N'-méthyl-N'-phényl-hydrazinocarbonyl)-1H-thiéno[2,3-c]pyrazol-3-yl]-benzamide; trifluoro-acetate et du N-{5-[N'-(4-Chloro-phényl)-N'-méthyl-hydrazinocarbonyl]-1H-thiéno[2,3-c]pyrazol-3-yl}-4-diéthylaminométhyl-benzamide.

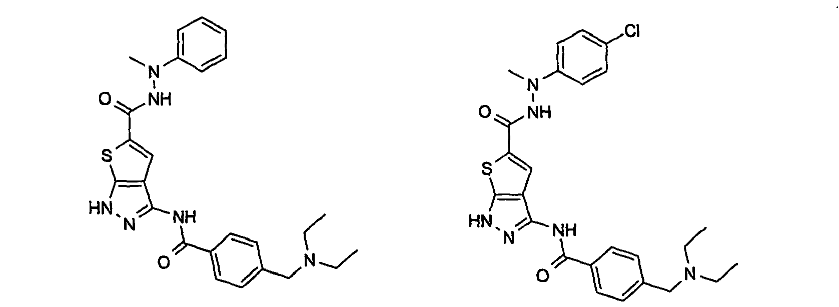

A une solution de 100 mg (0,19 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle dans 1,5 mL de diméthylformamide sous argon, sont ajoutés 14 mg (37 µmol) d' iodure de tetrabutylammonium puis une suspension de 61 mg (0,56 mmol) de chlorhydrate de diéthylamine et 77 mg (0,56 mmol) de carbonate de potassium dans 1,5 mL de diméthylformamide. Le mélange réactionnel est agité à une température voisine de 25°C pendant 80 heures, puis il est traité avec de la glace. Il est ensuite extrait 2 fois avec un mélange 50/50 (v/v) d'acétate d'éthyle et d'heptane. Les extraits organiques sont réunis, séchés sur sulfate de magnésium, puis concentrés à sec sous vide (2 kPa) à une température voisine de 40°C. Le résidu est dissous dans 5 mL de méthanol et la solution est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par LC/MS préparative (conditions B). On obtient ainsi 40 mg de 4-diéthylaminométhyl-N-[5-(N'-méthyl-N'-phényl-hydrazinocarbonyl)-1 H-thiéno[2,3-c]pyrazol-3-yl]-benzamide; trifluoro-acetate sous forme d'une poudre jaune fondant de 158 à 170°C. RMN ¹H (300 MHz, (CD₃)₂SO d₆, δ en ppm) : observation d'un mélange 80 % - 20 % de conformères avec 1,25 (t, J = 7,0 Hz, 6H) ; 3,11 (m, 4H) ; 3,18 (s, 3H) ; 4,41 (d, J = 5,5 Hz, 2H) ; 6,77 (t, J = 7,5 Hz, 0,8H) ; 6,82 (d, J= 8,0 Hz, 1,6H) ; 6,94 (t, J = 7,5 Hz, 0,2H) ; 7,02 (d, J = 8,0 Hz, 0,4H) ; 7,22 (t, J = 8,0 Hz, 1,8H) ; 7,31 (t, J = 8,0 Hz, 0,2H) ; 7,69 (d large, J = 8,0 Hz, 2H) ; 8,17 (d partiellement masqué, J = 8,0 Hz, 2H) ; 8,18 (s, 1H) ; 9,51 (m étalé, 1 H) ; 9,71 (s, 0,2H) ; 10,75 (s, 0,8H) ; 11,25 (s large, 0,2H) ; 11,35 (s large, 0,8H) ; 13,0 (m très étalé, 1 H) .
On isole de la même façon 14 mg de N-{5-[N'-(4-chloro-phényl)-N'-méthyl-hydrazinocarbonyl]-1H-thiéno[2,3-c]pyrazol-3-yl}-4-diéthylaminométhyl-benzamide sous forme d'un solide jaune pâle fondant de 170 à 178°C. LC-MS-DAD-ELSD : 511(+)/ ... =(M+H)(+)/ ... (1 Cl).
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle est décrit à l'exemple 11.

### Exemples 16a et 16b: Préparation du N-[5-(N'-Méthyl-N'-phényl-hydrazinocarbonyl)-1H-thiéno[2,3-c]pyrazo!-3-yl]-3-(4-méthyl-pipérazin-1-ylméthyl)-benzamide et du N-[5-(N'-(4-Chloro-phényl)-N'-méthyl-hydrazinocarbonyl)-1H-thiéno[2,3-c]pyrazol-3-yl]-3-(4-méthyl-pipérazin-1-ylméthyl)-benzamide.

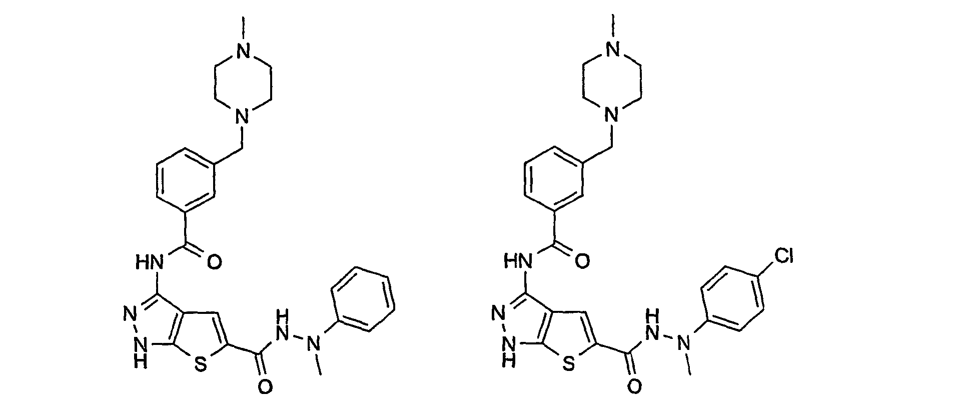

Une solution de 0,07 g (116 µmol) d'un mélange environ 70:30 de de 5-(N'-méthyl-N'-phényl-hydrazinocarbonyl)-3-[3-(4-méthyl-pipérazin-1-ylméthyl)-benzoylamino]-thiéno[2,3-c]pyrazole-1-carboxylate de *tert*-butyle et de 5-(N'-(4-chloro-phényl)-N'-méthyl-hydrazinocarbonyl)-3-[3-(4-méthyl-pipérazin-1-ylméthyl)-benzoylamino]-thiéno[2,3-c]pyrazole-1-carboxylate de *tert*-butyle dans 4 mL de méthanol est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par LC/MS préparative (conditions B). On obtient ainsi 70 mg de N-[5-(N'-méthyl-N'-phényl-hydrazinocarbonyl)-1H-thiéno[2,3-c]pyrazol-3-yl]-3-(4-méthyl-pipérazin-1-ylméthyl)-benzamide sous forme d'une poudre jaune fondant de 149 à 156°C. LC-MS-DAD-ELSD : 502(-)=(M-H)(-) ; 504(+)=(IVI+H)(+),
On isole de la même façon 29 mg de N-[5-(N'-(4-chloro-phényl)-N'-méthyl-hydrazinocarbonyl)-1H-thiéno[2,3-*c*]pyrazol-3-yl]-3-(4-méthyl-pipérazin-1-ylméthyl)-benzamide sous forme d'une poudre jaune pâle fondant de 149 à 159°C. RMN ¹H (400 MHz, (CD₃)₂SO d₆, δ en ppm): 2,43 (m étalé partiellement masqué, 2H) ; 2,79 (s, 3H) ; 3,03 (m étalé, 2H) ; 3,17 (s, 3H) ; de 3,25 à 3,88 (m partiellement masqué, 4H) ; 3,74 (s large, 2H) ; 6,82 (d, J = 9,0 Hz, 2H) ; 7,25 (d, J = 9,0 Hz, 2H) ; 7,54 (t, J = 8,0 Hz, 1 H) ; 7,57 (d, J = 8,0 Hz, 1 H) ; 8,01 (d partiellement masqué, J = 8,0 Hz, 1 H) ; 8,02 (s, 1 H) ; 8,18 (s, 1H) ; 9,50 (m très étalé, 1 H) ; 10,8 (s, 1 H) ; 11,25 (s, 1 H) .

### 5-(N'-Méthyl-N'-phényl-hydrazinocarbonyl)-3-[3-(4-méthyl-pipérazin-1-ylméthyl)-benzoylamino]-thiéno[2,3-c]pyrazole-1-carboxylate de tert-butyle et 5-(N'-(4-Chloro-phényl)-N'-méthyl-hydrazinocarbonyl)-3-[3-(4-méthyl-pipérazin-1-ylméthyl)-benzoylamino]-thiéno[2,3-c]pyrazole-1-carboxylate de tert-butyle:

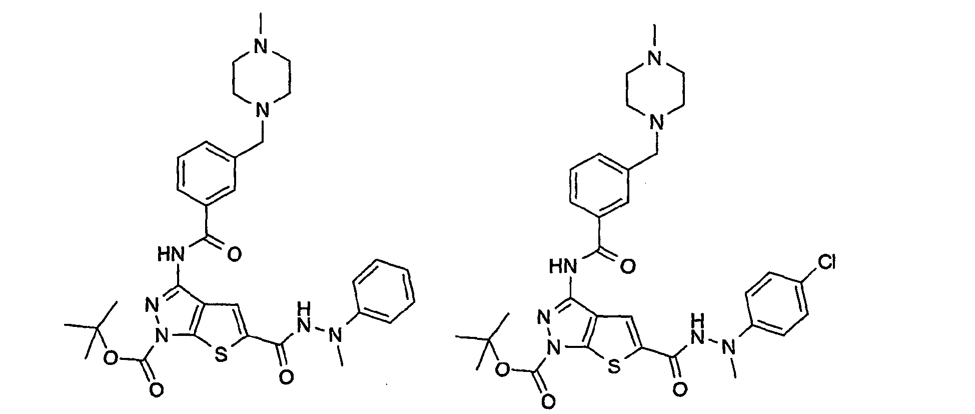

A une solution de 0,20 g (0,52 mmol) d'un mélange environ 70:30 de 3-amino-5-(N'-méthyl-N'-phényl-hydrazinocarbonyl)-thiéno[2,3-c]pyrazole-1-carboxylate de *tert*-butyle et de 3-amino-5-(N'-(4-chloro-phényl)-N'-méthyl-hydrazinocarbonyl)-thiéno[2,3-c]pyrazole-1-carboxylate de *tert*-butyle dans 15 mL de tetrahydrofuranne sous argon, sont ajoutés 143 mg (1,03 mmol) de carbonate de potassium et 261 mg (1,03 mmol) de chlorure de 3-(4-méthyl-pipérazin-1-ylméthyl)-benzoyle; chlorhydrate. Le mélange réactionnel est agité à une température voisine de 25°C pendant 16 heures, puis à reflux pendant 3 heures. 261 mg (1,03 mmol) de chlorure de 3-(4-méthyl-pipérazin-1-ylméthyl)-benzoyle; chlorhydrate sont rajoutés et l'agitation est poursuivie à reflux pendant 4 heures, puis à une température voisine de 25°C pendant 16 heures. 143 mg (1,03 mmol) de carbonate de potassium sont rajoutés et l'agitation est poursuivie à reflux pendant 4 heures, puis à une température voisine de 25°C pendant 16 heures. Le mélange réactionnel est ensuite refroidi à une température voisine de 25°C, puis versé sur de l'eau glacée. Le mélange est extrait à l'acétate d'éthyle. Les phases organiques sont réunies, séchées sur sulfate de magnésium, filtrées, puis concentrées à sec sous vide (2 kPa) à une température voisine de 40°C. Le résidu est purifié par chromatographie sur une cartouche de gel de silice de 70 g (0,015-0,040 mm) en éluant avec des mélanges dichlorométhane/ méthanol (95/5 puis 99/1 en volumes) à un débit de 40 mL/min. Les fractions contenant le produit attendu sont réunies puis concentrées à sec sous pression réduite (2 kPa) à une température voisine de 40°C. On obtient ainsi 0,07 g d'un mélange environ 70:30 de de 5-(N'-méthyl-N'-phényl-hydrazinocarbonyl)-3-[3-(4-méthyl-pipérazin-1-ylméthyl)-benzoylamino]-thiéno[2,3-c]pyrazole-1-carboxylate de *tert*-butyle et de 5-(N'-(4-chloro-phényl)-N'-méthyl-hydrazinocarbonyl)-3-[3-(4-méthyl-pipérazin-1-ylméthyl)-benzoylamino]-thiéno[2,3-c]pyrazole-1-carboxylate de *tert*-butyle sous forme d'une poudre blanche qui est utilisée directement dans l'étape suivante. LC-MS-DAD-ELSD : produit majoritaire 604(+)=(M+H)(+) ; 602(-)=(M-H)(-).
Le mélange environ 70:30 de 3-amino-5-(N'-méthyl-N'-phényl-hydrazinocarbonyl)-thiéno[2,3-c]pyrazole-1-carboxylate de *tert*-butyle et de 3-amino-5-(N'-(4-chloro-phényl)-N'-méthyl-hydrazinocarbonyl)-thiéno[2,3-c]pyrazole-1-carboxylate de *tert*-butyle est décrit à l'exemple 11. Le chlorure de 3-(4-méthyl-pipérazin-1-ylméthyl)-benzoyle; chlorhydrate peut être préparé selon le brevet WO 03066613 A1.

### Exemples 17a et 17b: Préparation du N-[5-(N'-Méthyl-N'-phényl-hydrazinocarbonyl)-1H-thiéno[2,3-c]pyrazol-3-yl]-4-pipéridin-1-ylméthyl-benzamide et du N-[5-(N'-(4-Chloro-phényl)-N'-méthyl-hydrazinocarbonyl)-1H-thiéno[2,3-c]pyrazol-3-yl]-4-pipéridin-1-ylméthyl-benzamide.

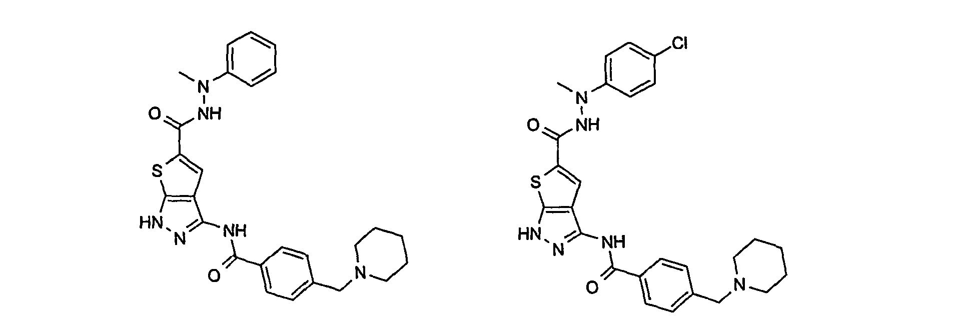

A une solution de 200 mg (0,37 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle dans 6 mL de diméthylformamide sous argon, sont ajoutés 27 mg (74 µmol) d' iodure de tetrabutylammonium puis 110 µL (1,11 mmol) de pipéridine. Le mélange réactionnel est agité à une température voisine de 25°C pendant 16 heures, puis il est versé dans de la glace. Il est ensuite extrait 2 fois avec un mélange 50/50 d'acétate d'éthyle et d'heptane. Les extraits organiques sont réunis, séchés sur sulfate de magnésium, puis concentrés à sec sous vide (2 kPa) à une température voisine de 40°C. Le résidu est dissous dans 4 mL de méthanol et la solution est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par LC/MS préparative (conditions B). On obtient ainsi 75 mg de N-[5-(N'-méthyl-N'-phényl-hydrazinocarbonyl)-1H-thiéno[2,3-c]pyrazol-3-yl]-4-pipéridin-1-ylméthyl-benzamide sous forme d'une poudre jaune fondant de 170 à 182°C. LC-MS-DAD-ELSD : 487(-)=(M-H)(-) ; 489(+)=(M+H)(+).
On isole de la même façon 5 mg de N-[5-(N'-(4-chloro-phényl)-N'-méthyl-hydrazinocarbonyl)-1 H-thiéno[2,3-c]pyrazol-3-yl]-4-pipéridin-1-ylméthyl-benzamide sous forme d'une poudre jaune pâle. LC-MS-DAD-ELSD : 523(+)/....=(M+H)(+)/...(1 Cl).
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle est décrit à l'exemple 11.

### Exemples 18a et 18b: Préparation du N-[5-(N'-Méthyl-N'-phényl-hydrazinocarbonyl)-1H-thiéno[2,3-c]pyrazol-3-yl]-4-pyrrolidin-1-ylméthyl-benzamide et du N-[5-(N'-(4-Chloro-phényl)-N'-méthyl-hydrazinocarbonyl)-1H-thiéno[2,3-c]pyrazol-3-yl]-4-pyrrolidin-1-ylméthyl-benzamide.

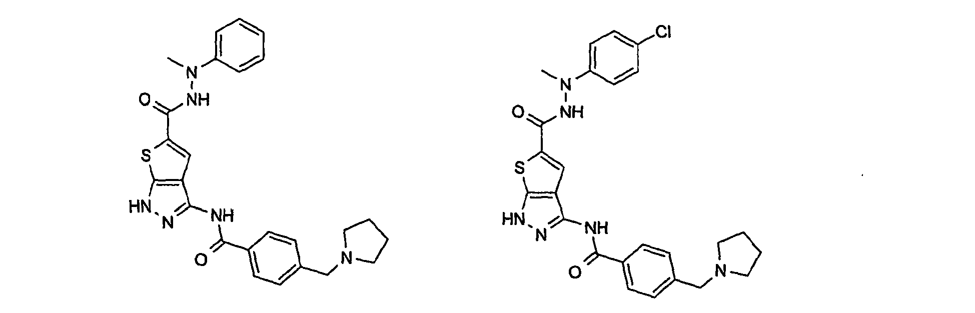

A une solution de 200 mg (0,37 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle dans 6 mL de diméthylformamide sous argon, sont ajoutés 27 mg (74 µmol) d' iodure de tetrabutylammonium puis 93 µL (1,11 mmol) de pyrrolidine. Le mélange réactionnel est agité à une température voisine de 25°C pendant 16 heures, puis il est versé dans de la glace. Il est ensuite extrait 2 fois avec un mélange 50/50 d'acétate d'éthyle et d'heptane. Les extraits organiques sont réunis, séchés sur sulfate de magnésium, puis concentrés à sec sous vide (2 kPa) à une température voisine de 40°C. Le résidu est dissous dans 4 mL de méthanol et la solution est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par LC/MS préparative (conditions B). On obtient ainsi 25 mg de N-[5-(N'-méthyl-N'-phényl-hydrazinocarbonyl)-1H-thiéno[2,3-c]pyrazol-3-yl]-4-pyrrolidin-1-ylméthyl-benzamide sous forme d'une poudre jaune fondant de 172 à 178°C. LC-MS-DAD-ELSD : 475(+)=(M+H)(+).
On isole de la même façon 18 mg de N-[5-(N'-(4-chloro-phényl)-N'-méthyl-hydrazinocarbonyl)-1H-thiéno[2,3-c]pyrazol-3-yl]-4-pyrrolidin-1-ylméthyl-benzamide sous forme d'une poudre jaune pâle fondant de 170 à 179°C.
RMN ¹H (400 MHz, (CD₃)₂SO d₆, δ en ppm): Il s'agit du sel de la structure attendue avec 1,87 (m, 2H) ; 2,05 (m, 2H) ; 3,13 (m, 2H) ; 3,18 (s, 3H) ; 3,40 (m, 2H) ; 4,45 (d, J = 4,5 Hz, 2H) ; 6,82 (d, J = 9,0 Hz, 2H) ; 7,25 (d, J = 9,0 Hz, 2H) ; 7,68 (d, J = 8,0 Hz, 2H) ; 8,16 (d, J = 8,0 Hz, 2H) ; 8,18 (s, 1H) ; 9,85 (m étalé, 1H); 10,85 (s, 1 H) ; 11,35 (s large, 1H); 13,0 (m très étalé, 1H).
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonylrthiéno[2,3-c]pyrazole-1-carboxylate de *tert*-butyle est décrit à l'exemple 11.

### Exemple 19: Préparation du 4-Azétidin-1-ylméthyl-N-[5-(N'-méthyl-N'-phényl-hydrazinocarbonyl)-1H-thiéno[2,3-c]pyrazol-3-yl]-benzamide.

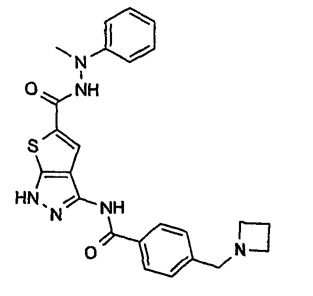

A une solution de 200 mg (0,37 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle dans 6 mL de diméthylformamide sous argon, sont ajoutés 27 mg (74 µmol) d' iodure de tetrabutylammonium puis 75 µL (1,11 mmol) d'azétidine. Le mélange réactionnel est agité à une température voisine de 25°C pendant 16 heures, puis il est versé dans de la glace. Il est ensuite extrait 2 fois avec un mélange 50/50 d'acétate d'éthyle et d'heptane. Les extraits organiques sont réunis, séchés sur sulfate de magnésium, puis concentrés à sec sous vide (2 kPa) à une température voisine de 40°C. Le résidu est dissous dans 4 mL de méthanol et la solution est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par LC/MS préparative (conditions B). On obtient ainsi 3 mg de 4-azétidin-1-ylméthyl-N-[5-(N'-méthyl-N'-phényl-hydrazinocarbonyl)-1H-thiéno[2,3-c]pyrazol-3-yl]-benzamide sous forme d'une poudre jaune fondant de 155 à 160°C. LC-MS-DAD-ELSD : 461 (+)=(M+H)(+).
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle est décrit à l'exemple 11.

### Exemples 20a et 20b: Préparation du 4-{[(2-Diméthylamino-éthyl)-méthyl-amino]-méthyl}-N-[5-(N'-méthyl-N'-phényl-hydrazinocarbonyl)-1H-thiéno[2,3-c]pyrazol-3-yl]-benzamide et du N-{5-[N'-(4-Chloro-phényl)-N'-méthyl-hydrazinocarbonyl]-1H-thiéno[2,3-c]pyrazol-3-yl}-4-([(2-diméthylamino-éthyl)-méthyl-amino]-méthyl)-benzamide.

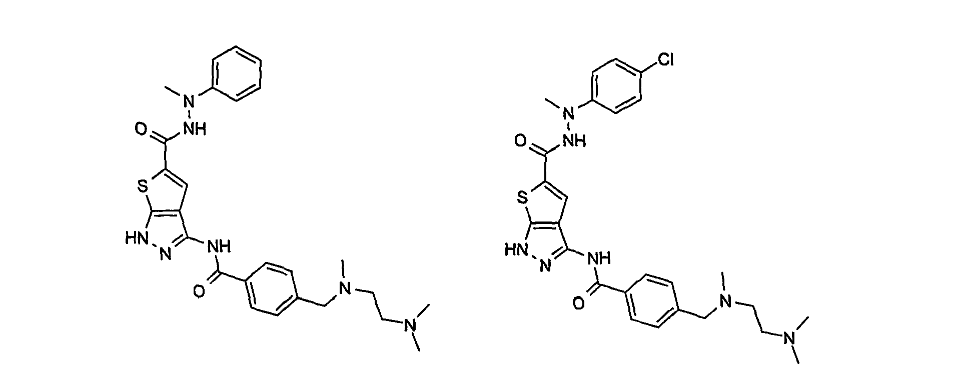

A une solution de 200 mg (0,37 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle dans 6 mL de diméthylformamide sous argon, sont ajoutés 27 mg (74 µmol) d' iodure de tetrabutylammonium puis 144 µL (1,11 mmol) de N,N,N'-triméthyléthylènediamine. Le mélange réactionnel est agité à une température voisine de 25°C pendant 16 heures, puis il est versé dans de la glace. Il est ensuite extrait 2 fois avec un mélange 50/50 d'acétate d'éthyle et d'heptane. Les extraits organiques sont réunis, séchés sur sulfate de magnésium, puis concentrés à sec sous vide (2 kPa) à une température voisine de 40°C. Le résidu est dissous dans 4 mL de méthanol et la solution est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par LC/MS préparative (conditions B). On obtient ainsi 40 mg de 4-{[(2-diméthylamino-éthyl)-méthyl-amino]-méthyl}-N-[5-(N'-méthyl-N'-phényl-hydrazinocarbonyl)-1H-thiéno[2,3-c]pyrazol-3-yl]-benzamide sous forme d'une poudre jaune. LC-MS-DAD-ELSD : 506(+)=(M+H)(+).
On isole de la même façon 26 mg de N-{5-[N'-(4-chloro-phényl)-N'-méthyl-hydrazinocarbonyl]-1H-thiéno[2,3-c]pyrazol-3-yl}-4-{[(2-diméthylamino-éthyl)-méthyl-amino]-méthyl}-benzamide sous forme d'une résine incolore. RMN ¹H (400 MHz, (CD₃)₂SO d₆, δ en ppm): 2,44 (m étalé partiellement masqué, 7H) ; 2,82 (s, 6H) ; 3,17 (s, 3H) ; 3,37 (m large, 2H) ; 6,82 (d, J = 9,0 Hz, 2H) ; 7,25 (d, J = 9,0 Hz, 2H) ; 7,60 (d large, J = 8,0 Hz, 2H) ; 8,12 (d, J = 8,0 Hz, 2H) ; 8,18 (s, 1 H) ; 10,85 (s, 1 H) ; 11,25 (s large, 1 H) ; 13,0 (m très étalé, 1 H) .
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle est décrit à l'exemple 11.

### Exemples 21a et 21b: Préparation du N-[5-(N'-Méthyl-N'-phényl-hydrazinocarbonyl)-1H-thiéno[2,3-c]pyrazol-3-yl]-4-perhydro-1,4-oxazepin-4-ylméthyl-benzamide et du N-{5-[N'-(4-Chloro-phényl)-N'-méthyl-hydrazinocarbonyl]-1H-thiéno[2,3-clpyrazol-3-yl}-4-perhydro-1,4-oxazepin-4-ylméthyl-benzamide.

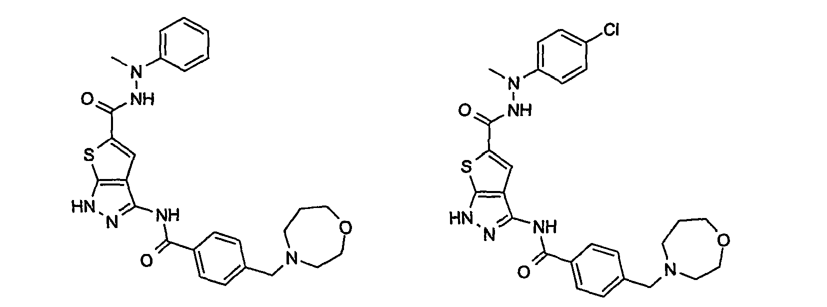

A une solution de 200 mg (0,37 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle dans 6 mL de diméthylformamide sous argon, sont successivement ajoutés 27 mg (74 µmol) d' iodure de tetrabutylammonium, 153 mg (1,11 mmol) de chlorhydrate d'homomorpholine et 153 mg (1,11 mmol) de carbonate de potassium. Le mélange réactionnel est agité à une température voisine de 25°C pendant 16 heures, puis il est versé dans de la glace. Il est ensuite extrait 2 fois avec un mélange 50/50 d'acétate d'éthyle et d'heptane. Les extraits organiques sont réunis, séchés sur sulfate de magnésium, puis concentrés à sec sous vide (2 kPa) à une température voisine de 40°C. Le résidu est dissous dans 4 mL de méthanol et la solution est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par LC/MS préparative (conditions B). On obtient ainsi 34 mg de N-[5-(N'-méthyl-N'-phényl-hydrazinocarbonyl)-1H-thiéno[2,3-c]pyrazol-3-yl]-4-perhydro-1,4-oxazepin-4-ylméthyl-benzamide sous forme d'une poudre jaune fondant de 174 à 179°C. LC-MS-DAD-ELSD : 505(+)=(M+H)(+). On isole de la même façon 38 mg de N-{5-[N'-(4-chloro-phényl)-N'-méthyl-hydrazinocarbonyl]-1H-thiéno[2,3-c]pyrazol-3-yl}-4-perhydro-1,4-oxazepin-4-ylméthyl-benzamide sous forme d'une poudre jaune. RMN ¹H (400 MHz, (CD₃)₂SO d₆, δ en ppm): Il s'agit du sel de la structure attendue avec 2,08 (m, 2H) ; de 3,13 à 4,07 (m, 8H) ; 3,18 (s, 3H) ; 4,50 (s large, 2H) ; 6,82 (d, J = 9,0 Hz, 2H) ; 7,25 (d, J = 9,0 Hz, 2H) ; 7,70 (d large, J = 8,0 Hz, 2H) ; 8,17 (d partiellement masqué, J = 8,0 Hz, 2H) ; 8,18 (s, 1H) ; 9,75 (m étalé, 1H) ; 10,85 (s, 1 H) ; 11,3 (s, 1 H) ; 12,95 (m très étalé, 1 H) .
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert-*butyle est décrit à l'exemple 11.

### Exemples 22a et 22b: Préparation du N-[5-(N'-Méthyl-N'-phényl-hydrazinocarbonyl)-1H-thiéno[2,3-c]pyrazol-3-yl]-3-morpholin-4-ylméthyl-benzamide et du N-{5-[N'-(4-Chloro-phényi)-N'-méthyl-hydrazinocarbonyl]-1H-thiéno[2,3-c]pyrazol-3-yl}-3-morpholin-4-ylméthyl-benzamide.

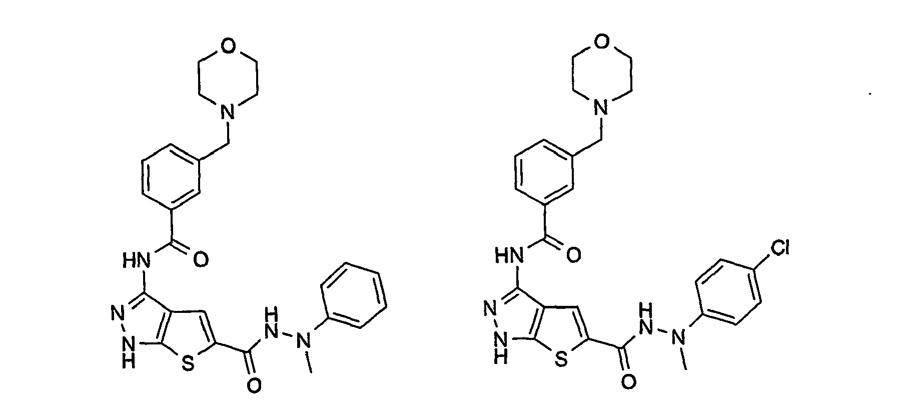

Une solution de 257 mg (0,44 mmol) d'un mélange environ 70:30 de 5-(N'-méthyl-N'-phényl-hydrazinocarbonyl)-3-(3-morpholin-4-ylméthyl-benzoylamino)-thiéno[2,3-c]pyrazofe-1-carboxylate de *tert*-butyle et de 5-(N'-(4-chloro-phényl)-N'-méthyl-hydrazinocarbonyl)-3-(3-morpholin-4-ylméthyl-benzoylamino)-thiéno[2,3-c]pyrazole-1-carboxylate de *tert*-butyle dans 4 mL de méthanol est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par LC/MS préparative (conditions B). On obtient ainsi 105 mg de N-[5-(N'-méthyl-N'-phényl-hydrazinocarbonyl)-1 H-thiéno[2,3-c]pyrazol-3-yl]-3-morpholin-4-ylméthyl-benzamide sous forme d'une poudre jaune fondant de 168 à 178°C. RMN ¹H (400 MHz, (CD₃)₂SO d₆, δ en ppm) : observation du sel de la structure attendue avec un mélange 80 % - 20 % de conformères tel que: 3,16 (m large partiellement masqué, 2H) ; 3,18 (s, 3H) ; 3,32 (m large, 2H) ; 3,63 (m large, 2H) ; 3,98 (m, 2H) ; 4,44 (s large, 2H) ; 6,77 (t, J = 7,5 Hz, 0,8H) ; 6,82 (d, J = 8,0 Hz, 1,6H) ; 6,94 (t, J = 8,0 Hz, 0,2H) ; 7,02 (d, J = 8,0 Hz, 0,4H) ; 7,22 (t, J = 8,0 Hz, 1 ,6H) ; 7,31 (d, J = 8,0 Hz, 0,4H) ; 7,66 (t, J = 8,0 Hz, 1 H) ; 7,74 (d, J = 8,5 Hz, 1 H) ; 8,15 (m, 3H) ; 9,70 (s, 0,2H) ; 10,0 (m étalé, 1 H) ; 10,75 (s, 0,8H) ; 11,25 (s, 0,2H) ; 11,35 (s, 0,8H) ; 12,95 (m très étalé, 1 H) .
On isole de la même façon 34 mg de N-{5-[N'-(4-chloro-phényl)-N'-méthyl-hydrazinocarbonyl]-1 H-thiéno[2,3-c]pyrazol-3-yl}-3-morpholin-4-ylméthyl-benzamide sous forme d'une poudre jaune. RMN ¹H (400 MHz, (CD₃)₂SO d₆, δ en ppm) : Il s'agit d'un sel de la structure attendue avec de 3,07 à 3,55 (m partiellement masqué, 4H) ; 3,18 (s, 3H) ; 3,63 (t large, J = 12,5 Hz, 2H) ; 3,99 (d large, J = 12,5 Hz, 2H) ; 4,44 (s large, 2H) ; 6,82 (d, J = 9,0 Hz, 2H) ; 7,25 (d, J = 9,0 Hz, 2H) ; de 7,60 à 7,76 (m, 2H) ; 8,17 (m, 3H) ; 9,90 (m étalé, 1 H) ; 10,8 (s, 1 H) ; 11,3 (s large, 1 H) ; 13,0 (m très étalé, 1 H) .

### 5-(N'-Méthyl-N'-phényl-hydrazinocarbonyl)-3-[3-(4-méthyl-pipérazin-1-ylméthyl)-benzoylamino]-thiéno[2,3-c]pyrazole-1-carboxylate de tert-butyle et 5-(N'-(4-Chloro-phényl)-N'-méthyl-hydrazinocarbonyl)-3-[3-(4-méthyt-pipérazin-1-ylméthyl)-benzoylamino]-thiéno[2,3-c]pyrazole-1-carboxylate de tert-butyle:

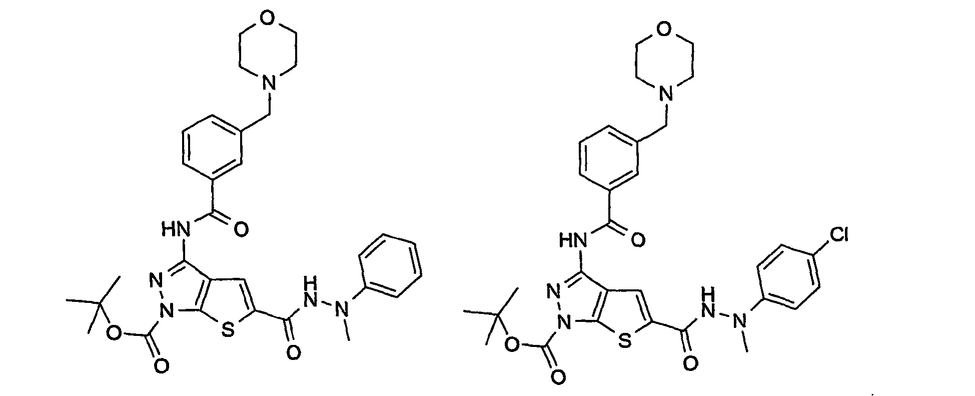

A une solution de 0,20 g (0,52 mmol) d'un mélange environ 70:30 de 3-amino-5-(N'-méthyl-N'-phényl-hydrazinocarbonyl)-thiéno[2,3-c]pyrazole-1-carboxylate de *tert*-butyle et de 3-amino-5-(N'-(4-chloro-phényl)-N'-méthyl-hydrazinocarbonyl)-thiéno[2,3-c]pyrazole-1-carboxylate de *tert*-butyle dans 15 mL de tetrahydrofuranne sous argon, sont ajoutés 214 mg (1,55 mmol) de carbonate de potassium et 285 mg (1,03 mmol) de chlorure de 3-(morpholin-1-ylméthyl)-benzoyle; chlorhydrate. Le mélange réactionnel est agité à une température voisine de 25°C pendant 16 heures, puis il est versé sur de l'eau glacée. Le mélange est extrait à l'acétate d'éthyle. Les phases organiques sont réunies, séchées sur sulfate de magnésium, filtrées, puis concentrées à sec sous vide (2 kPa) à une température voisine de 40°C. Le résidu est purifié par chromatographie sur une cartouche de gel de silice de 30 g (0,015-0,040 mm) en éluant avec du dichlorométhane pur puis avec un mélange dichlorométhane/ méthanol (90/10 en volumes). Les fractions contenant les produits attendus sont réunies puis concentrées à sec sous pression réduite (2 kPa) à une température voisine de 40°C. On obtient ainsi 257 mg d'un mélange environ 70:30 de 5-(N'-méthyl-N'-phényl-hydrazinocarbonyl)-3-(3-morpholin-4-ylméthyl-benzoylamino)-thiéno[2,3-c]pyrazole-1-carboxylate de *tert*-butyle et de 5-(N'-(4-chloro-phény!)-N'-méthyl-hydrazinocarbonyl)-3-(3-morpholin-4-ylméthyl-benzoylamino)-thiéno[2,3-c]pyrazole-1-carboxylate de *tert*-butyle sous forme d'une mousse jaune qui est utilisée directement dans l'étape suivante.
Le mélange environ 70:30 de 3-amino-5-(N'-méthyl-N'-phényi-hydrazinocarbonyl)-thiéno[2,3-c]pyrazole-1-carboxylate de *tert*-butyle et de 3-amino-5-(N'-(4-chloro-phényl)-N'-méthyl-hydrazinocarbonyl)-thiéno[2,3-c]pyrazole-1-carboxylate de *tert*-butyle est décrit à l'exemple 11. Le chlorure de 3-(morpholin-1-ylméthyl)-benzoyle; chlorhydrate peut être préparé selon le brevet WO 9008128 A1.

### Exemples 23a et 23b: Préparation du 4-[(2-Diéthylamino-éthylamino)-méthyl]-N-[5-(N'-méthyl-N'-phényl-hydrazinocarbonyl)-1 H-thiéno[2,3-c]pyrazol-3-yl]-benzamide et du N-{5-[N'-(4-Chloro-phényl)-N'-méthyl-hydrazinocarbonyl]-1H-thiéno[2,3-c]pyrazol-3-yl}-4-[(2-diéthylamino-éthylamino)-méthyl]-benzamide.

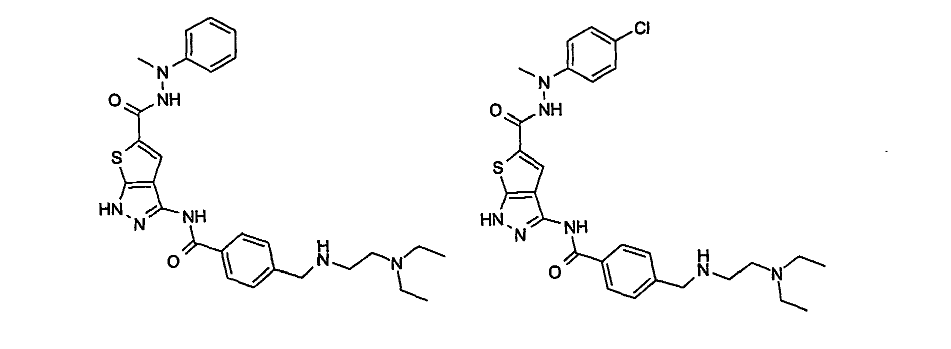

A une solution de 200 mg (0,37 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1 -carboxylate de *tert*-butyle dans 6 mL de diméthylformamide sous argon, sont successivement ajoutés 27 mg (74 µmol) d' iodure de tetrabutylammonium, 156 µL (1,11 mmol) de N,N-diéthyléthylènediamine et 153 mg (1,11 mmol) de carbonate de potassium. Le mélange réactionnel est agité à une température voisine de 25°C pendant 16 heures, puis il est versé dans de la glace. Il est ensuite extrait 2 fois avec un mélange 50/50 d'acétate d'éthyle et d'heptane. Les extraits organiques sont réunis, séchés sur sulfate de magnésium, puis concentrés à sec sous vide (2 kPa) à une température voisine de 40°C. Le résidu est dissous dans 4 mL de méthanol et la solution est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par LC/MS préparative (conditions B). Les fractions contenant les produits attendus sont réunies puis concentrées à sec sous pression réduite (2 kPa) à une température voisine de 40°C. Le résidu est repurifié par chromatographie sur une cartouche de gel de silice de 4. g (0,035-0,060 mm) en éluant avec un mélange dichlorométhane/méthanol puis avec un mélange de dichlorométhane et de méthanol/ammoniaque à 28% (85/15 en volumes). Les fractions contenant les produits attendus sont réunies puis concentrées à sec sous pression réduite (2 kPa) à une température voisine de 40°C. On obtient ainsi 8 mg de 4-[(2-diéthylamino-éthylamino)-méthyl]-N-[5-(N'-méthyl-N'-phényl-hydrazinocarbonyl)-1 H-thiéno[2,3-c]pyrazol-3-yl]-benzamide sous forme d'une poudre jaune pâle fondant de 143 à 150°C. LC-MS-DAD-ELSD : 520(+)/...=(M+H)(+)/...(1 Cl).
On isole de la même façon 10 mg de N-{5-[N'-(4-chloro-phényl)-N'-méthyl-hydrazinocarbonyl]-1 H-thiéno[2,3-c]pyrazol-3-yl}-4-[(2-diéthylamino-éthylamino)-méthyl]-benzamide sous forme d'une poudre jaune. RMN'H (400 MHz, (CD₃)₂SO d₆, δ en ppm): Il s'agit du sel de la structure attendue avec 1,21 (t large, J = 7,0 Hz, 6H) ; 3,18 (s, 3H) ; 3,20 (m étalé partiellement masqué, 6H) ; de 3,29 à 3,54 (m masqué, 2H) ; 4,31 (m large, 2H) ; 6,82 (d, J = 9,0 Hz, 2H) ; 7,25 (d, J = 9,0 Hz, 2H) ; 7,64 (d large, J = 8,0 Hz, 2H) ; 8,16 (d, J = 8,0 Hz, 2H) ; 8,19 (s, 1 H) ; 9,20 (m étalé, 2H) ; 10,85 (s, 1 H) ; 11,3 (s large, 1 H) ; 13,0 (m très étalé, 1 H) .
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle est décrit à l'exemple 11.

### Exemple 24: Préparation du 4-[(Méthyl-phényl-amino)-méthyl]-N-[5-(N'-méthyl-N'-phényl-hydrazinocarbonyl)-1H-thiéno[2,3-c]pyrazol-3-yl]-benzamide.

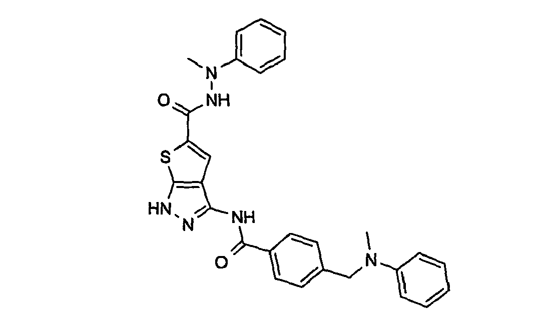

A une solution de 200 mg (0,37 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényi)-N'-méthyl-hydrazinocarbonyl)-thiéno[2,3-c]pyrazole-1-carboxylate de *tert*-butyle dans 6 mL de diméthylformamide sous argon, sont successivement ajoutés 27 mg (74 µmol) d' iodure de tetrabutylammonium et 121 µL (1,11 mmol) de N-méthylaniline. Le mélange réactionnel est agité à une température voisine de 25°C pendant 15 jours, puis il est versé dans de la glace. Il est ensuite extrait 2 fois avec un mélange 50/50 d'acétate d'éthyle et d'heptane. Les extraits organiques sont réunis, lavés avec de la saumure saturée, séchés sur sulfate de magnésium, puis concentrés à sec sous vide (2 kPa) à une température voisine de 40°C. Le résidu est dissous dans 4 mL de méthanol et la solution est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par chromatographie sur une cartouche de gel de silice de 15 g (0,015-0,040 mm) en éluant avec du dichlorométhane pur puis avec un mélange dichlorométhane/méthanol/ammoniaque à 28% (12/3/0,5 en volumes) à un débit de 10 mL/min. Les fractions contenant le produit attendu sont réunies puis concentrées à sec sous pression réduite (2 kPa) à une température voisine de 40°C. Le résidu est purifié par LC/MS préparative (conditions B). On obtient ainsi 11 mg de 4-[(méthyl-phényl-amino)-méthyl]-N-[5-(N'-méthyl-N'-phényl-hydrazinocarbonyl)-1H-thiéno[2,3-c]pyrazol-3-yl]-benzamide sous forme d'une poudre beige fondant à 242°C. LC-MS-DAD-ELSD : 511(+)=(M+H)(+).
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle est décrit à l'exemple 11.

### Exemple 25: Préparation du 4-[(Diisopropylamino)-méthyl]-N-[5-(N'-méthyl-N'-phényl-hydrazinocarbonyl)-1H-thiéno[2,3-c]pyrazol-3-yl]-benzamide; trifluoroacétate.

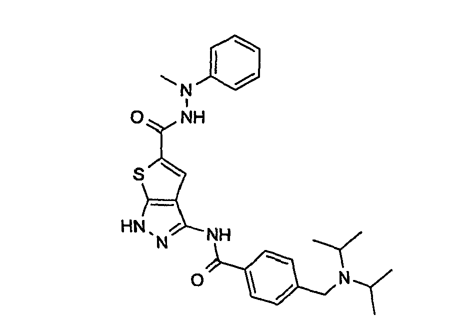

A une solution de 200 mg (0,37 mmol) d'un mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert*-butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle dans 6 mL de diméthylformamide sous argon, sont successivement ajoutés 27 mg (74 µmol) d' iodure de tetrabutylammonium et 156 µL (1,11 mmol) de diisopropylamine. Le mélange réactionnel est agité à une température voisine de 25°C pendant 9 jours, puis 220 µL (1,56 mmol) de diisopropylamine sont rajoutés et le mélange réactionnel est agité à une température voisine de 25°C pendant 6 jours. Il est ensuite versé dans de la glace et extrait 2 fois avec un mélange 50/50 d'acétate d'éthyle et d'heptane. Les extraits organiques sont réunis, lavés avec de la saumure saturée, séchés sur sulfate de magnésium, puis concentrés à sec sous vide (2 kPa) à une température voisine de 40°C. Le résidu est dissous dans 4 mL de méthanol et la solution est introduite dans un tube pour four à micro-ondes. Le tube est bouché et le mélange réactionnel est irradié à une température voisine de 100°C pendant 30 minutes. Le mélange réactionnel est ensuite concentré à sec sous vide (2 kPa) à une température voisine de 40°C. Le résidu est purifié par LC/MS préparative (conditions B). On obtient ainsi 10 mg de 4-[(diisopropylamino)-méthyl]-N-[5-(N'-méthyl-N'-phényl-hydrazinocarbonyl)-1H-thiéno[2,3-c]pyrazol-3-yl]-benzamide; trifluoroacétate sous forme d'une poudre beige fondant de 170 à 182°C. RMN ¹H (400 MHz, (CD₃)₂SO d₆, δ en ppm): pour ce lot, tous les signaux sont larges avec de 1,27 à 1,42 (m, 12H) ; 3,18 (s, 3H) ; 3,70 (m, 2H) ; 4,48 (s, 2H) ; de 6,71 à 6,88 (m, 3H) ; 7,22 (t, J = 8,0 Hz, 2H) ; 7,70 (m, 2H) ; 8,17 (m, 3H) ; 8,55 (s, 1 H) ; 10,75 (s, 1 H) ; 11,3 (s, 1 H) ; 13,05 (s, 1 H) .
Le mélange environ 70:30 de 3-(4-chlorométhyl-benzoylamino)-5-(N'-méthyl-N'-phényl-hydrazinocarbonyl)-thiéno[2,3-c]pyrazole-1-carboxylate de *tert-*butyle et de 3-(4-chlorométhyl-benzoylamino)-5-(N'-(4-chloro-phényl)-N'-méthyl-hydrazinocarbonyl)-thiéno[2,3-c]pyrazole-1-carboxylate de *tert*-butyle est décrit à l'exemple 11.

### Exemple 26: Préparation du N-[5-(N'-Benzyl-hydrazinocarbonyl)-1H-thiéno[2,3-c]pyrazol-3-yl]-4-méthoxy-benzamide.

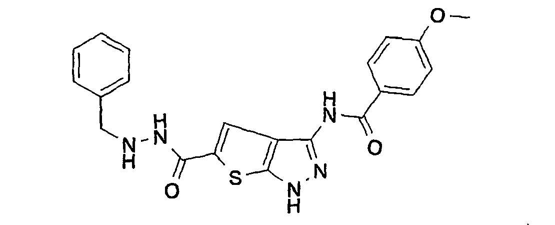

Le N-[5-(N'-benzyl-hydrazinocarbonyl)-1H-thiéno[2,3-*c*]pyrazol-3-yl]-4-méthoxy-benzamide est préparé suivant l'exemple 1. Rdt = 3%. SM: ES m/z=422 MH+ pic de base. RMN ¹H (400 MHz, (CD₃)₂SO d₆, δ en ppm) : 3,85 (s, 3H) ; 3,98 (s, 2H) ; 7,06 (d, J = 8,5 Hz, 2H) ; 7,26 (t, J = 7,5 Hz, 1 H) ; 7,33 (t, J = 7,5 Hz, 2H) ; 7,38 (d, J = 7,5 Hz, 2H) ; 7,94 (s large, 1 H) ; 8,05 (d, J = 8,5 Hz, 2H) ; 10,15 (s, 1 H) ; 10,95 (m étalé, 1 H) ; 12,85 (m très étalé, 1H).

### Exemple 27: Préparation du 4-Méthoxy-N-[5-(N'-pyridin-2-yl-hydrazinocarbonyl)-1H-thiéno[2,3-c]pyrazol-3-yl]-benzamide.

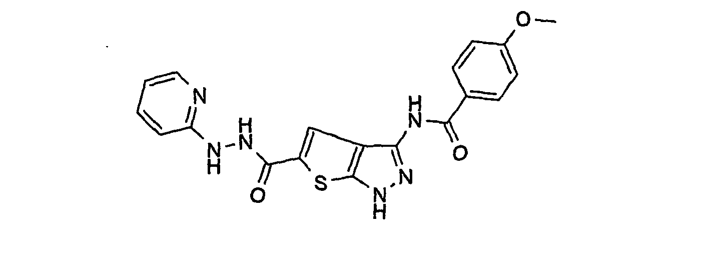

Le 4-méthoxy-N-[5-(N'-pyridin-2-yl-hydrazinocarbonyl)-1H-thiéno[2,3-c]pyrazol-3-yl]-benzamide est préparé suivant l'exemple 1. Rdt = 56%. SM: IE m/z=408 M+. m/z=300 (M-C5H6N3)+ m/z=135 C8H7O2+ pic de base. RMN ¹H (400 MHz, (CD₃)₂SO d₆, δ en ppm): 3,85 (s, 3H) ; 6,63 (d, J = 8,5 Hz, 1H) ; 6,71 (m, 1H) ; 7,07 (d large, J = 8,5 Hz, 2H) ; 7,54 (m, 1H) ; de 8,03 à 8,12 (m, 3H) ; 8,20 (s, 1 H) ; 8,44 (s, 1 H) ; 10,5 (s, 1H) ; 11,0 (s, 1H) ; 12,9 (s, 1H).

### Exemple 28: Préparation du N-{5-[N'-(4-Chloro-phényl)-N'-méthyl-hydrazinocarbonyl]-1H-thiéno[2,3-c]pyrazol-3-yl}-4-(4-méthyl-pipérazin-1-yl)-benzamide.

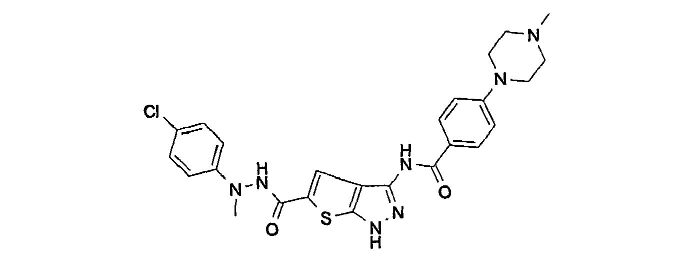

Dans un ballon de 20 mL sec sous argon, on introduit le 1-(1-éthoxy-éthyl)-3-[4-(4-méthyl-pipérazin-1-yl)-benzoylamino]-1H-thiéno[2,3-c]pyrazole-5-carboxylate d'éthyle (0,5 g, 1,03 mmol, 1 éq) et du toluène (5 mL). La suspension est agitée à une température voisine de 0 °C, puis une solution de triméthylaluminium (1,54 mL, 3 éq) est additionnée au goutte à goutte pour éviter des hausses de températures. Une fois l'addition terminée, on laisse agiter pendant 30 min à une température voisine de 25 °C, puis on additionne une solution de N-(4-chloro-phényl)-N-méthyl-hydrazine (0,480 g, 3,09 mmol, 3 éq). Le mélange réactionnel est alors chauffé à une température voisine de 50 °C jusqu'à disparition totale du produit de départ. Après 2h30, on laisse revenir à une température voisine de 25 °C, puis on dilue le mélange avec de l'acétate d'éthyle. On y ajoute quelques gouttes de phosphate monopotassique jusqu'à la fin du dégagement gazeux, puis on laisse agiter pendant 1 heure. Alors, on évapore le solvent puis le résidu est dissous dans un mélange de dichlorométhane et de méthanol. On place cette solution directement sur gel de silice pour purification par chromatographie en éluant au départ avec un mélange méthanol/dichlorométhane (10/90 en volumes). Intermédiaire éthoxyéthyle: LC/MS RT = 3.41 min.; M+H+ 596.1, M-H-594.2. Après évaporation, le solide obtenu est placé directement dans 5 mL de tetrahydrofuranne et 2 mL d'acide chlorhydrique 5N à une température voisine de 50 °C. On obtient ainsi le N-{5-[N'-(4-chloro-phényl)-N'-méthyl-hydrazinocarbonyl]-1H-thiéno[2,3-c]pyrazol-3-yl}-4-(4-méthyl-pipérazin-1-yl)-benzamide (119 mg, 22% pour les deux étapes). MS: ES m/z=524 MH+ pic de base. RMN ¹H (400 MHz, (CD₃)₂SO d₆, δ en ppm) : 2,23 (s, 3H) ; 2,44 (m, 4H) ; 3,17 (s, 3H) ; de 3,25 à 3,33 (m partiellement masqué, 4H) ; 6,82 (d, J = 9,0 Hz, 2H) ; 7,00 (d large, J = 9,0 Hz, 2H) ; 7,24 (d, J = 9,0 Hz, 2H) ; 7,97 (d large, J = 9,0 Hz, 2H) ; 8,16 (s, 1 H) ; 10,8 (s, 1 H) ; 10,9 (m étalé, 1 H) ; 12,9 (m étalé, 1 H) .

### 1-(1-Ethoxy-éthyl)-3-[4-(4-méthyl-pipérazin-1-yl)-benzoylamino]-1H-thiéno[2,3-c]pyrazole-5-carboxylate d'éthyle:

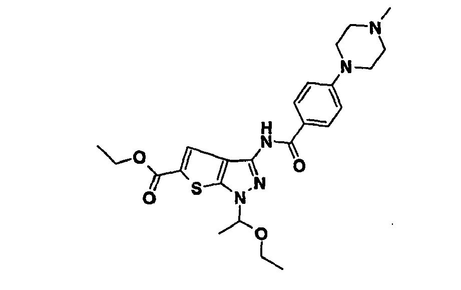

Dans un ballon de 500 mL sous argon est placé le 3-bromo-1-(1-éthoxy-éthyl)-1H-thiéno[2,3-c]pyrazole-5-carboxylate d'éthyle (10,9 g, 31 mmol), le 4-(4-méthyl-pipérazin-1-yl)-benzamide (9 g, 41 mmol, 1,3 éq), de l'iodure de cuivre (I) (0,601 g, 3,1 mmol, 0.1 éq), du phosphate tripotassique (20,1 g, 94 mmol, 3 éq) préalablement pulvérisé, et 70 mL de dioxanne anhydre préalablement dégazé à l'argon. A cette suspension est ajouté à la seringue la N,N'-diméthyléthylènediamine (0,336 mL, 3,16 mmol, 0,1 éq). Le mélange réactionnel est alors chauffé à une température voisine de 110 °C pour une période de 24 heures. Alors, le dioxanne est évaporé sous vide et le résidu est dilué avec de l'acétate d'éthyle (300 mL) et 150 mL d'eau. La phase organique est lavée avec 100 mL d'eau, séchée sur sulfate de magnésium, puis concentrée à sec sous pression réduite. Le résidu est purifié par chromatographie sur gel de silice avec comme éluant de départ un mélange dichlorométhane/méthanol (90/10 en volumes). On obtient ainsi le 1-(1-éthoxy-éthyl)-3-[4-(4-méthyl-pipérazin-1-yl)-benzoylamino]-1H-thiéno[2,3-c]pyrazole-5-carboxylate d'éthyle (4,05 g, 27 %) sous forme d'une huile jaune pâle qui est utilisée directement dans l'étape suivante.
Le 3-bromo-1-(1-éthoxy-éthyl)-1H-thiéno[2,3-c]pyrazole-5-carboxylate d'éthyle est décrit à l'exemple 1.

### N-(4-Chloro-phényl)-N-méthyl-hydrazine:

On place la N'-benzhydrylidène-N-(4-chloro-phényl)-N-méthyl-hydrazine(1,5 g, 4,68 mmol) dans 20 mL de tetrahydrofuranne sous agitation. On y ajoute 5 mL d'acide chlorhydrique 5N puis on laisse agiter la solution à une température voisine de 50 °C pendant 16 heures. Alors, on neutralise la phase aqueuse avec de la soude 5N puis on extrait avec de l'acétate d'éthyle. L'huile obtenue après extraction est purifiée par chromatographie sur gel de silice avec comme éluant de départ un mélange acétate d'éthyle/heptane (10/90 en volumes). On obtient ainsi la N-(4-chloro-phényl)-N-méthyl-hydrazine sous forme d'huile jaune pâle, 0,53 g (72%). (Rf = 0,48, AcOEt/heptane 20/80 (v/v)).

### N'-Benzhydrylidène-N-(4-chloro-phényl)-N-méthyl-hydrazine:

Dans un ballon de 20 mL préalablement séché sous argon on introduit la N-benzhydrylidène-N'-(4-chloro-phényl)-hydrazine (0,2 g, 0,65 mmol, 1 éq) et 3,2 mL de tetrahydrofuranne. La solution est refroidie à une température voisine de -10 °C puis on ajoute une solution 0,5 M d'hexaméthylbis-silylamidure de potassium (1,43 mL, 1,1 éq). La solution s'assombrit, on laisse agiter à cette température pendant 30 min puis on additionne une solution d'iodure de méthyle dans le méthyl *tert*-butyléther (0,49 mL, 1,5 éq). On laisse alors le mélange revenir à une température voisine de 25°C et on laisse agiter pendant 4 heures. Après traitement avec de l'hydrogénocarbonate de sodium, on extrait avec de l'acétate d'éthyle. Les extraits organiques sont réunis, séchés sur sulfate de magnésium puis concentrés à sec sous vide. L'huile résiduelle est purifiée par chromatographie sur gel de silice avec comme éluant un mélange acétate d'éthyle/heptane (10/90 en volumes). La N'-benzhydrylidène-N-(4-chloro-phényl)-N-méthyl-hydrazine est obtenue avec un rendement de 96% sous la forme d'un solide jaune qui est utilisée directement dans l'étape suivante.

### N-Benzhydrylidène-N'-(4-chloro-phényl)-hydrazine:

Dans un ballon de 500 mL, on place 260 mL de 2-méthyl-2-butanol que l'on dégaze à l'argon pendant 30 minutes. Une fois le dégazage fini, on place dans un ballon annexe sec sous argon 60 mL de 2-méthyl-2-butanol, du diacétate de palladium (II) (0,857g, 1,27 mmol, 0,025 éq) et le ligand MePHOS (0,928g, 2,55 mmol, 0,05 éq). On laisse agiter pendant 30 minutes à une température voisine de 25°C. Par ailleurs, dans le ballon de 500 mL, on ajoute le 1,4-dichlorobenzène (7,49 g, 1 éq) et de la soude en poudre (2,85 g, 1,4 éq). Le catalyseur est alors transféré vers le ballon de 500 mL que l'on chauffe à une température voisine de 100 °C. On finit par y ajouter la benzhydrylidène-hydrazine (10 g, 50,9 mmol, 1 éq). On laisse agiter la suspension résultante qui change de couleur rapidement (rouge foncé) jusqu'à disparition totale de l'halogénure. Alors, on évapore le solvant à sec puis on reprend le résidu avec du dichlorométhane. Le solide est filtré sur Célite^{®}, puis lavé abondamment avec du dichlorométhane. La phase organique est lavée avec une solution saturée de chlorure d'ammonium, séchée sur sulfate de magnésium, puis concentrée à sec sous vide. Le résidu est purifiée par chromatographie sur une colonne de gel de silice avec comme éluant de départ un mélange acétate d'éthyle/heptane (10/90 en volumes). On obtient ainsi la N-benzhydrylidène-N'-(4-chloro-phényl)-hydrazine (85%) sous forme d'un solide jaune (Rf = 0,35, AcOEt/Heptane 25/75 (v/v)).

### 4-(4-Méthyl-pipérazin-1-yl)-benzamide:

Dans un ballon de 250 mL, on introduit le 4-fluorobenzonitrile (12 g, 99 mmol, 1 éq) et 100 mL de DMF. A cette solution, on ajoute la N-méthyl pipérazine (16 mL, 1,6 éq, 123 mmol). La solution orange est chauffée à une température voisine de 90°C pendant 15 heures. Alors, on évapore le solvent à sec puis on dilue le résidu avec 500 mL d'éther diéthylique. La solution est lavée avec une solution d'hydrogénocarbonate de sodium (2 x 100 mL) puis par une solution saturée de chlorure de sodium (100 mL). Après évaporation, le 4-(4-méthyl-pipérazin-1-yl)-benzonitrile (solide orange, 10 g, 77%) est utilisé sans autre purification dans l'étape suivante d'hydrolyse. Il est ajouté à une température voisine de 0 °C sur une solution d'acide sulfurique à 98% (25 mL) et 5 mL d'eau. On chauffe alors la solution pourpre à une température voisine de 100 °C pendant 8 heures. La solution est refroidie puis hydrolysée en la versant sur de la glace. Le pH est réajusté à 9 -10 avec de la soude en pastilles. Le précipité obtenu est filtré et lavé abondamment avec de l'eau puis du tetrahydrofuranne qui solubilise partiellement le produit. On décante l'eau. Après évaporation à sec de la phase organique, on obtient un solide qui est purifié par chromatographie sur gel de silice avec comme éluant un mélange méthanol/dichlorométhane (15/85 en volumes). On obtient ainsi le 4-(4-méthyl-pipérazin-1-yl)-benzamide (8,7g, 80%) sous forme d'un solide blanc qui est utilisé directement dans l'étape suivante.

### Exemple 29: Préparation du N-(2-Méthoxyméthyl-pyrrolidin-1-yl)-3-[4-(4-méthyl-pipérazin-1-yl)-benzoylaminol-1H-thiéno[2,3-c]pyrazole-5-carboxamide.

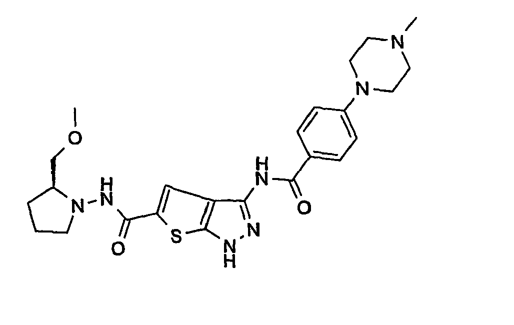

Le N-(2-Méthoxyméthyl-pyrrolidin-1-yl)-3-[4-(4-méthyl-pipérazin-1-yl)-benzoylamino]-1H-thiéno[2,3-c]pyrazole-5-carboxamide est préparé suivant l'exemple 28. LC/MS intermédiaire éthoxyéthyle RT = 2.62 min., M+H+ 571.1, M-H- 568.1 ; Produit final: Rdt = 33%. MS: ES m/z=498 MH+ pic de base. RMN ¹H (400 MHz, (CD₃)₂SO d₆, δ en ppm) : 1,58 (m, 1 H) ; de 1,70 à 1,87 (m, 2H) ; 2,00 (m, 1 H) ; 2,25 (s, 3H) ; 2,48 (m partiellement masqué, 4H) ; 2,86 (m large, 1 H) ; de 2,98 à 3,36 (m étalé, 3H) ; 3,21 (s, 3H) ; 3,32 (m, 4H) ; 3,46 (m, 1H) ; 6,98 (d, J = 9,0 Hz, 2H) ; 7,96 (d, J = 9,0 Hz, 2H) ; 8,05 (s large 1 H).

### Exemple 30: Préparation du N-[5-(N'-Benzyl-N'-méthyl-hydrazinocarbonyl)-1H-thiéno[2,3-c]pyrazol-3-yl]-4-(4-méthyl-pipérazin-1-yl)-benzamide.

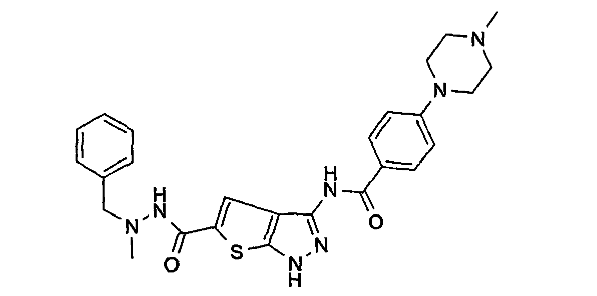

Le N-[5-(N'-benzyl-N'-méthyl-hydrazinocarbonyl)-1H-thiéno[2,3-c]pyrazol-3-yl]-4-(4-méthyl-pipérazin-1-yl)-benzamide est préparé suivant l'exemple 28. LC/MS intermédiaire éthoxyéthyle RT = 3.19 min., M+H+ 576.1, M-H- 574.2. Produit final : Rdt =16%. MS: ES m/z=504 MH+ pic de base. RMN ¹H(400 MHz, (CD₃)₂SO d₆, δ en ppm) : les signaux du spectre sont larges avec un mélange de rotamères 60 % - 40 %: 2,36 (s, 3H) ; de 2,54 à 2,72 (m, 7H) ; 3,36 (m partiellement masqué, 4H) ; 3,89 (s, 1,2H) ; 3,98 (s, 0,8H) ; 7,02 (d, J = 8,5 Hz, 2H) ; de 7,16 à 7,45 (m, 5H) ; 7,89 (s, 0,6H) ; 7,99 (d, J = 8,5 Hz, 2H) ; 8,08 (s, 0,4H) ; 8,85 (s, 0,4H) ; 9,58 (s, 0,6 H) ; 9,70 (m étalé, 0,4H) ; 10,7 (m étalé, 0,6H) ; 12,65 (m étalé, 0,4H) ; 12,85 (m étalé, 0,6H).

### Exemple 31: Préparation du N-(Pipéridin-1-yl)-3-[4-(4-méthyl-pipérazin-1-yl)-benzoylamino]-1H-thiéno[2,3-c]pyrazole-5-carboxamide.

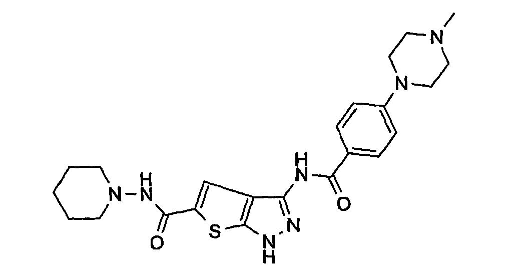

Le N-(pipéridin-1-yl)-3-[4-(4-Méthyl-pipérazin-1-yl)-benzoylamino]-1H-thiéno[2,3-c]pyrazole-5-carboxamide est préparé suivant l'exemple 28. LC/MS intermédiaire éthoxyéthyle RT = 2.97 min., M+H+ 540.4, M-H- 538.2. Produit final : Rdt = 38%. SM: ES m/z=468 MH+ m/z-234.8 (M + 2H)2+ /2 pic de base. RMN ¹H (400 MHz, (CD₃)₂SO d₆, δ en ppm) : 1,55 (m large, 2H) ; 1,88 (m large, 4H) ; 2,87 (s, 3H) ; de 3,05 à 3,21 (m, 4H) ; de 3,43 à 3,59 (m, 6H) ; de 4,02 à 4,10 (m, 2H) ; 7,10 (d, J = 9,0 Hz, 2H) ; 8,04 (d, J = 9,0 Hz, 2H) ; 8,27 (s, 1H).

### Exemple 32: Préparation du N-[5-(N'-Benzyl-hydrazinocarbonyl)-1H-thiéno[2,3-c]pyrazol-3-yl]-4-(4-méthyl-pipérazin-1-yl)-benzamide.

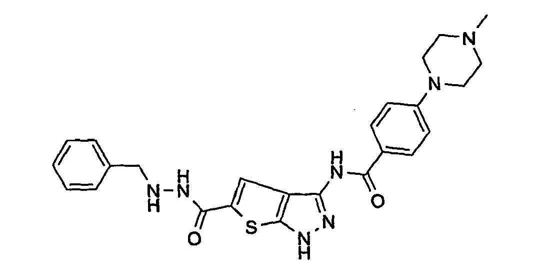

Le N-[5-(N'-benzyl-hydrazinocarbonyl)-1H-thiéno[2,3-c]pyrazol-3-yl]-4-(4-méthyl-pipérazin-1-yl)-benzamide est préparé suivant l'exemple 28. LC/MS intermédiaire éthoxyéthyle RT = 3.10, MH+ 562.3, MH- 560.2. Produit final : Rdt = 13%. SM: ES m/z=490 MH+ pic de base. RMN ¹H (400 MHz, (CD₃)₂SO d₆, δ en ppm) : 2,23 (s, 3H) ; 2,44 (m, 4H) ; 3,30 (m partiellement masqué, 4H) ; 3,97 (d, J = 5,0 Hz, 2H) ; 5,37 (m, 1 H) ; 7,00 (d, J = 9,0 Hz, 2H) ; 7,25 (t, J = 7,5 Hz, 1 H) ; 7,33 (t, J = 7,5 Hz, 2H) ; 7,38 (d, J = 7,5 Hz, 2H) ; de 7,92 à 7,98 (m, 3H) ; 10,1 (d, J = 5,0 Hz, 1H); 10,8 (m très étalé, 1 H) ; 12,9 (m très étalé, 1H).

### Exemple 33: Préparation du N-[5-(N'-Ethyl-N'-phényl-hydrazinocarbonyl)-1H-thiéno[2,3-c]pyrazol-3-yl]-4-(4-méthyl-pipérazin-1-yl)-benzamide.

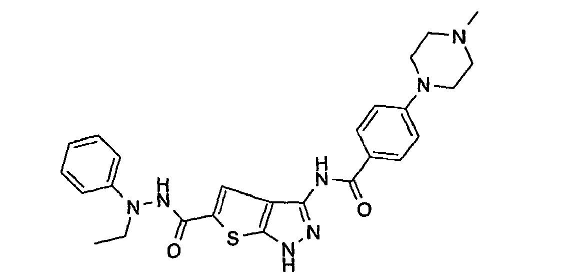

Le N-[5-(N'-éthyl-N'-phényl-hydrazinocarbonyl)-1H-thiéno[2,3-c]pyrazol-3-yl]-4-(4-méthyl-pipérazin-1-yl)-benzamide est préparé suivant l'exemple 28. LC/MS intermédiaire éthoxyéthyle: RT = 3.25 min., M+H+ 576.3, M-H- 574.1. Produit final: Rdt = 28%. SM: ES m/z=504 MH+ pic de base. RMN ¹H (400 MHz, (CD₃)₂SO d₆, δ en ppm) : 1,18 (t, J = 7,0 Hz, 3H) ; 2,21 (s, 3H) ; 2,43 (m, 4H) ; 3,31 (m partiellement masqué, 4H) ; 3,53 (q, J = 7,0 Hz, 2H) ; 6,74 (t, J = 7,5 Hz, 1H); 6,82 (d, J = 7,5 Hz, 2H) ; 7,01 (d large, J = 8,5 Hz, 2H) ; 7,21 (t, J = 7,5 Hz, 2H) ; 7,98 (d large, J = 8,5 Hz, 2H) ; 8,22 (s, 1H) ; 10, 55 (s, 1 H) ; 10,85 (m étalé, 1 H) ; 12,9 (m étalé, 1 H) .

### Exemple 34: Préparation du N-[5-(N'-Benzyl-N'-phényl-hydrazinocarbonyl)-1H-thiéno[2,3-c]pyrazol-3-yl]-4-(4-méthyl-pipérazin-1-yl)-benzamide.

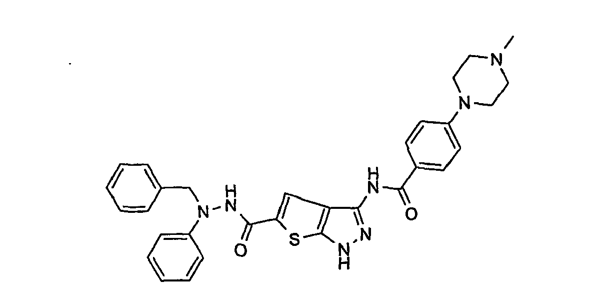

Le N-[5-(N'-benzyl-N'-phényl-hydrazinocarbonyl)-1H-thiéno[2,3-c]pyrazol-3-yl]-4-(4-méthyl-pipérazin-1-yl)-benzamide est préparé suivant l'exemple 28. LC/MS intermédiaire éthoxyéthyle: RT = 3.68 min., M+H+ 638.5. Produit final: Rdt = 8%. SM: ES m/z=566 MH+ pic de base. RMN ¹H (400 MHz, (CD₃)₂SO d₆, δ en ppm) : tous les signaux du spectre sont larges avec 2,32 (s, 3H) ; 2,59 (m, 4H) ; 3,35 (m partiellement masqué, 4H) ; 4,75 (s, 2H) ; 6,75 (t, J= 7,5 Hz, 1 H) ; 6,81 (d, J = 8,5 Hz, 2H) ; 7,02 (d, J = 8,5 Hz, 2H) ; de 7,11 à 7,38 (m, 5H) ; 7,49 (d, J = 8,5 Hz, 2H) ; 7,98 (d, J = 8,5 Hz, 2H) ; 8,17 (s, 1 H) ; 10,8 (s, 1 H) ; 10,9 (m étalé, 1 H) ; 12,95 (m étalé, 1 H) .

### Exemple 35: Préparation du N-[5-(N'-Méthyl-N'-phényl-hydrazinocarbonyl)-1H-thiéno[2,3-c]pyrazol-3-yl]-4-(4-méthyl-pipérazin-1-yl)-benzamide.

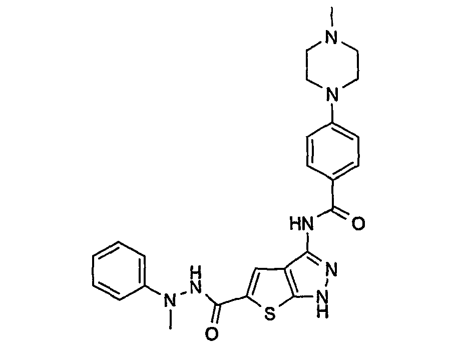

Le N-[5-(N'-méthyl-N'-phényl-hydrazinocarbonyl)-1H-thiéno[2,3-c]pyrazol-3-yl]-4-(4-méthyl-pipérazin-1-yl)-benzamide est préparé suivant l'exemple 28. LC/MS intermédiaire éthoxyéthyle: RT = 2.74 min., M+H+ 561.0. Produit final: Rdt = 22%. SM: ES m/z=490 MH+ pic de base. RMN ¹H (400 MHz, (CD₃)₂SO d₆, δ en ppm) : tous les signaux du spectre sont larges avec 2,32 (s, 3H) ; 2,59 (m, 4H) ; 3,35 (m partiellement masqué, 4H) ; 4,75 (s, 2H) ; 6,75 (t, J= 7,5 Hz, 1 H) ; 6,81 (d, J = 8,5 Hz, 2H) ; 7,02 (d, J = 8,5 Hz, 2H) ; de 7,11 à 7,38 (m, 5H) ; 7,49 (d, J = 8,5 Hz, 2H) ; 7,98 (d, J = 8,5 Hz, 2H) ; 8,17 (s, 1 H) ; 10,8 (s, 1 H) ; 10,9 (m étalé, 1 H) ; 12,95 (m étalé, 1 H).

### Exemple 36: Préparation du N-{5-[N'-(4-Chloro-phényl)-N'-cyclobutylméthyl-hydrazinocarbonyl]-1H-thiéno[2,3-c]pyrazol-3-yl}-4-(4-méthyl-pipérazin-1-yl)-benzamide.

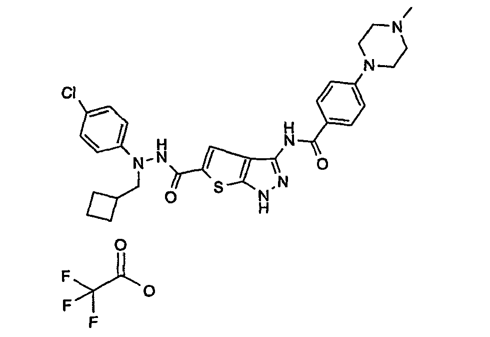

Le N-{5-[N'-(4-chloro-phényl)-N'-cyclobutylméthyl-hydrazinocarbonyl]-1H-thiéno[2,3-c]pyrazol-3-yl}-4-(4-méthyl-pipérazin-1-yl)-benzamide est préparé suivant l'exemple 28. LC/MS intermédiaire : éthoxyéthyle RT = 4.20 min., M+H+ 650.1, M-H- 648.1. Produit final: Rdt = 2%. MS: ES m/z=578 MH+ pic de base. RMN ¹H (400 MHz, (CD₃)₂SO d₆, δ en ppm) : De 1,71 à 1,88 (m, 3H) ; 2,03 (m, 1 H) ; 2,63 (m, 1 H) ; 2,85 (s, 3H) ; 3,14 (m large, 4H) ; de 3,47 à 3,56 (m partiellement masqué , 2H) ; 3,51 (d, J = 7,0 Hz, 2H) ; 4,07 (m large, 2H) ; 6,83 (d, J = 9,0 Hz, 2H) ; 7,11 (d, J = 9,0 Hz, 2H) ; 7,22 (d, J = 9,0 Hz, 2H) ; 8,04 (d, J = 9,0 Hz, 2H) ; 8,20 (s, 1H); 10,05 (m étalé, 1 H) ; 10,6 (s, 1 H) ; 10,95 (m étalé, 1 H) ; 12,9 (m étalé, 1 H).

### N-(4-Chloro-phényl)-N-cyclobutylméthyl-hydrazine:

La N-(4-Chloro-phényl)-N-cyclobutylméthyl-hydrazine est préparée suivant l'exemple 28
Rdt. (2 étapes), 0,65 g (63%). Rf = 0,43, (AcOEt/heptane 30/70 (v/v)).

### N'-Benzhydrylidène-N-(4-chloro-phényl)-N-cyclobutylméthyl-hydrazine:

Rf = 0,77, (AcOEt/heptane 30/70 (v/v)) (deprotection immédiate). La N-benzhydrylidène-N'-(4-chloro-phényl)-hydrazine est décrite à l'exemple 28.

### Exemple 37: Préparation du N-{5-[N'-Ethyl-N'-(4-fluoro-phényl)-hydrazinocarbonyl]-1H-thiéno[2,3-c]pyrazol-3-yl}-4-(4-méthyl-pipérazin-1-yl)-benzamide.

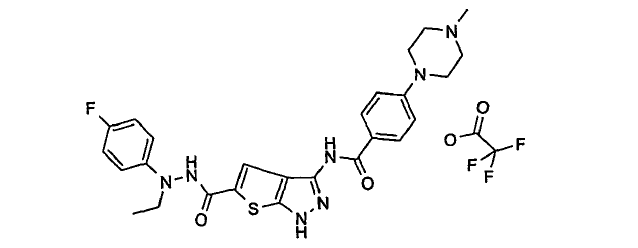

Le N-{5-[N'-éthyl-N'-(4-fluoro-phényl)-hydrazinocarbonyl]-1H-thiéno[2,3-c]pyrazol-3-yl}-4-(4-méthyl-pipérazin-1-yl)-benzamide est préparé suivant l'exemple 28. LC/MS intermédiaire éthoxyéthyle RT = 3.20 min., M+H+ 594.2, M-H- 592.3. Produit final : Rdt =16%. MS: ES m/z=522 MH+ pic de base. RMN ¹H (400 MHz, (CD₃)₂SO d₆, δ en ppm) : observation d'un mélange de rotamères 80 %- 20 %: 1,16 (t, J = 7,0 Hz, 2,4H) ; 1,31 (t, J = 7,0 Hz, 0,6H) ; 2,87 (s, 3H) ; de 3,02 à 3,20 (m, 4H) ; de 3,35 à 3,59 (m partiellement masqué, 2H); 3,50 (q partiellement masqué, J = 7,0 Hz, 1,6H); 4,08 (m, 2H) ; 4,30 (q, J = 7,0 Hz, 0,4H) ; 6,83 (dd, J = 4,5 et 9,0 Hz, 1,6H) ; de 7,00 à 7,17 (m, 4,4H) ; 8,03 (d, J = 9,0 Hz, 2H) ; 8,17 (s, 0,2H) ; 8,21 (s, 0,8H) ; 9,67 (s, 0,2H) ; 9,77 (m étalé, 1 H) ; 10,55 (s, 0,8H) ; 10,95 (m, 1 H) ; 12,9 (m très étalé, 1H).

### N-Ethyl-N-(4-fluoro-phényl)-hydrazine:

La N-Ethyl-N-(4-fluoro-phényl)-hydrazine est réparée suivant l'exemple 28 Huile jaune pâle, 0,32 g (30%).

### N'-Benzhydrylidène-N-éthyl-N-(4-fluoro-phényl)-hydrazine:

La N'-Benzhydrylidène-N-éthyl-N-(4-fluoro-phényl)-hydrazine est préparée suivant l'exemple 28.
Solide orange, 1,9 g (86%), Rf = 0,64 (AcOEt/heptane 20/80 (v/v)).

### N-Benzhydrylidène-N'-(4-fluoro-phényl)-hydrazine:

La N-Benzhydrylidène-N'-(4-fluoro-phényl)-hydrazine est préparée suivant l'exemple 28.
Solide jaune, rendement 54% (Rf = 0,33, AcOEt/Heptane 10/90 (v/v)), LC/MS MH+ 291.1, MH- 289.2.

### Exemple 38: Préparation du N-{5-[N'-(4-Fluoro-phényl)-N'-(2-méthoxy-éthyl)-hydrazinocarbonyl]-1H-thiéno[2,3-c]pyrazol-3-yl}-4-(4-méthyl-pipérazin-1-yl)-benzamide.

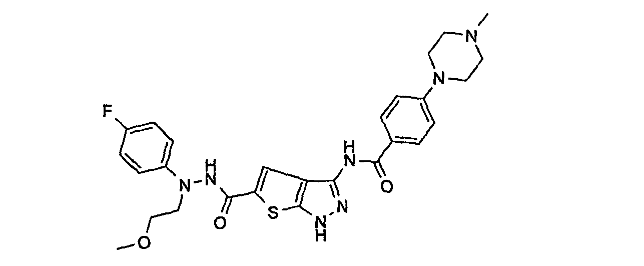

Le N-{5-[N'-(4-fluoro-phényl)-N'-(2-méthoxy-éthyl)-hydrazinocarbonyl]-1H-thiéno[2,3-c]pyrazol-3-yl}-4-(4-méthyl-pipérazin-1-yl)-benzamide est préparé suivant l'exemple 28. Rdt = 45%. MS: ES m/z=552 MH+ pic de base., RMN ¹H (400 MHz, (CD₃)₂SO d₆, δ en ppm) : 2,23 (s, 3H) ; 2,44 (m, 4H) ; 3,27 (s, 3H) ; de 3,28 à 3,31 (m, 4H) ; 3,58 (m, 2H) ; 3,64 (m, 2H) ; 6,83 (dd, J = 4,5 et 9,0 Hz, 2H) ; de 6,98 à 7,06 (m, 4H) ; 7,98 (d, J = 8,5 Hz, 2H) ; 8,21 (s large, 1 H) ; 10,7 (s, 1 H) ; 10,9 (m étalé, 1 H) ; 12,9 (m étalé, 1 H).

### N-(4-Fluoro-phényl)-N-(2-méthoxy-éthyl)-hydrazine:

La N-(4-Fluoro-phényl)-N-(2-méthoxy-éthyl)-hydrazine est préparée suivant l'exemple 28.
0,57 g (60%). Rf = 0,10 (AcOEt/heptane 20/80 (v/v)), LC/MS RT = 4.18, MH 183.3.

### N'-Benzhydrylidène-N-(4-fluoro-phényl)-N-(2-méthoxy-éthyl)-hydrazine:

La N'-Benzhydrylidène-N-(4-fluoro-phényl)-N-(2-méthoxy-éthyl)-hydrazine est préparée suivant l'exemple 28.
1,2 g (66%). Rf = 0,26 (AcOEt/heptane 10/90 (v/v)). La N-benzhydrylidène-N'-(4-fluoro-phényl)-hydrazine est décrite à l'exemple 37.

### Exemple 39: Préparation du N-[5-(N'-Ethyl-N'-o-tolyl-hydrazinocarbonyl)-1H-thiéno[2,3-c]pyrazol-3-yl]-4-(4-méthyl-pipérazin-1-yl)-benzamide.

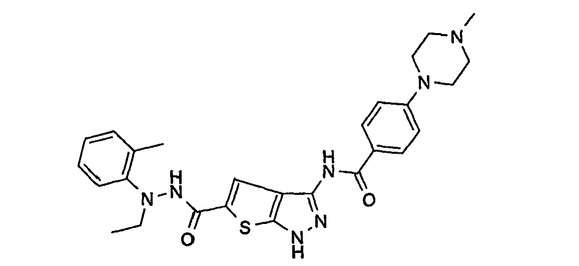

Le N-[5-(N'-éthyl-N'-*o*-tolyl-hydrazinocarbonyl)-1H-thiéno[2,3-c]pyrazol-3-yl]-4-(4-méthyl-pipérazin-1-yl)-benzamide est préparé suivant l'exemple 28. Rdt = 14% ; MS: ES m/z=518 MH+ pic de base. RMN ¹H (400 MHz, (CD₃)₂SO d₆, δ en ppm) : 1,15 (t, J = 7,0 Hz, 3H) ; 2,23 (s, 3H) ; 2,29 (s, 3H) ; 2,44 (m, 4H) ; de 3,23 à 3,33 (m partiellement masqué, 6H) ; 6,96 (t, J = 7,5 Hz, 1H) ; 7,00 (d, J = 9,0 Hz, 2H) ; de 7,12 à 7,17 (m, 2H) ; 7,29 (d, J = 8,0 Hz, 1 H) ; 7,98 (d, J = 8,5 Hz, 2H) ; 8,12 (s, 1 H) ; 10,2 (s, 1H) ; 11,0 (m étalé, 1 H) ; 12,9 (m très étalé, 1 H).

### Exemple 40: Préparation du N-[5-(N'-Ethyl-N'-m-tolyl-hydrazinocarbonyl)-1H-thiéno[2,3-c]pyrazol-3-yl]-4-(4-méthyl-pipérazin-1-yl)-benzamide.

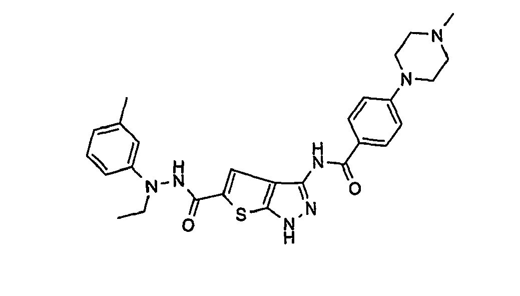

Le N-[5-(N'-éthyl-N'-*m*-tolyl-hydrazinocarbonyl)-1H-thiéno[2,3-c]pyrazol-3-yl]-4-(4-méthyl-pipérazin-1-yl)-benzamide est préparé suivant l'exemple 28. Rdt = 18%. MS: ES m/z=518 MH+ pic de base. RMN ¹H (400 MHz, (CD₃)₂SO d₆, δ en ppm) : observation d'un mélange de rotamères 80 % - 20%: 1,16 (t, J = 7,0 Hz, 3H) ; 2,22 (s, 3H) ; 2,24 (s, 2,4H) ; 2,28 (s, 0,6H) ; 2,44 (m, 4H) ; de 3,25 à 3,35 (m masqué, 4H) ; 3,52 (q, J = 7,0 Hz, 2H) ; 6,57 (d, J = 7,5 Hz, 0,8H) ; de 6,59 à 6,66 (m, 1 ,6H) ; 6,73 (d, J = 7,5 Hz, 0,2H) ; de 6,81 à 6,87 (m, 0,4H) ; 7,00 (d, J = 9,0 Hz, 2H) ; 7,08 (t, J = 7,5 Hz, 0,8H) ; 7,17 (t, J = 7,5 Hz, 0,2H) ; 7,99 (d, J = 9,0 Hz, 2H) ; 8,16 (s, 0,2H) ; 8,22 (0,8H) ; 9,62 (m étalé, 0,2H) ; 10,5 (s, 0,8H) ; 11,1 (m étalé, 1H); 12,9 (m étalé, 1H).

### Exemple 41: Préparation du N-[5-(N'-Ethyl-N'-m-tolyl-hydrazinocarbonyl)-1H-thiéno[2,3-c]pyrazol-3-yl]-4-(4-méthyl-pipérazin-1-yl)-benzamide.

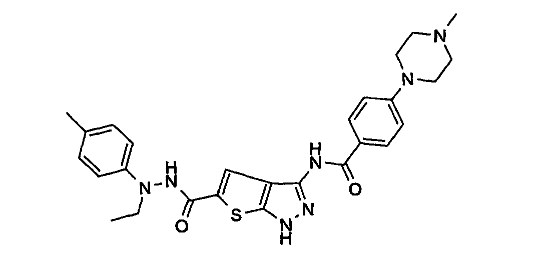

Le N-[5-(N'-éthyl-N'-*m*-tolyl-hydrazinocarbonyl)-1H-thiéno[2,3-c]pyrazol-3-yl]-4-(4-méthyl-pipérazin-1-yl)-benzamide est préparé suivant l'exemple 28. Rdt = 3%. SM: ES m/z=518 MH+ pic de base. RMN ¹H (400 MHz, (CD₃)₂SO d₆, δ en ppm) : 1,15 (t, J = 7,0 Hz, 3H) ; 2,20 (s, 3H) ; 2,23 (s, 3H) ; 2,45 (m, 4H) ; de 3,20 à 3,41 (m masqué, 4H) ; 3,49 (q, J = 7,0 Hz, 2H) ; 6,73 (d, J = 9,0 Hz, 2H) ; de 6,96 à 7,04 (m, 4H) ; 7,99 (d, J = 9,0 Hz, 2H) ; 8,21 (s, 1 H) ; 10,45 (s, 1 H) ; 11,25 (m étalé, 1 H) ; 13,1 (m étalé, 1 H).

### Exemple 42: Préparation du N-{5-[N'-(4-Fluoro-phényl)-N'-isobutyl-hydrazinocarbonyl]-1H-thiéno[2,3-c]pyrazol-3-yl}-4-(4-méthyl-pipérazin-1-yl)-benzamide.

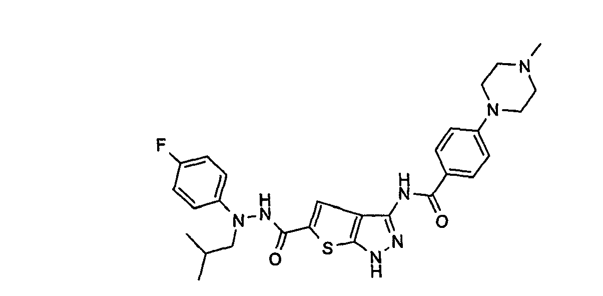

Le N-{5-[N'-(4-fluoro-phényl)-N'-isobutyl-hydrazinocarbonyl]-1H-thiéno[2,3-c]pyrazol-3-yl}-4-(4-méthyl-pipérazin-1-yl)-benzamide est préparé suivant l'exemple 28. Rdt = 59%, SM: ES m/z=550 MH+ pic de base. RMN ¹H (400 MHz, (CD₃)₂SO d₆, δ en ppm) : 0,97 (d, J = 6,5 Hz, 6H) ; 1,91 (m, 1H); 2,22 (s, 3H); 2,44 (m, 4H); de 3,24 à 3,34 (m partiellement masqué, 6H) ; 6,81 (dd, J = 4,5 et 9,0 Hz, 2H) ; de 6,98 à 7,06 (m, 4H) ; 7,98 (d, J = 9,0 Hz, 2H) ; 8,21 (s, 1 H) ; 10,6 (s, 1 H) ; 11,0 (m étalé, 1 H) ; 13,0 (m étalé, 1H).

### N-(4-Fluoro-phényl)-N-isobutyl-hydrazine:

La N-(4-Fluoro-phényl)-N-isobutyl-hydrazine est préparée suivant l'exemple 28.
Huile incolore, 0,62 g (82%). Rf = 0,34 (AcOEt/heptane 20/80 (v/v)), LC/MS RT = 2.48 min..

### N'-Benzhydrylidène-N-(4-fluoro-phényl)-N-isobutyl-hydrazine:

La N'-Benzhydrylidène-N-(4-fluoro-phényl)-N-isobutyl-hydrazine est préparée suivant l'exemple 28.
Solide jaune, 1,51 g (44%). Rf= 0,52 (acétone/heptane 3/97 (v/v)), LC/MS RT= 6.03 min., pas d'ionisation. La N-benzhydrylidène-N'-(4-fluoro-phényl)-hydrazine est décrite à l'exemple 37.

### Exemple 43: Préparation du N-{5-[N'-(3-Bromo-phényl)-N'-éthyl-hydrazinocarbonyl]-1H-thiéno[2,3-c]pyrazol-3-yl}-4-(4-méthyl-pipérazin-1-yl)-benzamide.

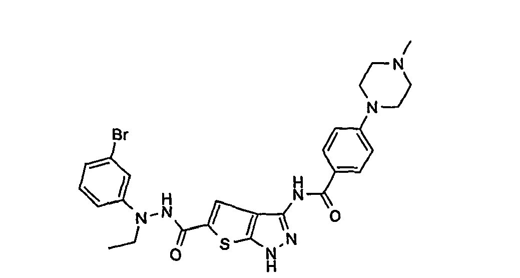

Le N-{5-[N'-(3-bromo-phényl)-N'-éthyl-hydrazinocarbonyl]-1H-thiéno[2,3-c]pyrazol-3-yl)-4-(4-méthyl-pipérazin-1-yl)-benzamide est préparé suivant l'exemple 28. Rdt = 5%. SM: ES m/z=582 MH⁺ pic de base. RMN ¹H (400 MHz, (CD₃)₂SO d₆, δ en ppm) : 1,16 (t, J = 7,0 Hz, 3H) ; 2,22 (s, 3H) ; 2,44 (m, 4H) ; de 3,26 à 3,34 (m partiellement masqué, 4H) ; 3,54 (q, J = 7,0 Hz, 2H) ; 6,82 (dd, J = 2,0 et 8,0 Hz, 1 H) ; 6,89 (dd, J = 2,0 et 8,0 Hz, 1H) ; 6,92 (t, J = 2,5 Hz, 1 H) ; 7,01 (d, J = 9,0 Hz, 2H) ; 7,16 (t, J = 8,5 Hz, 1H) ; 7,98 (d, J = 9,0 Hz, 2H) ; 8,24 (s, 1H) ; 10,7 (s, 1H); 10,95 (m étalé, 1H) ; 12,95 (m étalé, 1 H).

### N-(3-Bromo-phényl)-N-éthyl-hydrazine:

La N-(3-Bromo-phényl)-N-éthyl-hydrazine est préparée suivant l'exemple 28. Huile incolore, 2,80 g, quantitatif. LC/MS RT = 2.70.

### N'-Benzhydrylidène-N-(3-bromo-phényl)-N-éthyl-hydrazine:

La N'-Benzhydrylidène-N-(3-bromo-phényl)-N-éthyl-hydrazine est préparée suivant l'exemple 28.
Solide orange, 13,5 g (80%). Rf = 0,46 (Et₂O/heptane 3/97 (v/v)).

### N-Benzhydrylidène-N'-(3-bromo-phényl)-hydrazine:

Dans un ballon de 250 mL, on place le chlorhydrate de la (3-bromo-phényl)-hydrazine (10 g, 44 mmol, 1 éq), la benzophénone (8,14 g, 44 mmol, 1éq), et de l'éthanol (100 mL). On ajoute alors 2 mL d'acide sulfurique concentré. La suspension résultante est chauffée à une température voisine de 80 °C. Après 30 min à cette température, la suspension devient une solution. On agite à cette température pendant 18 heures, puis on concentre à 95% la solution. Le résidu est dilué avec de l'acétate d'éthyle et lavé avec une solution d'hydrogénocarbonate de sodium. Après séchage sur sulfate de magnésium, la solution est concentrée et le résidu est purifié par chromatographie sur une colonne de gel de silice avec comme éluant un mélange éther/heptane (3/97 en volumes). On obtient ainsi 15 g (95%) de N-benzhydrylidène-N'-(3-bromo-phényl)-hydrazine.

### Exemple 44: Préparation du 6-(4-Méthyl-[1,4]diazepan-1-yl)-N-[5-(N'-méthyl-N'-phényl-hydrazinocarbonyl)-1H-thiéno[2,3-c]pyrazol-3-yl]-nicotinamide.

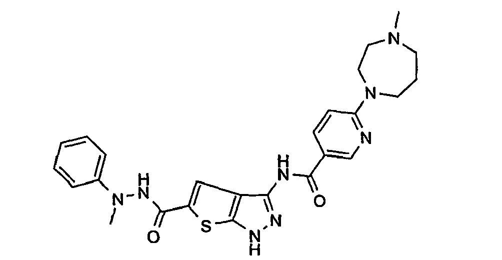

Le 6-(4-méthyl-[1,4]diazepan-1-yl)-N-[5-(N'-méthyl-N'-phényl-hydrazinocarbonyl)-1H-thiéno[2,3-c]pyrazol-3-yl]-nicotinamide est préparé suivant l'exemple 28. MS: ES m/z=505 MH+ pic de base. RMN ¹H (400 MHz, (CD₃)₂SO d₆, δ en ppm) : 1,89 (m, 2H) ; 2,26 (s, 3H) ; 2,47 (m partiellement masqué, 2H) ; 2,61 (m, 2H) ; 3,17 (s, 3H) ; 3,67 (t, J = 6,0 Hz, 2H) ; 3,80 (m, 2H) ; 6,71 (d, J = 9,0 Hz, 1 H) ; 6,76 (t, J = 7,5 Hz, 1H) ; 6,82 (d, J = 8,0 Hz, 2H) ; 7,22 (m, 2H) ; 8,15 (dd partiellement masqué, J = 2,5 et 9,0 Hz, 1H) ; 8,16 (s, 1H) ; 8,80 (d, J = 2,5 Hz, 1 H) ; 10,75 (s, 1 H) ; 11,0 (m étalé, 1 H) ; 12,9 (m, étalé, 1 H).

### 4-(4-Méthyl-[1,4]diazepan-1-yl)-benzamide:

Dans un ballon de 100 mL, on introduit le 4-fluorobenzonitrile (5 g, 41 mmol, 1 éq) et 35 mL de diméthylformamide. A cette solution, on ajoute la N-méthyl homopipérazine (5,18 g, 45 mmol, 1,1 éq). La solution est chauffée à une température voisine de 90 °C pendant 36 heures, puis le solvant est évaporé à sec. Le résidu est dilué avec 150 mL d'acétate d'éthyle et 30 mL d'eau. La phase aqueuse est extraite avec de l'acétate d'éthyle et les phases organiques réunies sont séchées sur sulfate de magnésium. Après évaporation, on purifie l'huile résiduelle par chromatographie sur une colonne de gel de silice avec comme éluant un gradient 0-10% méthanol/ dichlorométhane (Rf = 0,2 ,méthanol/dichlorométhane 10/90 (v/v)). Le 4-(4-méthyl-[1,4]diazepan-1-yl)-benzonitrile (solide jaune) est placé directement dans un mélange de 20 mL d'acide sulfurique à 98% et de 2 mL d'eau. Cette solution orange est chauffée à une température voisine de 100 °C pendant 18 heures, puis elle est refroidie et hydrolysée en la versant sur de la glace. Le pH est réajusté à 9 -10 avec de la soude en pastilles. Le précipité jaune obtenu est filtré et lavé abondamment avec de l'eau puis séché sous vide. On obtient ainsi le 4-(4-méthyl-[1,4]diazepan-1-yl)-benzamide (3,7g, 38%) sous forme d'un solide jaune qui est utilisé directement dans l'étape suivante.

### Exemple 45: Préparation du N-[5-(Benzylidène-hydrazinocarbonyl)-1H-thiéno[2,3-c]pyrazol-3-yl]-4-(4-méthyl-pipérazin-1-yl)-benzamide.

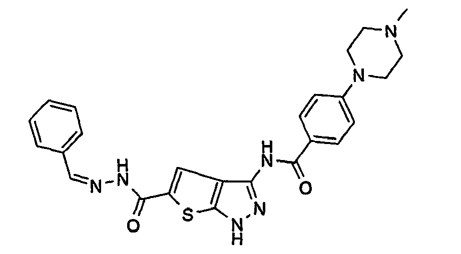

Dans un ballon de 5 mL sec sous argon, on place le N-(5-hydrazinocarbonyl-1H-thiéno[2,3-c]pyrazol-3-yl)-4-(4-méthyl-pipérazin-1-yl)-benzamide (0,150 g, 0,318 mmol, 1 éq), de l'éthanol (1 mL) et à la seringue du benzaldéhyde (0,048 mL, 0,47 mmol, 1,3 éq). On ajoute à la suspension deux gouttes d'acide chlorhydrique 5N. Le mélange réactionnel est agité à une température voisine de 50 °C pendant 3 heures, puis le solvant est évaporé et le résidu est placé directement sur une colonne de gel de silice neutralisé. L'élution est faite avec un mélange méthanol/dichlorométhane (10/90 en volumes). Le produit obtenu est directement additionné à un mélange de 1 mL de tetrahydrofuranne et 0.5 mL d'acide chlorhydrique 5N. Le précipité obtenu est filtré, lavé à l'eau et séché. Le N-[5-(benzylidène-hydrazinocarbonyl)-1H-thiéno[2,3-c]pyrazol-3-yl]-4-(4-méthyl-pipérazin-1-yl)-benzamide est obtenu sous forme d'un solide blanc (0,029g, 16 %). MS: ES m/z=488 MH+ pic de base.. RMN ¹H (400 MHz, (CD₃)₂SO de, δ en ppm) : 2,53 (s partiellement masqué, 3H) ; 2,87 (m, 4H) ; 3,47 (m, 4H) ; 7,03 (d, J = 9,0 Hz, 2H) ; de 7,39 à 7,49 (m, 3H) ; 7,79 (d, J = 7,5 Hz, 2H) ; 8,01 (d, J = 9,0 Hz, 2H) ; 8,28 (s, 1 H) ; 8,39 (s, 1 H) .

### N-(5-hydrazinocarbonyl-1H-thiéno[2,3-c]pyrazol-3-yl)-4-(4-méthyl-pipérazin-1-yl)-benzamide:

Dans un ballon de 15 mL, on introduit le 3-[4-(4-méthyl-pipérazin-1-yl)-benzoylamino]-1H-thiéno[2,3-c]pyrazole-5-carboxylate d'éthyle (0,5 g, 1,03 mmol, 1 éq), de l'éthanol (2 mL) et on ajoute une solution 1 M d'hydrazine (3,09 mL, 3,09 mmol) ainsi que 2 mL d'hydrazine monohydrate. Le mélange réactionnel est agité à reflux pendant 5 heures, puis le solvant est évaporé et le résidu est repris dans l'eau. Le précipité blanc est filtré, lavé à l'eau, puis séché à une température voisine de 80 °C sous vide. On obtient ainsi le N-(5-hydrazinocarbonyl-1H-thiéno[2,3-c]pyrazol-3-yl)-4-(4-méthyl-pipérazin-1-yl)-benzamide (0,15 g, 49 %), Rf = 0,32 (méthanol/dichlorométhane 10/90 (v/v)), qui est utilisé directement dans l'étape suivante. Le 3-[4-(4-méthyl-pipérazin-1-yl)-benzoylamino]-1H-thiéno[2,3-c]pyrazole-5-carboxlate d'éthyle peut être obtenu par déprotection du 1-(1-éthoxy-éthyl)-3-[4-(4-méthyl-pipérazin-1-yl)-benzoylamino]-1H-thiéno[2,3-c]pyrazole-5-carboxylate d'éthyle selon l'exemple 28.

### Exemple 46: Préparation du N-[5-(N'-Méthyl-N'-phényl-hydrazinocarbonyl)-1H-thiéno[2,3-c]pyrazol-3-yl]-4-(2-morpholin-4-yl-éthoxy)-benzamide.

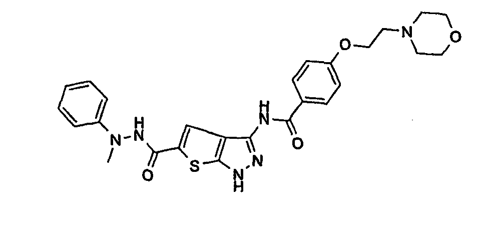

Le N-[5-(N'-méthyl-N'-phényl-hydrazinocarbonyl)-1H-thiéno[2,3-c]pyrazol-3-yl]-4-(2-morpholin-4-yl-éthoxy)-benzamide est préparé suivant l'exemple 28. (intermédiaire éthoxyéthyle: LC/MS RT = 3.10 min., MH+ 593.5). La purification de l'intermédiaire se fait sur gel de silice avec comme éluant un mélange méthanol/dichlorométhane (10/90 en volumes). Le traitement de l'étape de déprotection est effectué de la façon suivante: le mélange réactionnel est traité avec de l'eau puis on extrait avec 5 mL d'acétate d'éthyle. La phase aqueuse est basifiée avec de la soude 5N puis extraite à nouveau avec de l'acétate d'éthyle (2 x 5 mL). Cette phase organique est séchée sur sulfate de magnésium puis concentrée à sec. Le solide jaune obtenu est trituré avec de l'heptane. Après filtration du solide, celui ci est séché sous vide pour donner le N-[5-(N'-méthyl-N'-phényl-hydrazinocarbonyl)-1H-thiéno[2,3-c]pyrazol-3-yl]-4-(2-morpholin-4-yl-éthoxy)-benzamide, 0,192 g (40% pour deux étapes). SM: ES m/z=521 MH+ pic de base. RMN ¹H (400 MHz, (CD₃)₂SO d₆, δ en ppm) : 2,48 (m partiellement masqué, 4H) ; 2,72 (t, J = 6,0 Hz, 2H) ; 3,17 (s, 3H) ; 3,58 (m, 4H) ; 4,18 (t, J = 6,0 Hz, 2H) ; 6,76 (t, J = 7,5 Hz, 1 H) ; 6,82 (d, J = 8,5 Hz, 2H) ; de 7,00 à 7,10 (m, 3H) ; 7,22 (t large, J = 8,0 Hz, 2H) ; 8,05 (d large, J = 8,5 Hz, 2H) ; 8,16 (s large, 1 H) ; 10,75 (s large, 1 H) ; 10,9 (m très étalé, 1 H) ; 12,9 (m très étalé, 1 H) .

### 4-(2-Morpholin-4-yl-éthoxy)-benzamide:

Dans un ballon de 250 mL, on introduit du carbonate de potassium (15,1 g, 109 mmol, 3 éq), de l'acétonitrile (73 mL) puis le 4-hydroxy-benzamide (5 g, 36,5 mmol, 1 éq). La suspension est agitée pendant 30 min avant addition du chlorhydrate de la 4-(2-chloro-éthyl)-morpholine (8,82 g, 47 mmol, 1,3 éq). A cette suspension on ajoute 10 mL de diméthylformamide, puis le mélange est agité à une température voisine de 40 °C pendant 16 heures. On évapore alors le solvant puis on dilue à l'eau et on extrait avec du dichlorométhane. La phase aqueuse est acidifiée avec de l'acide chlorhydrique 5 N et réextraite au dichlorométhane. Les phases organiques sont combinées, séchées sur sulfate de magnésium, puis concentrées à sec sous vide. On obtient ainsi le 4-(2-morpholin-4-yl-éthoxy)-benzamide 2,17 g (23%) sous forme d'un solide blanc qui est utilisé directement dans l'étape suivante.

### Exemple 47: Préparation du 4-(4-méthyl-[1,4]diazepan-1-yt)-N-[5-(N'-méthyl-N'-phényl-hydrazinocarbonyl)-1H-thiéno[2,3-c]pyrazol-3-yl]-benzamide.

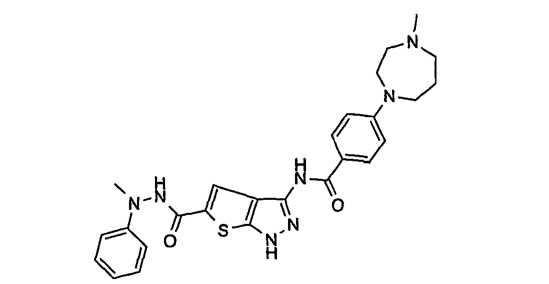

Le 4-(4-méthyl-[1,4]diazepan-1-yl)-N-[5-(N'-méthyl-N'-phényl-hydrazinocarbonyl)-1 H-thiéno[2,3-c]pyrazol-3-yl]-benzamide est préparé suivant l'exemple 28. SM: IE m/z=503 M+. m/z=382 (M - C7H9N2)+ m/z=217 C12H15N3O+. pic de base. RMN ¹H (400 MHz, (CD₃)₂SO d₆, δ en ppm) : 1,90 (m, 2H) ; 2,26 (s, 3H) ; 2,45 (m, 2H) ; 2,62 (m, 2H) ; 3,17 (s, 3H) ; 3,52 (t, J = 6,0 Hz, 2H) ; 3,59 (m, 2H) ; de 6,73 à 6,79 (m, 3H) ; 6,82 (d, J = 8,5 Hz, 2H) ; 7,21 (t, J = 7;5 Hz, 2H) ; 7,94 (d, J = 8,5 Hz, 2H) ; 8,16 (s, 1 H) ; 10,7 (s, 1 H) ; 10,8 (m étalé, 1 H) ; 12,9 (m étalé, 1 H). Le 4-(4-méthyl-[1,4]diazepan-1-yl)-benzamide est décrit à l'exemple 44.

### Analyses par LC/MS :

Les analyses LC/MS ont été réalisées sur un appareil Micromass modèle LCT relié à un appareil HP 1100. L'abondance des produits a été mesurée à l'aide d'un détecteur à barrette de diodes HP G1315A sur une gamme d'onde de 200-600 nm et un détecteur à dispersion de lumière Sedex 65. L'acquisition des spectres de masses a été réalisée sur une gamme de 180 à 800 unités de masse atomique. Les données ont été analysées en utilisant le logiciel Micromass MassLynx. La séparation a été effectuée sur une colonne Hypersil Gold de Thermo C18 50x3 mm, particules de 3µm en éluant par un gradient linéaire de 5 à 95% d'acétonitrile contenant 0,1% (v/v) d'acide formique dans l'eau contenant 0,1% (v/v) d'acide formique en 5 mn à un débit de 0,8 mL/mn. Le temps total d'analyse, incluant la période de rééquilibration de la colonne, est de 7 mn. Les spectres MS ont été réalisés en électrospray (ES⁺/ES⁻) sur un appareil Platform II (Micromass). Les principaux ions observés sont décrits.

### Purification par LC/MS:

Les produits peuvent être purifiés par LC/MS en utilisant un système Waters FractionsLynx composé d'une pompe à gradient Waters modèle 600, d'une pompe de régénération Waters modèle 515, d'une pompe de dilution Waters Reagent Manager, d'un auto-injecteur Waters modèle 2700, de deux vannes Rheodyne modèle LabPro, d'un détecteur à barrette de diodes Waters modèle 996, d'un spectromètre de masse Waters modèle ZMD et d'un collecteur de fractions Gilson modèle 204. Le système est contrôlé par le logiciel Waters FractionLynx. Avant purification, les échantillons sont préparés de la façon suivante : chaque échantillon est solubilisé dans le diméthylsulfoxyde à une concentration voisine de 60 mg/mL et injecté à raison de 1 mL par injection.

Méthode A : La séparation est effectuée alternativement sur deux colonnes Waters Symmetry (C₁₈, 5µM, 19x50 mm), une colonne étant en cours de régénération par un mélange eau/acétonitrile 95/5 (v/v) contenant 0,07% (v/v) d'acide trifluoroacétique, pendant que l'autre colonne était en cours de séparation. L'élution des colonnes est conduite en utilisant un gradient linéaire en 11 minutes de 20 à 95% d'acétonitrile contenant 0,07% (v/v) d'acide trifluoroacétique dans l'eau contenant 0,07% (v/v) d'acide trifluoroacétique, à un débit de 10 mL/min. A la sortie de la colonne de séparation le flux de solvant est divisé par un système LC Packing Accurate : un millième de l'effluent dilué par du méthanol à un débit de 0,5 mL/mn et envoyé vers les détecteurs, à raison de 75% vers le détecteur à barrette de diodes, et les 25% restants vers le spectromètre de masse. Le reste de l'effluent (999/1000) est envoyé vers le collecteur de fractions où le flux est éliminé tant que la masse du produit attendu n'est pas détectée par le logiciel FractionLynx. Les formules moléculaires des produits attendus sont fournies au logiciel FractionLynx qui déclenche la collecte du produit quand le signal de masse détecté correspond à l'ion [M+H]⁺ et/ou au [M+Na]⁺. Dans certains cas, dépendant des résultats de LC/MS analytique, quand un ion intense correspondant à [M+2H]⁺⁺ a été détecté, la valeur correspondant à la moitié de la masse moléculaire calculée (MW/2) est aussi fournie au logiciel FractionLynx. Dans ces conditions, la collecte est aussi déclenchée quand le signal de masse de l'ion [M+2H]⁺⁺ et/ou [M+Na+H]⁺⁺ sont détectés. Les produits sont collectés en tube de verre tarés. Après collecte, les solvants sont évaporés, dans des évaporateurs centrifuges Savant AES 2000 ou Genevac HT8 et les masses de produits sont déterminées par pesée des tubes après évaporation des solvants. Alternativement, les solvants sont évaporés à sec dans un évaporateur rotatif sous vide (2 kPa) à une température voisine de 40°C. Chaque produit obtenu est analysé par LC/MS.

Méthode A' : La séparation est effectuée de la même manière que la méthode A, l'élution des colonnes est effectuée avec un gradient linéaire en 11 minutes de 5 à 95% d'acétonitrile contenant 0,07% (v/v) d'acide trifluoroacétique dans l'eau contenant 0,07% (v/v) d'acide trifluoroacétique, à un débit de 10 mL/min.

Méthode B : La séparation est effectuée sur une colonne Waters SunFire (C₁₈, 5µM, 30x100 mm), L'élution est conduite en utilisant un gradient linéaire en 11 minutes de 5 à 95% d'acétonitrile contenant 0,07% (v/v) d'acide trifluoroacétique dans l'eau contenant 0,07% (v/v) d'acide trifluoroacétique, à un débit de 30 mL/min. A la sortie de la colonne de séparation, le flux de solvant est divisé par un système LC Packing Accurate : un dix-millième de l'effluent dilué par du méthanol à un débit de 1 mL/mn et envoyé vers les détecteurs, à raison de 75% vers le détecteur à barrette de diodes, et les 25% restants vers le spectromètre de masse. Le reste de l'effluent (9999/10000) est envoyé vers le collecteur de fractions où le flux est éliminé tant que la masse du produit attendu n'est pas détectée par le logiciel FractionLynx. Les formules moléculaires des produits attendus sont fournies au logiciel FractionLynx qui déclenche la collecte du produit quand le signal de masse détecté correspond à l'ion [M+H]⁺ et/ou au [M+Na]⁺. Dans certains cas, dépendant des résultats de LC/MS analytique, quand un ion intense correspondant à [M+₂H]⁺⁺ a été détecté, la valeur correspondant à la moitié de la masse moléculaire calculée (MW/2) est aussi fournie au logiciel FractionLynx. Dans ces conditions, la collecte est aussi déclenchée quand le signal de masse de l'ion [M+2H]⁺⁺ et/ou [M+Na+H]⁺⁺ sont détectés. Les produits sont collectés en tube de verre. Après collecte, les solvants sont évaporés, dans un évaporateur centrifuge Jouan RC1010. Alternativement, les solvants sont évaporés à sec dans un évaporateur rotatif sous vide (2 kPa) à une température voisine de 40°C. Chaque produit obtenu est analysé par LC/MS.

Les résidus secs sont repris par du méthanol et de nouveau évaporés conduisant à des produits secs sous forme de poudre.

Méthode B' : La séparation est effectuée de la même manière que la méthode B, l'élution des colonnes est effectuée avec un gradient linéaire en 11 minutes de 10 à 95% d'acétonitrile contenant 0,07% (v/v) d'acide trifluoroacétique dans l'eau contenant 0,07% (v/v) d'acide trifluoroacétique, à un débit de 30 mL/min.

Les produits selon l'invention peuvent être sous forme non chirale, ou racémique, ou enrichie en un stéréo-isomère, ou enrichie en un énantiomère ; et peuvent éventuellement être salifiés.

Un produit conforme à l'invention pourra être utilisé pour la fabrication d'un médicament utile pour traiter un état pathologique, en particulier un cancer.

La présente invention concerne aussi les compositions thérapeutiques contenant un composé selon l'invention, en association avec un excipient pharmaceutiquement acceptable selon le mode d'administration choisi. La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

Les formes liquides seront de préférence injectables et, de ce fait, auront une formulation acceptable pour une telle utilisation.

Des voies d'administration par injection acceptables incluent les voies intraveineuse, intra-péritonéale, intramusculaire, et sous cutanée, la voie intraveineuse étant préférée.

La dose administrée des composés de l'invention sera adaptée par le praticien en fonction de la voie d'administration du patient et de l'état de ce dernier.

Les composés de la présente invention peuvent être administrés seuls ou en mélange avec d'autres anticancéreux. Parmi les associations possibles on peut citer :
- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine
- les dérivés du platine comme notamment le cisplatine, le carboplatine ou l'oxaliplatine
- les agents antibiotiques comme notamment la bléomycine, la mitomycine, la dactinomycine
- les agents antimicrotubules comme notamment la vinblastine, la vincristine, la vindésine, la vinorelbine, les taxoides (paclitaxel et docétaxel)
- les anthracyclines comme notamment la doxorubicine, la daunorubicine, l'idarubicine, l'épirubicine, la mitoxantrone, la losoxantrone
- les agents inhibiteurs de topoisomérases des groupes I et II telles que l'étoposide, le teniposide, l'amsacrine, l'irinotecan, le topotecan et le tomudex
- les fluoropyrimidines telles que le 5-fluorouracile, l'UFT, la floxuridine
- les analogues de cytidine telles que la 5-azacytidine, la cytarabine, la gemcitabine, la 6-mercaptomurine, la 6-thioguanine
- les analogues d'adénosine telles que la pentostatine, la cytarabine ou le phosphate de fludarabine
- le méthotrexate et l'acide folinique
- les enzymes et composés divers tels que la L-asparaginase, l'hydroxyurée, l'acide trans-rétinoique, la suramine, la dexrazoxane, l'amifostine, l'herceptin ainsi que les hormones oestrogéniques, androgéniques
- les agents antivasculaires tels que les dérivés de la combretastatine ou de la colchicine et leurs prodrogues.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

La progression du cycle cellulaire est souvent gérée par des kinases cycline dépendantes (CDK) qui sont activées par une interaction avec des proteines appartenant à la famille des cyclines, activation qui se termine par la phosphorylation de substrats et finalement par la division cellulaire. En plus les inhibiteurs endogènes des CDK qui sont activés (famille des INK4 et des KIP/CIP) régulent de façon négative l'activité des CDK. La croissance des cellules normales est due à une balance entre les activateurs des CDK (les cyclines) et les inhibiteurs endogènes des CDK. Dans plusieurs types de cancers, l'expression ou l'activité aberrante de plusieurs de ces régulateurs du cycle cellulaire a été décrite.

La cycline E active la kinase Cdk2 qui agit ensuite pour phosphoryler la protéine pRb (protéine du rétinoblastome) résultant en un engagement dans la division cellulaire irréversible et une transition vers la phase S (PL Toogood, Medicinal Research Reviews (2001), 21(6) ; 487-498. La kinase CDK2 et peut être CDK3 sont nécessaires pour la progression dans la phase G1 et l'entrée en phase S. Lors de la formation de complexe avec la cycline E, elles maintiennent l'hyperphosphorylation de pRb pour aider la progression de la phase G1 en phase S. Dans les complexes avec la Cycline A, CDK2 joue un rôle dans l'inactivation de E2F et est nécessaire pour la réalisation de la phase S (TD. Davies et al. (2001) Structure 9, 389-3).

Le complexe CDK1/cycline B régule la progression du cycle cellulaire entre la phase G2 et la phase M. La régulation négative du complexe CDK/Cycline B empêche les cellules normales d'entrer en phase S avant que la phase G2 ait été correctement et complètement achevée. (K.K. Roy and E.A. Sausville Current Pharmaceutical Design, 2001, 7,1669-1687.

Un niveau de régulation de l'activité des CDK existe. Les activateurs de cycline dépendantes kinases (CAK) ont une action positive de régulation des CDK. CAK phosphoryle les CDK sur le résidu thréonine pour rendre l'enzyme cible totalement active.

La présence de défauts dans les molécules intervenant sur le cycle cellulaire entraîne l'activation des CDK et la progression du cycle, il est normal de vouloir inhiber l'activité des enzymes CDK pour bloquer la croissance cellulaire des cellules cancéreuses.

De nombreuses protéines impliquées dans la ségrégation des chromosomes et l'assemblage du fuseau ont été identifiées dans la levure et la drosophile. La désorganisation de ces protéines conduit à la non ségrégation des chromosomes et à des fuseaux monopolaires ou désorganisés. Parmi ces protéines, certaines kinases, dont **Aurora** et Ipl1, provenant respectivement de drosophile et de S. *cerevisiae,* sont nécessaires pour la ségrégation des chromosomes et la séparation du centrosome. Un analogue humain de Ipl1 de levure a été récemment cloné et caractérisé par différents laboratoires. Cette kinase, nommée Aurora 2, STK15 ou BTAK appartient à la famille des kinases à sérine/thréonine. Bischoff et al. ont montré que Aurora 2 est oncogène, et est amplifié dans les cancers colorectaux humains (EMBO J, 1998, 17, 3052-3065). Cela a également été exemplifié dans des cancers impliquant des tumeurs épithéliales telles que le cancer du sein.

**Tie-2** (TEK) est un membre d'une famille de récepteurs à tyrosine kinase, spécifique des cellules endothéliales. Tie2 est le premier récepteur à activité tyrosine kinase dont on connaît à la fois l'agoniste (angiopoïetine 1 ou Ang1) qui stimule l'autophosphorylation du récepteur et la signalisation cellulaire [S.

Davis et al (1996) Cell 87, 1161-1169*]* et l'antagoniste (angiopoïetine 2 ou Ang2) [P.C. Maisonpierre et al. (1997) Science 277, 55-60]. L'angiopoïetine 1 peut synergiser avec le VEGF dans les derniers stades de la néo-angiogénèse [AsaharaT. Circ. Res.(1998) 233-240]. Les expériences de knock-out et les manipulations transgéniques de l'expression de Tie2 ou de Ang1 conduisent à des animaux qui présentent des défauts de vascularisation [D.J. Dumont et al (1994) Genes Dev. 8, 1897-1909 et C. Suri (1996) Cell 87, 1171-1180]*.* La liaison d'Ang1 à son récepteur conduit à l'autophosphorylation du domaine kinase de Tie2 qui est essentielle pour la néovascularisation ainsi que pour le recrutement et l'interaction des vaisseaux avec les péricytes et les cellules musculaires lisses ; ces phénomènes contribuent à la maturation et la stabilité des vaisseaux nouvellement formés [P.C. Maisonpierre et al (1997) Science 277, 55-60]*.* Lin et al (1997) J. Clin. invest. 100, 8: 2072-2078 *et* Lin P. (1998) PNAS 95, 8829-8834, ont montré une inhibition de la croissance et de la vascularisation tumorale, ainsi qu'une diminution des métastases de poumon, lors d'infections adénovirales ou d'injections du domaine extracellulaire de Tie-2 (Tek) dans des modèles de xénogreffes de tumeur du sein et de mélanome.

Les inhibiteurs de Tie2 peuvent être utilisés dans les situations où une néovascularisation se fait de façon inappropriée (c'est-à-dire dans la rétinopathie diabétique, l'inflammation chronique, le psoriasis, le sarcome de Kaposi, la néovascularisation chronique due à la dégénération maculaire, l'arthrite rhumatoïde, l'hémoangiome infantile et les cancers).

**FAK** est une tyrosine kinase cytoplasmique jouant un rôle important dans la transduction du signal transmis par les intégrines, famille de récepteurs hétérodimériques de l'adhésion cellulaire. FAK et les intégrines sont colocalisés dans des structures périmembranaires appelées plaques d'adhérence. Il a été montré dans de nombreux types cellulaires que l'activation de FAK ainsi que sa phosphorylation sur des résidus tyrosine et en particulier son autophosphorylation sur la tyrosine 397 étaient dépendantes de la liaison des intégrines à leurs ligands extracellulaires et donc induites lors de l'adhésion cellulaire [Kornberg L, et al. J. Biol. Chem. 267(33): 23439-442. (1992)]. L'autophosphorylation sur la tyrosine 397 de FAK représente un site de liaison pour une autre tyrosine kinase, Src, via son domaine SH2 [Schaller et al. Mol. Cell. Biol. 14 :1680-1688. 1994; Xing et al. Mol. Cell. Biol. 5 :413-421. 1994]. Src peut alors phosphoryler FAK sur la tyrosine 925, recrutant ainsi la protéine adaptatrice Grb2 et induisant dans certaines cellules l'activation de la voie ras et MAP Kinase impliquée dans le contrôle de la prolifération cellulaire [Schlaepfer et al. Nature; 372:786-791. 1994; Schlaepfer et al. Prog. Biophy. Mol. Biol. 71:435-478. 1999; Schlaepfer and Hunter, J. Biol. Chem. 272:13189-13195. 1997]. L'activation de FAK peut aussi induire la voie de signalisation jun NH2-terminal kinase (JNK) et résulter dans la progression des cellules vers la phase G1 du cycle cellulaire [Oktay et al., J. Cell. Biol.145 :1461-1469. 1999]. Phosphatidylinositol,3-OH kinase (PI3-kinase) se lie aussi à FAK sur la tyrosine 397 et cette interaction pourrait être nécessaire à l'activation de PI3-kinase [Chen and Guan, Proc. Nat. Acad. Sci. USA. 91: 10148-10152. 1994; Ling et al. J. Cell. Biochem. 73 :533-544. 1999]. Le complexe FAK/Src phosphoryle différents substrats comme la paxilline et p130CAS dans les fibroblastes [Vuori et al. Mol. Cell. Biol. 16: 2606-2613. 1996].

Les résultats de nombreuses études soutiennent l'hypothèse que les inhibiteurs de FAK pourraient être utiles dans le traitement du cancer. Des études ont suggéré que FAK puisse jouer un rôle important dans la prolifération et/ou la survie cellulaire *in vitro.* Par exemple, dans les cellules CHO, certains auteurs ont démontré que la surexpression de p125FAK conduit à une accélération de la transition G1 à S, suggérant que p125FAK favorise la prolifération cellulaire [Zhao J.-H et al. J. Cell Biol. 143:1997-2008. 1998]. D'autres auteurs ont montré que des cellules tumorales traitées avec des oligonucléotides anti-sens de FAK perdent leur adhésion et entrent en apoptose (Xu et al, Cell Growth Differ. 4:413-418. 1996). Il a également été démontré que FAK promeut la migration des cellules *in vitro.* Ainsi, des fibroblastes déficients pour l'expression de FAK (souris « knockout » pour FAK) présentent une morphologie arrondie, des déficiences de migration cellulaire en réponse à des signaux chimiotactiques et ces défauts sont supprimés par une réexpression de FAK [DJ. Sieg et al., J. Cell Science. 112:2677-91. 1999]. La surexpression du domaine C-terminal de FAK (FRNK) bloque l'étirement des cellules adhérentes et réduit la migration cellulaire *in vitro* [Richardson A. and Parsons J.T. Nature. 380:538-540. 1996]. La surexpression de FAK dans des cellules CHO, COS ou dans des cellules d'astrocytome humain favorise la migration des cellules. L'implication de FAK dans la promotion de la prolifération et de la migration des cellules dans de nombreux types cellulaires *in vitro,* suggère le rôle potentiel de FAK dans les processus néoplasiques. Une étude récente a effectivement démontré l'augmentation de la prolifération des cellules tumorales *in vivo* après induction de l'expression de FAK dans des cellules d'astrocytome humain [Cary L.A. et al. J. Cell Sci. 109:1787-94. 1996; Wang D et al. J. Cell Sci. 113:4221-4230. 2000]. De plus, des études immunohistochimiques de biopsies humaines ont démontré que FAK était surexprimé dans les cancers de la prostate, du sein, de la thyroïde, du colon, du mélanome, du cerveau et du poumon, le niveau d'expression de FAK étant directement corrélé aux tumeurs présentant le phénotype le plus agressif [Weiner TM, et al. Lancet. 342(8878):1024-1025. 1993 ; Owens et al. Cancer Research. 55:2752-2755. 1995; Maung K. et al. Oncogene. 18:6824-6828. 1999; Wang D et al. J. Cell Sci. 113:4221-4230. 2000].

KDR (Kinase insert Domain Receptor) aussi appelée VEGF-R2 (Vascular Endothelial Growth Factor Receptor 2), est exprimé uniquement dans les cellules endothéliales. Ce récepteur se fixe au facteur de croissance angiogénique VEGF, et sert ainsi de médiateur à un signal transductionnel via l'activation de son domaine kinase intracellulaire. L'inhibition directe de l'activité kinase de VEGF-R2 permet de réduire le phénomène d'angiogénèse en présence de VEGF exogène (Vascular Endothelial Growth Factor : facteur de croissance vasculaire endothélial) (Strawn et al., Cancer Research, 1996, vol. 56, p.3540-3545). Ce processus a été démontré notamment à l'aide de mutants VEGF-R2 (Millauer et al., Cancer Research, 1996, vol. 56, p.1615-1620). Le récepteur VEGF-R2 semble n'avoir aucune autre fonction chez l'adulte que celle liée à l'activité angiogénique du VEGF. Par conséquent, un inhibiteur sélectif de l'activité kinase du VEGF-R2 ne devrait démontrer que peu de toxicité.

En plus de ce rôle central dans le processus dynamique angiogénique, des résultats récents suggèrent que l'expression de VEGF contribue à la survie des cellules tumorales après des chimio- et radio-thérapies, soulignant la synergie potentielle d'inhibiteurs de KDR avec d'autres agents (Lee et al. Cancer Research, 2000, vol. 60, p.5565-5570).

Protocoles expérimentaux sur les tests biochimiques

### 1. Aurora 1 et Aurora 2

L'effet inhibiteur de composés vis-à-vis des kinases Aurora 1 et Aurora 2 est déterminé par un test enzymatique utilisant une détection de radioactivité.

L'activité kinase de Aurora 1 et Aurora 2 est évaluée par la phosphorylation du substrat Numa-histidine en présence d'ATP radiomarqué ([³³P]ATP) en utilisant des plaques 96 puits Flash plate où le nickel-chelate est fixé à la surface de la microplaque. La quantité de phosphate ³³P incorporé au substrat NuMA est proportionnelle à l'activité de l'enzyme Aurora 1 ou Aurora 2.

### Protéines :

Les protéines sont produites dans le laboratoire de production de protéines du groupe Sanofi-Aventis.
Aurora 1 : complexe recombinant Aurora-B/INCENP-C3 , purifié à environ 50% dont l'extrémité N-terminale de Aurora-B a été marquée à l'histidine.
Aurora 2 : protéine recombinante entière comprenant une queue histidine en N-terminal, a été exprimée dans E.coli et purifiée à plus de 82 %.
NuMA (protéine Nucléaire qui s'associe avec l'appareil mitotique) : fragment de 424 acides amines, exprimé dans E.coli dont l'extrémité N-terminale a été marquée à l'histidine et utilisé comme substrat pour les deux enzymes Aurora.

### Protocole :

Les microplaques utilisées sont des plaques Flash-Plate, 96 puits, nickel chélate (Perkin Elmer, modèle SMP107).

Les produits à évaluer sont incubés dans un volume réactionnel de 100 µl par puits, en présence de 10 nM de Aurora 1 ou Aurora 2, 500 nM de substrat NuMA dans un tampon composé de 50 mM de Tris/HCl (pH 7.5), 50 mM NaCl, 5 mM MgCl2 (Aurora-B) ou 10 mM MgCl2 (Aurora-A) et 1 mM de DTT, à 37°C.

Dans chaque puits, 80 µl du tampon d'incubation enzyme/substrat sont distribués puis 10 µl du produit à évaluer, en concentrations variables. La réaction est initiée par addition de 1µM d'ATP final contenant 0.2 µCi de [³³P]ATP (10 µl). Après 30 minutes d'incubation, la réaction est arrêtée par simple élimination du tampon réactionnel et chaque puits est lavé deux fois avec 300 µl du tampon Tris/HCl. La radioactivité est alors mesurée dans chaque puits à l'aide d'un appareil à scintillation, modèle Packard, Top count.

L'activité enzymatique contrôle d'Aurora est exprimée par le nombre de coup par minute obtenu en 30 minutes après déduction du bruit de fond (mélange réactionnel ne contenant pas l'enzyme). L'évaluation des divers produits testés est exprimée en pourcentage d'inhibition de l'activité Aurora par rapport au contrôle.

### 2. CDK2/cycline E :

### Purification du complexe CDK2/CyclineE-(His)₆ par IMAC (Immobilized Metal Affinity Chromatography) :

Deux baculovirus recombinants portant les séquences humaines codant respectivement pour CDK2 et la CyclineE (cette dernière comportant un tag hexa-histidine en C terminal) sont utilisés pour co-infecter des cellules d'insecte Sf21. Deux à trois jours après le début de la co-infection, les cellules sont récoltées par centrifugation, puis conservées à -40°C jusqu'à leur utilisation. Après décongélation et lyse mécanique des cellules, le complexe présent dans le surnageant de lyse est purifié par chromatographie d'affinité sur Nickel (IMAC), et conservé à -80°C.

### Essai Flashplate CDK2/CyclinE en format 96 puits.

Un format en plaques 96 puits coatés à la streptavidine est utilisé pour tester l'activité des composés sur l'activité kinase de CDK2/Cycline E.

Pour réaliser cet essai, le substrat peptidique biotynilé, fragment de la protéine pRb, (biotinyl-SACPLNLPLQNNHTAADMYLSPVRSPKKKGSTTR-OH) est solubilisé à la concentration de 1 mM dans du tampon kinase (HEPES/ NaOH 50 mM, NaCl 1 mM, MgCl₂ 5 mM, pH 7.5) afin de constituer une solution-stock conservée à -20°C sous forme d'aliquots de 110 µl. Le jour de l'expérience, un aliquot de cette solution est décongelé et dilué dans du tampon kinase contenant 1 mM de Dithiothréitol, ajouté au tampon extemporanément, afin d'obtenir une concentration de 14.3 µM. 70 µl de cette solution sont ajoutés dans chaque puits de la Flashplate afin d'obtenir une concentration finale en substrat de 10 µM lors de la réaction enzymatique conduite dans un volume final du milieu réactionnel de 100 µl (cf. ci-après).

Des dilutions intermédiaires d'inhibiteurs (produits de l'invention) à différentes concentrations sont préparées dans le DMSO à partir de solutions stock à 10 mM dans des tubes séparés. On réalise ainsi des dilutions à 1000 µM, 333.3 µM, 111.1 µM, 37.03 µM, 12.35 µM, 4.11 µM et 1.37 µM. Un µl de chacune de ces solutions (ou 1 µl de DMSO pour les contrôles) est transferré dans les puits de la plaque de test.

Dans chaque puits, sont ensuite ajoutés 19 µl d'une solution d'un mélange d'adénosinetriphosphate (ATP) et d'ATPy³³P dans le tampon kinase à la concentration de 5,26 µM d'ATP total et de 52,6 µCi/ml de ³³P. La réaction enzymatique est déclenchée par addition de 10 µl par puits d'une solution de CDK2/Cycline E à 200 nM dans le tampon kinase contenant 1 mM de dithiothréitol (ou 10µl de tampon kinase contenant 1 mM de dithiothréitol pour les blancs réactionnels).

Après addition de chacun des réactifs, le volume final de chaque puits est de 100µl, la concentration finale de substrat est de 10 µM, les concentrations finales en inhibiteurs sont de10 µM, 3,33 µM, 1,11 µM, 0,37 µM, 0,123 µM, 0,041 µM et 0,014 µM (selon la concentration de la dilution intermédiaire), la concentration finale en ATP est de 1 µM, la quantité finale de ³³P est de 1 µCi/puits, la concentration finale de complexe CDK2/Cycline E est de 20 nM.

Après l'addition de tous les réactifs, la plaque de test incubée à 30 °C sous agitation orbitale à 650 rpm.

Lorsque l'incubation est terminée, la plaque est lavée trois fois par 300 µl par puits de PBS (Phosphate Buffered Saline, pH=7,4 sans calcium ni magnésium, référence 10010-015, Gibco BRL). L'incorporation de ³³P au peptide est quantifiée par comptage par scintillation avec un appareil Packard Topcount.NXT. L'activité inhibitrice des produits de l'invention est évaluée par mesure de la concentration d'inhibiteur permettant une diminution de l'activité enzymatique de 50 % (CI50).

### 3. Tie2

La séquence codante de Tie2 humain correspondant aux acides aminés du domaine intracellulaire 776-1124 a été générée par PCR en utilisant le cDNA isolé de placenta humain comme modèle. Cette séquence a été introduite dans un vecteur d'expression *baculovirus* pFastBacGT sous forme de protéine de fusion GST.

L'effet inhibiteur des molécules est déterminé dans un test de phosphorylation de PLC par Tie2 en présence de GST-Tie2 purifiée à environ 80 % d'homogénéité. Le substrat est composé des fragments SH2-SH3 de la PLC exprimée sous forme de protéine de fusion GST.

L'activité kinase de Tie2 est mesurée dans un tampon MOPS 20mM pH 7.2, contenant 10 mM MgCl₂, 10 mM MnCl₂, 1 mM DTT, 10 mM de glycérophosphate. Dans une plaque 96 puits FlashPlate maintenue sur glace, on dépose un mélange réactionnel composé de 70 µl de tampon kinase contenant 100 ng d'enzyme GST-Tie2 par puits. Ensuite 10 µl de la molécule à tester diluée dans du DMSO à une concentration de 10 % maximum sont ajoutés. Pour une concentration donnée, chaque mesure est effectuée en quatre exemplaires. La réaction est initiée en ajoutant 20 µl de solution contenant 2 µg de GST-PLC, 2 µM d'ATP froid et 1 µCi d'³³P[ATP]. Après 1 heure d'incubation à 37°C, la réaction est stoppée en ajoutant 1 volume (100 µl) d'EDTA à 200 mM. Après élimination du tampon d'incubation, les puits sont lavés trois fois avec 300 µl de PBS. La radioactivité est mesurée sur un MicroBeta1450 Wallac.

L'inhibition de l'activité Tie2 est calculée et exprimée en pourcentage d'inhibition par rapport à l'activité contrôle déterminée en l'absence de composé.

### Activité des produits préparés:

L'activité des produits a été déterminée par mesure de l'inhibition de l'activité de Aurora 1, Aurora 2, CDK2 et Tie2. Les résultats sont donnés dans le tableau 1, ci-après (IC50, nM):

| **Exemple** | **Aurora 1** | **Aurora 2** | **CDK2** | **Tie2** |
|---|---|---|---|---|
| 1 | 8 | 8 | 177 | 117 |
| 2 | 9 | 9 | 97 | 92 |
| 3 | 31 | 45 | 471 | 192 |
| 4 | 6 | 28 | 508 | 115. |
| 5 | 33 | 28 | 664 | 75 |
| 6 | 14 | 20 | 579 | 230 |
| 7 | 16 | 17 | 428 | 186 |
| 8 | 29 | 8 | 134 | 252 |
| 9 | 131 | 157 | 65 | 820 |
| 10 | 102 | 46 | 92 | 367 |
| 11a | 178 | 31 | 156 | 520 |
| 11b | 216 | 73 | 77 | 704 |
| 12a | 128 | 31 | 126 | 564 |
| 12b | 151 | 68 | 40 | 218 |
| 13a | 141 | 23 | 151 | 679 |
| 13b | 182 | 85 | 49 | 324 |
| 14a | 158 | 19 | 166 | 737 |
| 14b | 148 | 44 | 46 | 425 |
| 15a | 114 | 12 | 210 | 623 |
| 15b | 329 | 37 | 82 | 393 |
| 16a | 992 | 301 | 942 | 3213 |
| 16b | 936 | 606 | 301 | 2007 |
| 17a | 59 | 7 | 175 | 482. |
| 17b | 335 | 35 | 93 | 230 |
| 18a | 122 | 14 | 181 | 624 |
| 18b | 287 | 50 | 96 | 388 |
| 19 | 304 | 26 | 239 | 888 |
| 20a | 190 | 70 | 347 | 842 |
| 20b | 155 | 75 | 109 | 369 |
| 21a | 55 | 14 | 122 | 430 |
| 21b | 160 | 34 | 82 | 301 |
| 22a | 147 | 81 | 717 | 2212 |
| 22b | 956 | 762 | 703 | 3190 |
| 23a | 199 | 65 | 143 | 431 |
| 23b | 259 | 101 | 138 | 543 |
| 24 | 288 | 49 | 1486 | 1027 |
| 25 | 249 | 25 | 317 | 328 |
| 26 | 42 | 23 | 75 | 175 |
| 27 | 51 | 31 | 669 | 294 |
| 28 | 6 | 2 | 132 | 106 |
| 29 | 28 | 11 | 585 | 163. |
| 30 | 12 | 6 | 221 | 128 |
| 31 | 96 | 26 | 275 | 3063 |
| 32 | 25 | 4 | 109 | 38 |
| 33 | 13 | 5 | 100 | 8 |
| 34 | 13 | 5 | 1581 | 38 |
| 35 | 4 | 4 | 121 | 29 |
| 36 | 9 | 5 | 1924 | 6 |
| 37 | 32 | 20 | 54 | 56 |
| 38 | 21 | 4 | 214 | 58 |
| 39 | 15 | 27 | 53 | 22 |
| 40 | 11 | 17 | 105 | 25 |
| 41 | 15 | 25 | 294 | 44 |
| 42 | 24 | 6 | 1122 | 61 |
| 43 | 5 | 1 | 32 | 8 |
| 44 | 21 | 6 | 201 | 107 |
| 45 | 33 | 21 | 146 | 29 |
| 46 | 12 | 6 | 100 | 275 |
| 47 | 8 | 5 | 289 | 75 |

## Revendications

1. Produit, **caractérisé en ce qu'**il répond à la formule générale (I) suivante: 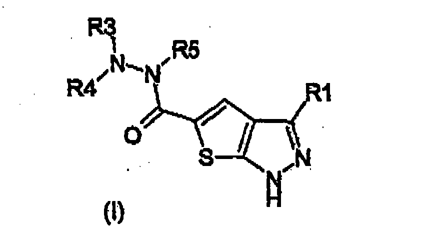 dans laquelle:
(i) R1 est indépendamment sélectionné dans le groupe constitué par - NHCO(R2), -NHCONH(R2), -NHCOO(R2), dans lequel R2 est indépendamment sélectionné dans le groupe constitué par -H, -(C₁-C₂₄)alkyle, -(C₃-C₉)cycloalkyle, -(C₃-C₉)cycloalkyléne, hétérocycloalkyle, hétérocycloalkylène, aryle, hétéroaryle, -(C₁-C₄)alkyl-aryle, -(C₁-C₆)alkyl-hétéroaryle, -aryl-(C₁-C₈)alkyle, hétéroaryl-(C₁-C₆)alkyle, éventuellement substitués ;
(ii) Chacun des R3, R4, et R5 est indépendamment sélectionné dans le groupe constitué par -H, -(C₁-C₈)alkyle, -(C₁-C₆)alkyl-aryle, -(C₁-C₆)akyl-hétéroaryle, -aryle, -hétéroaryle, éventuellement substitués,
ou bien [(R3 et R4) ou (R3 et R5)] sont liés entre eux pour former un hétérocycle mono ou bicyclique, saturé ou insaturé, comprenant de 2 à 10 chaînons carbonés et de 1 à 5 hétéroatomes choisis parmi N, O, et S, éventuellement substitué, ou pour former un groupement N=CH. aryle, le dit aryle étant éventuellement substitué.

2. Produit selon la revendication 1, **caractérisé en ce que** R5 est H..

3. Produit selon la revendication 1, **caractérisé en ce que** R4 est H.

4. Produit selon la revendication 1, **caractérisé en ce que** R4 est methyl ou ethyl.

5. Produit selon la revendication 1, **caractérisé en ce que** R3 et R4 forment avec l'azote auquel ils sont rattachés, un héterocycle de 5 à 6 chaînons renfermant en outre le cas échéant un atome de N, O, ou S, éventuellement substitués.

6. Produit selon la revendication 1, **caractérisé en ce que** R1 est NHCO(R2), R2 étant tel que défini à la revendication 1.

7. Produit selon la revendication 1, **caractérisé en ce que** R3 est aryle ou hétéroaryle, -(C1-C6)alkyl-aryle ou -(C1-C6)alkyl-hétéroaryle, éventuellement substitué.

8. Produit selon l'une quelconque des revendications 1 à 7, **caractérisé en ce qu'**il réponde à la formule (l') : 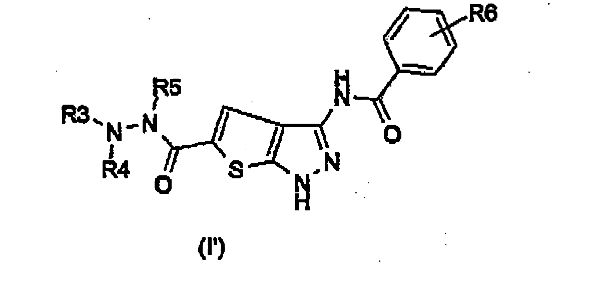 dans laquelle :
- Chacun des groupements R3, R4, et R5 sont tels que définis aux revendications 1 à 7,
- R6 est choisi parmi le groupe constitué par halogène. (C₁-C₃)alkyle-NR₇R₈, (C₁-C₆)alcoxy, (C₀-C₃)alkyle-hétérocycle, (C₀-C₃)alkyle-aryle, (C₀-C₃)alkyle-hétéroaryle, C₀-C₃)alkyle-cycloalkyle où les cycles sont éventuellement substitués par un ou plusieurs substituants (C₁-C₃)alkyle, halogène, alcoxy, et où R₇ et R₈ sont indépendamment sélectionnés dans le groupe constitué par -H, (C₀-C₃)alkyle, aryle, (C₁-C₃)alkyle-N-[(C₀-C₃)alkyle]₂.

9. Produit selon la revendication 8, **caractérisé en ce** en que R₆ est en position 3 ou 4.

10. Produit selon la revendication 9, **caractérisé en ce que** R6 est (C₀-C₃)alkyle-hétérocycle, le dit hétérocycle étant éventuellement substitué par un ou plusieurs substituants (C₁-C₃)alkyle, halogène, alcoxy.

11. Produit selon l'une des revendications 1 à 9, **caractérisé en ce que** aryle et hétéroaryle sont chacun indépendamment choisis parmi phényle, pyridyle, indolyle, benzimidazolyle, pyrazolyle, thiényle et pyrrolyle, éventuellement substitué.

12. Produit selon la revendication 1, **caractérisé en ce qu'**il est choisi parmi
5-(N'-phényl-hydrazinocarbonyl)-1H-thiéno[2,3-c]pyrazol-3-yl]-4-méthoxy-benzamide
5-(N'-méthyl-N'-phényl-hydrazinocarbonyl)-1H-thiéno[2,3-c]pyrazol-3-yl]-4-méthoxy-benzamide,
N-[5-(N'-Cyclohexyl-hydrazinocarbonyl)-1H-thiéno[2,3-c]pyrazol-3-yl]-4-méthoxy-benzamide,
N-[5-(N'-Benzyl-N'-phényl-hydrazinocarbonyl)-1H-thiéno[2,3-c]pyrazol-3-yl]-4-méthoxy-benzamide,
N-{5-[N'-(2-Ethyl-phényl)-hydrazinocarbonyl]-1H-thiéno[2,3-c]pyrazol-3-yl}-4-méthoxy-benzamide,
N-[5-[N'-(2-Fluoro-phényl)-hydrazinocarbonyl]-1H-thiéno[2,3-c]pyrazol-3-yl)-4-méthoxy-benzamide,
4-Méthoxy-N-[6-N'-o-tolyl-hydrazinocarbonyl)-1H-thiéno[2,3-c]pyrazol-3-yl]-benzamide.
N-[5-(N'-Méthyl-N'-phényl-hydrazinocarbonyl)-1H-thiéno[2,3-c]pyrazol-3-yl]-4-morpholin-4-ylméthyl-benzamide,
4-Bromo-N-[5-{N'-méthyl-N'-phényl-hydrazinocarbonyl)-1H-thiéno[2,3-c]pyrazol-3-yl]-benzamide,
N-[5-(N'-Méthyl-N'-phényl-hydrazinocarbonyl)-1H-thiéno[2,3-c]pyrazol-3-yl]-4-(4-méthyl-pipérazin-1-ylméthyl)-benzamide,
4-(3,5-Diméthyl-pipérazin-ylméthyl)-N-[5-(N'-méthyl-N'-phényl-hydrazinocarbonyl)-1H-thiéno[2,3-c]pyrazol-3-yl]-benzamide,
4-(3,5-Diméthyl-pipérazin-1-ylméthyl)-N-[5-(N'-(4-chloro-phényl)-N'-méthyl-hydrazinocarbonyl)-1H-thiéno[2,3-c]pyrazol-3-yl]-benzamide,
4-(4-Méthyl-perhydro-1,4-diazepin-1-ylméthyl)-N-[5-(N'-méthyl-N'-phényl-hydrazinocarbonyl)-1H-thiéno[2,3-c]pyrazol3-yl]-benzamide,
4-(4-Méthyl-perhydro-1,4-diazepin-1-ylméthyl)-N-[5-(N'-(4-chloro-phényl)-N'-méthyl-hydrazinocarbonyl)-1H-thiéno[2,3-c]pyrazol-3-yl]-benzamide,
N-5-(N'-[Méthyl-N'-phényl-hydrazinocarbonyl)-1H-thiéno[2,3-c]]pyrazol-3-yl]-4-pipérazin-1-ylméthyl-benzamide,
N-[5-[N'-(4-Chloro-phényl)-N'-méthyl-hydrazinocarbonyl]-1H-thiéno[2,3-c]pyrazol-3-yl]-4-pipérazin-1-ylméthyl-benzamide,
N-[5-(N'-Méthyl-N'-phényl-hydrazinocarbonyl)-1H-thiéno[2,3-c]pyrazol-3-yl]-4-(3-méthyl-piperazin-1-ylméthyl)-benzamide,
N-{5-[N'-(4-Chloro-phényl)-N'-méthyl-hydrazinocarbonyl]-1H-thiéno[2,3-c]pyrazol-3-yl}-4-(3-méthyl-piperazin-1-ylméthyl)-benzamide,
4-Diéthylaminométhyl-N-[5-(N'-méthyl-N'-phényl-hydrazinocarbonyl)-1H-thiéno[2,3-c]pyrazol-3-yl]benzamide: trifluoro-acetate,
N-[5-[N'-(4-Chlorophényl)-N'-méthyl-hydrazinocarbonyl]-1H-thiéno[2,3-c]pyrazol-3-yl}-4-diéthylaminométhyl-benzamide,
N-[5-(N'-Méthyl-N'-phényl-hydrazinocarbonyl)-1H-thiéno[2,3-c]pyrazol-3-yl]-3-(4-méthyl-pipérazin-1-ylméthyl)-benzamide.
N-[5-(N'-(4-Chloro-phényl)-N'-méthyl-hydrazinocarbonyl)-1H-thiéno[2,3-c]pyrazol-3-yl]-3-(4-méthyl-pipérazin-1-ylméthyl)-benzamide,
N-[5-(N'-Méthyl-N'-phényl-hydrazinocarbonyl)-1H-thiéno[2,3-c]pyrazol-3-yl]-4-pipéridin-1-ylméthyl-benzamide,
N-[5-(N'-(4-Chloro-phényl)-N'-méthyl-hydrazinocarbonyl)-1H-thiéno[2,3-c]pyrazol-3-yl]-4-pipéridin-1-ylméthyl-benzamide,
N-[5-(N'-Méthyl-N'-phényl-hydrazinocarbonyl)-1H-thiéno[2,3-c]pyrazol-3-yl]-4-pyrrolidin-1-ylméthyl-benzamide,
N-[5-(N'-(4-Chloro-phényl)-N'-méthyl-hydrazinocarbonyl)-1H-thiéno[2,3-c]pyrazol-3-yl]-4-pyrrolidin-1-ylméthyl-benzamide,
4-Azétidin-1-ylméthyl-N-[5-(N'-méthyl-N'-phényl-hydrazinocarbonyl)-1H-thiéno[2,3,c]pyrazol-3-yl]benzamide,
4-{[(2-Diméthylamino-éthyl)-méthyl-amino]-méthyl}-N-[5-(N'-méthyl-N'-phényl-hydrazinocarbonyl)-1H-thiéno[2,3-c]pyrazol-3-yl]-benzamide,
N-{5-[N'-(4-Chloro-phényl)-N'-méthyl-hydrazinocarbonyl]-1H-thiéno[2,3-c]pyrazol-3-yl]-4-{[(2-diméthylamino-éthyl)-méthyl-amino]-méthyl}-benzamide,
N-[5-(N'-Méthyl-N'-phényl-hydrazinocarbonyl)-1H-thiéno[2,3-c]pyrazol-3-yl]-4-perhydro-1,4-oxazepin-4-ylméthyl-benzamide,
N-{5-[N'-(4-Chloro-phényl)-N'-méthyl-hydrazinocarbonyl]-1H-thiéno[2,3-c]pyrazol-3-yl]-4-perhydro-1,4oxazepin-4-ylméthyl-benzamide,
N-[5-(N'-Méthyl-N'-phényl-hydrazinocarbonyl)-1H-thiéno[2,3-c]pyrazol-3-yl]-3-morpholin-4-ylméthyl-benzamide,
N-{5-[N'-(4-Chloro-phényl)-N'-méthyl-hydrazinocarbonyl]-1H-thiéno[2,3-c]pyrazol-3-yl}-3-morpholin-4-ylméthyl-benzamide,
4-[(2-Diéthylamino-éthylamino)-méthyl]-N-[5-(N'-méthyl-N'-phényl-hydrazinocarbonyl)-1H-thiéno[2,3-c]pyrazol-3-yl]-benzamide,
N-{5-[N'-(4-Chloro-phényl)-N'-méthyl-hydrazinocarbonyl]-1H-thiéno[2,3-c]pyrazol-3-yl}-4-[(2-diéthylamino-éthylamino)-méthyl]-benzamide,
4-[(Méthyl-phényl-amino)-méthyl]N-[5-(N'-méthyl-N'-phényl-hydrazinocarbonyl)-1H-thiéno[2,3-c]pyrazol-3-yl]-benzamide.
4-[(Diisopropylamino)-méthyl]-N-[5-(N'-méthyl-N'-phényl-hydrazinocarbonyl)-1H-thiéno[2,3-c]pyrazol-3-yl]-benzamide; trifluoroacétate,
N-[5-(N'-Benzyl-hydrazinocarbonyl)-1H-thiéno[2,3-c]pyrazol-3-yl]-4-méthoxy-benzamide,
4-Méthoxy-N-[5-(N'-pyridin-2-yl-hydrazinocarbonyl)-1H-thiéno[2,3-c]pyrazol-3-yl]-benzamide,
N-[5-[N'(4-Chloro-phényl)-méthyl-hydrazinocarbonyl]-thiéno[2,3-c]pyrazol-3-yl]-4-(4-méthyl-pipérazin-1-yl)-benzamide,
N-[5-(N'-Benzyl-N'-méthyl-hydrazinocarbonyl)-1H-thiéno[2,3-c]pyrazol-3-yl]-4-(4-méthyl-pipérazin-1-yl)-benzamide,
N-(Pipéridin-1-yl)-3-[4-(4-méthyl-pipérazin-1-yl)-benzoylamino]-1H-thiéno[2,3-c]pyrazole-5-carboxamide,
N-[5-(N'-Benzyl-hydrazinocarbonyl)-1H-thiéno[2,3-c]]pyrazol-3-yl]-4-(4-méthyl-pipérazin-1-yl)-benzamide,
N-[5-(N'-Ethyl-N'-phenyl-hydrazinocarbonyl)-1H-thiéno[2,3-c]pyrazol-3-yl]-4-(4-méthyl-pipérazin-1-yl)-benzamide,
N-[5-(N'-Benzyl-N'-phényl-hydrazinocarbonyl)-1H-thiéno[2,3-c]pyrazol-3-yl]-4-(4-méthyl-pipérazin-1-yl)-benzamide.
N-(5-(N'-Méthyl-N'-phényl-hydrazinocarbonyl)-1H-thiéno[2,3-c]pyrazol-3-yl]-4-(4-méthyl-pipérazin-1-yl)-benzamide,
N-{5-[N'-(4-Chloro-phényl)-N'-cyclobutylméthyl-hydrazinocarbonyl]-1H-thiéno[2,3-c]pyrazol-3-yl}-4-(4-méthyl-pipérazin-1-yl)-benzamide,
N-{5-[N'-Ethyl-N'-(4-fluoro-phényl)hydrazinocarbonyl]-1H-thiéno[2,3-c]pyrazol-3-yl]-4-(4-methyl-piperazin-1-yl)-benzamide,
N-{5-[N'-(4-Fluoro-phényl)-N'-(2-méthoxy-éthyl)-hydrazinocarbonyl]-1H-thiéno[2,3-c]pyrazol-3-yl)-4-(4-méthyl-pipérazin-1-yl)-benzamide,
N-[5-(N'-Ethyl-N'-o-totyl-hydrazinocarbonyl)-1H-thiéno[2,3-c]pyrazol-3-yl]-4-(4-méthyl-pipérazin-1-yl)-benzamide,
N-[5-N'-Ethyl-N'-m-tolyl-hydrazinocarbonyl)-1H-thiéno[2,3-c]pyrazol-3-yl]-4-(4-méthyl-pipérazin-1-yl)-benzamide,
N-[5-(N'-Ethyl-N'-m-tolyl-hydrazinocarbonyl)-1H-thiéno[2,3-c]pyrazol-3-yl]-4-(4-méthyl-pipérazin-1-yl)-benzamide,
N-{5-[N'-(4-Fluro-phényl)-N'-isobutyl-hydrazinocarbonyl]-1H-thiéno[2,3-c]pyrazol-3-yl}-4-(4-méthyl-pipérazin-1-yl)benzamide,
N-{5-[N'-(3-Bromo-phényl)-N'-éthyl-hydrazinocarbonyl]-1H-thiéno[2,3-c]pyrazol-3-yl]-4-(4-méthyl-pipérazin-1-yl)-benzamide,
6-(4-Méthyl-[1,4]diazepan-1-yl)-N-[5-(N'-méthyl-N'-phényl-hydrazinocarbonyl)-1H-thiéno[2,3-c]pyrazol-3-yl]-nicotinamide,
N-[5-(Benzylidène-hydrazinocarbonyl)-1H-thiéno[2,3-c]pyrazol-3-yl]-4-(4-méthyl-pipérazin-1-yl)-benzamide,
N-[5-(N'-Méthyl-N'-phényl-hydrazinocarbonyl)-1H-thiéno[2,3-c]pyrazol-3-yl]-4-(2-morpholin-4-yl-éthoxy)-benzamide,
4-(4-méthyl-[1,4]diazepan-1-yl)-N-[5-(N'-méthyl-N'-phényl-hydrazinocarbonyl)-1H-thiéno[2,3-c]pyrazol-3-yl]-benzamide,

13. Produit selon l'une quelconque des revendications précédentes, **caractérisé en ce qu'**il est sous forme :
1) non chirale, ou
2) racémique, ou
3) enrichie en un stéréo-isomère, ou
4) enrichie en un énantiomère ;
et **en ce qu'**il est éventuellement saliflé.

14. Procédé de préparation d'un produit de formule générale (1) suivante : 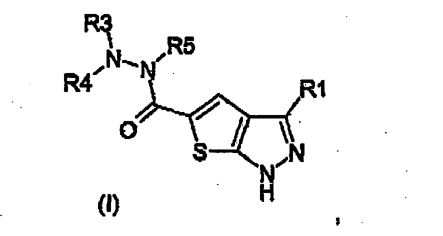 dans laquelle R1 est NHCO(R2), et dans lequel R3, R4 et R5 sont tels que définis précédemment, ledit produit de formule générale (I) étant obtenu par :
(i) couplage entre (4) un acide de formule générale (X) suivante : 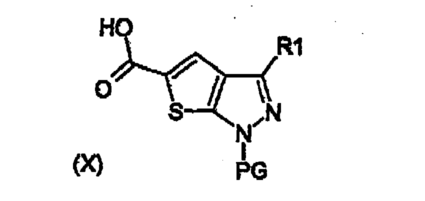 dans laquelle R1 est tel que défini précédemment, et dans lequel PG est un groupe protecteur de la fonction NH libre intracyclique du noyau thiéno[2,3-c]pyramie, et
(I-b) une hydrazine (R3)(R4)N-NH(R5) ) dans laquelle R3, R4 et R5 sont tels que définis précédemment, en présence d'un agent de couplage et en présence d'une base telle qu'une amine tertiaire ou un carbonate d'un métal alcalin ; puis
(ii) divague de PG.

15. Procédé de préparation d'un produit de formule générale (X) selon la revendication 14, **caractérisé en ce qu'**il est obtenu par saponification de la fonction ester du noyau thiophène d'un produit de formule générales (IX): 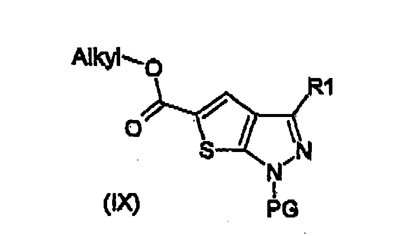 dans lequel R1 est tel que défini précédemment

16. Procédé de préparation d'un produit de formule générale (IX) selon la revendication 15, **caractérisé en ce qu'**il est obtenu par couplage entre :
(i) un produit de formule générale (IIa) suivante : 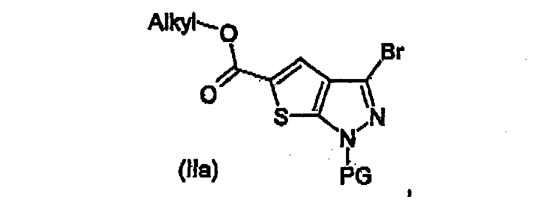 dans laquelle Alkyl est tel que défini précédemment, et dans laquelle PG est un groupe protecteur de la fonction NH libre intracyclique du noyau thiéno[2,3-c]pyrazole, et
(ii) un produit de formule générale (R2)CONH₂, en présence :
- d'un catalyseur tel que l'iodure de cuivre (I),
- d'une amine telle que le *trans*-1,2-diaminocyclohexane, le *trans*-1,2-bis(méthylamino)cyclohexane ou, de préférence le N,N'-diméthyl-1,2-diaminoéthane, et
- d'une base telle que le phosphate tripotassique ou le carbonate de césium.

17. Procédé de préparation d'un produit de formule générales (IIa) selon la revendication 16, **caractérisé en ce qu'**il est obtenu par réaction entre un mercaptoacétate d'alkyle Alkyl-OCO-CH₂-SH, en présence d'une base telle que le carbonate de sodium et un composé (IIIa) : 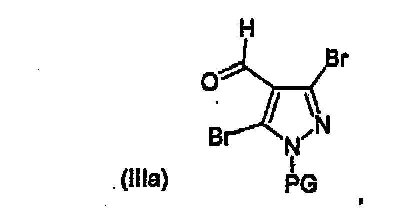 dans lequel PG est tel que défini précédemment :

18. Procédé de préparation d'un produit de formule générale (IIIa) selon la revendication 17, **caractérisé en ce qu'**il est obtenu par (I) formylation du 3,4,5-tribromo-pyrazole pour l'obtention de 3,5-dibromo4-foormyl-pyrazole (III), puis (II) protection de la fonction amine lntracyclique de (III) par l'introduction du groupe protecteur pG.

19. Procédé selon la revendication 18, **caractérisé en ce que** le groupe protecteur PG est introduit via :
(i) réaction avec de l'éthylvinyl éther en présence d'un acide tel que l'acide chlorhydrique, pour l'obtention d'un groupe PG = 1-éthoxy-éthyle, au sein d'un solvant lnerte tel que le toluène, ou ;
(ii) réaction avec du di-tert-butyldicarbonate en présence d'une base telle que la triéthylamine, la pyridine, ou la N,N-dimétylamino-pyridine, pour l'obtention d'un groupe PG = tert-butyloxycarbonyte, au sein d'un solvant Inerte tel que le dichlorométhane.

20. Composition pharmaceutique comprenant un produit selon l'une quelconque des revendications 1 à 13, en combinaison avec un excipient phamaceutiquement acceptable.

21. Utilisation d'un produit selon l'une quelconque des revendications 1 à 13, pour la fabrication d'un médicament utile pour traiter un état pathologique. en particulier le cancer.

22. Utilisation d'un produit selon l'une quelconque des revendications 1 à 13, pour la fabrication d'un medicament utile pour traiter un état pathologique, choisi parmi !e psorials, le glaucome, les leucémies, les maladies liées au système nerveux central, les lnflammations, et les maladies liées à une dérégulation des protéines JNK.

23. Produit, **caractérisé en ce qu'**il répond à la formule général (XVI) suivante : 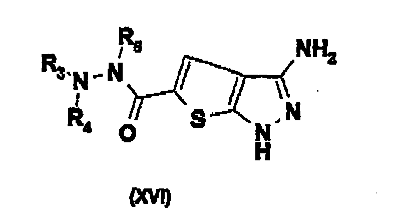 dans laquelle R3, R4 et R5 sont tels que définis l'une des revendications 1 à 7.

24. Produit, **caractérisé en ce qu'**il répond à la formule générale (XVII) suivante: 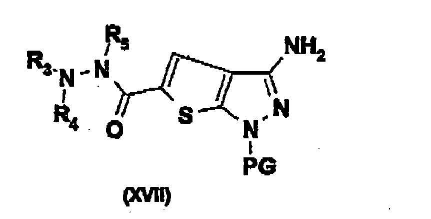 dans laquelle. R3, R4, R5 sont tels que définis l'une des revendications 1 à 7 et PG est un groupe protecteur.

25. Produit, **caractérisé en ce qu'**il répond à la formule générale (XVIII) suivante: 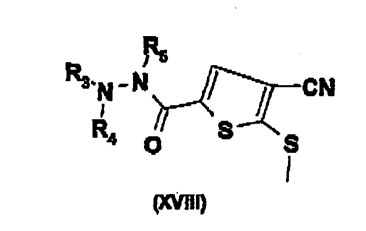 dans laquelle R3, R4, R5 sont tels que définis l'une des revendications 1 à 7.

26. Produit, **caractérisé en ce qu'**il répond à la formule générale (XIX) suivante: 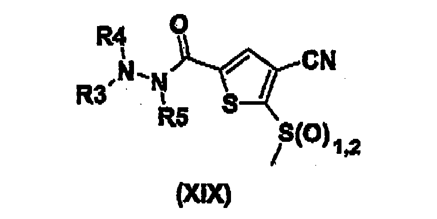 dans laquelle R3, R4, R5 sont tels que définis l'une des revendications 1 à 7.

27. Produit **caractérisé en ce qu'**il répond à la formule générale (XX) suivante : 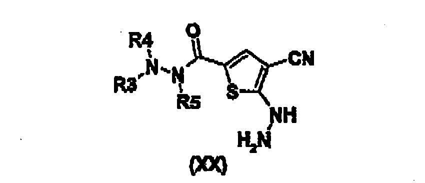 dans laquelle R3, R4. R5 sont tels que définis l'une des revendications 1 à 7.

28. Produit, **caractérisé e**n ce qu'il répond à la formule générale (IVb) suivante : 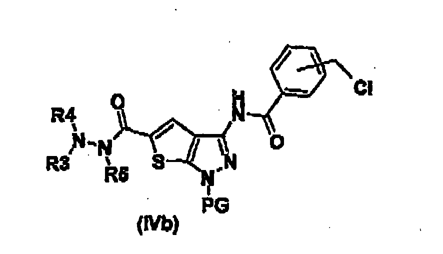 dans laquelle R3, R4, R5 sont tels que définis l'une des revendications 1 à 7 et PG est un groupe protecteur.

## Claims

1. Product, **characterized in that** it corresponds to the general formula (I) below: 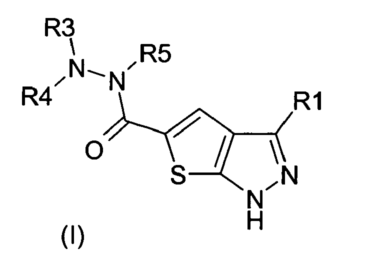 in which:
(i) R1 is independently selected from the group consisting of -NHCO(R2), -NHCONH(R2) and - NHCOO(R2), in which R2 is independently selected from the group consisting of -H, - (C₁-C₂₄) alkyl, - (C₃-C₉) cycloalkyl, - (C₃-C₉) cycloalkylene, heterocycloalkyl, heterocycloalkylene, aryl, heteroaryl, - (C₁-C₆) alkyl-aryl, - (C₁-C₆) alkyl-heteroaryl, -aryl-(C₁-C₆)alkyl and heteroaryl-(C₁-C₆)alkyl, optionally substituted;
(ii) each of the R3, R4 and R5 is independently selected from the group consisting of -H, -(C₁-C₆) alkyl, (C₁-C₆) alkyl-aryl, - (C₁-C₆) alkyl-heteroaryl, -aryl and -heteroaryl, optionally substituted, or alternatively [(R3 and R4) or (R3 and R5)] ] are linked together to form a saturated or unsaturated, 2- to 10-membered monocyclic or bicyclic carbon heterocycle containing from 1 to 5 heteroatoms chosen from N, O and S, optionally substituted, or to form a group N=CH-aryl, said aryl being optionally substituted.

2. Product according to Claim 1, **characterized in that** R5 is H.

3. Product according to Claim 1, **characterized in that** R4 is H.

4. Product according to Claim 1, **characterized in that** R4 is methyl or ethyl.

5. Product according to Claim 1, **characterized in that** R3 and R4 form, with the nitrogen atom to which they are attached, a 5- or 6-membered heterocycle also containing, where appropriate, an N, O or S atom, optionally substituted.

6. Product according to Claim 1, **characterized in that** R1 is NHCO(R2), R2 being as defined in Claim 1.

7. Product according to Claim 1, **characterized in that** R3 is aryl or heteroaryl, -(C₁-C₆)alkyl-aryl or - (C₁-C₆)alkyl-heteroaryl, optionally substituted.

8. Product according to any one of Claims 1 to 7, **characterized in that** it corresponds to formula (I'): 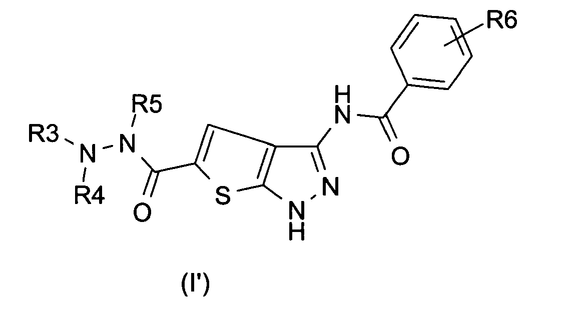 in which:
- each of the groups R3, R4 and R5 is as defined in Claims 1 to 7,
- R6 is chosen from the group consisting of halogen, (C₁-C₃) alkyl-NR7R8, (C₁-C₆) alkoxy, (C₀-C₃) alkyl-heterocycle, (C₀-C₃)alkyl-aryl, (C₀-C₃)alkyl-heteroaryl and (C₀-C₃)alkyl-cycloalkyl, in which the rings are optionally substituted with one or more substituents (C₁-C₃)alkyl, halogen or alkoxy, and in which R7 and R8 are independently selected from the group consisting of -H, (C₀-C₃) alkyl, aryl and (C₁-C₃)alkyl-N-[(C₀-C₃)alkyl]₂.

9. Product according to Claim 8, **characterized in that** R₆ is in position 3 or 4.

10. Product according to Claim 9, **characterized in that** R6 is (C₀-C₃)alkyl-heterocycle, said heterocycle being optionally substituted with one or more substituents (C₁-C₃)alkyl, halogen or alkoxy.

11. Product according to one of Claims 1 to 9,
**characterized in that** aryl and heteroaryl are each independently chosen from phenyl, pyridyl, indolyl, benzimidazolyl, pyrazolyl, thienyl and pyrrolyl, optionally substituted.

12. Product according to Claim 1, **characterized in that** it is chosen from:
5-(N'-Phenylhydrazinocarbonyl)-1H-thieno[2,3-c]pyrazol-3-yl]-4-methoxybenzamide
5-(N'-Methyl-N'-phenylhydrazinocarbonyl)-1H-thieno[2,3-c]pyrazol-3-yl]-4-methoxybenzamide,
N-[5-(N'-Cyclohexylhydrazinocarbonyl)-1H-thieno[2,3-c]-pyrazol-3-yl]-4-methoxybenzamide,
N-[5-(N'-Benzyl-N'-phenylhydrazinocarbonyl)-1H-thieno[2,3-c]pyrazol-3-yl]-4-methoxybenzamide,
N-{5-[N'-(2-Ethylphenyl)hydrazinocarbonyl]-1H-thieno[2,3-c]pyrazol-3-yl}-4-methoxybenzamide,
N-{5-[N'-(2-Fluorophenyl)hydrazinocarbonyl]-1H-thieno[2,3-c]pyrazol-3-yl}-4-methoxybenzamide,
4-Methoxy-N-[5-(N'-o-tolylhydrazinocarbonyl)-1H-thieno[2,3-c]pyrazol-3-yl]benzamide.
N-[5-(N'-Methyl-N'-phenylhydrazinocarbonyl)-1H-thieno[2,3-c]pyrazol-3-yl]-4-morpholin-4-ylmethylbenzamide,
4-Bromo-N-[5-(N'-methyl-N'-phenylhydrazinocarbonyl)-1H-thieno[2,3-c]pyrazol-3-yl]benzamide,
N-[5-(N'-Methyl-N'-phenylhydrazinocarbonyl)-1H-thieno[2,3-c]pyrazol-3-yl]-4-(4-methylpiperazin-1-ylmethyl)benzamide,
4-(3,5-Dimethylpiperazin-1-ylmethyl)-N-[5-(N'-methyl-N'-phenylhydrazinocarbonyl)-1H-thieno[2,3-c]pyrazol-3-yl]benzamide,
4-(3,5-Dimethylpiperazin-1-ylmethyl)-N-[5-(N'-(4-chlorophenyl)-N'-methylhydrazinocarbonyl)-1H-thieno[2,3-c]pyrazol-3-yl]benzamide,
4-(4-Methylperhydro-1,4-diazepin-1-ylmethyl)-N-[5-(N'-methyl-N'-phenylhydrazinocarbonyl)-1H-thieno[2,3-c]-pyrazol-3-yl]benzamide,
4-(4-Methylperhydro-1,4-diazepin-1-ylmethyl)-N-[5-(N'-(4-chlorophenyl)-N'-methylhydrazinocarbonyl)-1H-thieno[2,3-c]pyrazol-3-yl]benzamide,
N-[5-(N'-Methyl-N'-phenylhydrazinocarbonyl)-1H-thieno[2,3-c]pyrazol-3-yl]-4-piperazin-1-ylmethylbenzamide,
N-(5-[N'-(4-Chlorophenyl)-N'-methylhydrazinocarbonyl]-1H-thieno[2,3-c]pyrazol-3-yl}-4-piperazin-1-ylmethylbenzamide,
N-[5-(N'-Methyl-N'-phenylhydrazinocarbonyl)-1H-thieno[2,3-c]pyrazol-3-yl]-4-(3-methylpiperazin-1-ylmethyl)benzamide,
N-{5-[N'-(4-Chlorophenyl)-N'-methylhydrazinocarbonyl]-1H-thieno[2,3-c]pyrazol-3-yl}-4-(3-methylpiperazin-1-ylmethyl)benzamide,
4-Diethylaminomethyl-N-[5-(N'-methyl-N'-phenyl-hydrazinocarbonyl)-1H-thieno[2,3-c]pyrazol-3-yl]benzamide trifluoroacetate,
N-{5-[N'-(4-Chlorophenyl)-N'-methylhydrazinocarbonyl]-1H-thieno[2,3-c]pyrazol-3-yl}-4-diethylaminomethylbenzamide,
N-[5-(N'-Methyl-N'-phenylhydrazinocarbonyl)-1H-thieno[2,3-c]pyrazol-3-yl]-3-(4-methylpiperazin-l-ylmethyl)benzamide,
N-[5-(N'-(4-Chlorophenyl)-N'-methylhydrazinocarbonyl)-1H-thieno[2,3-c]pyrazol-3-yl]-3-(4-methylpiperazin-1-ylmethyl)benzamide,
N-[5-(N'-Methyl-N'-phenylhydrazinocarbonyl)-1H-thieno[2,3-c]pyrazol-3-yl]-4-piperid-l-ylmethylbenzamide,
N-[5-(N'-(4-Chlorophenyl)-N'-methylhydrazinocarbonyl)-1H-thieno[2,3-c]pyrazol-3-yl]-4-piperid-1-ylmethylbenzamide,
N-[5-(N'-Methyl-N'-phenylhydrazinocarbonyl)-1H-thieno[2,3-c]pyrazol-3-yl]-4-pyrrolidin-1-ylmethylbenzamide,
N-[5-(N'-(4-Chlorophenyl)-N'-methylhydrazinocarbonyl)-1H-thieno[2,3-c]pyrazol-3-yl]-4-pyrrolidin-1-ylmethylbenzamide,
4-Azetidin-1-ylmethyl-N-[5-(N'-methyl-N'-phenyl-hydrazinocarbonyl)-1H-thieno[2,3-c]pyrazol-3-yl]benzamide,
4-{[(2-Dimethylaminoethyl)methylamino]methyl}-N-[5-(N'-methyl-N'-phenylhydrazinocarbonyl)-1H-thieno[2,3-c]-pyrazol-3-yl]benzamide,
N-{5-[N'-(4-Chlorophenyl)-N'-methylhydrazinocarbonyl]-1H-thieno[2,3-c]pyrazol-3-yl}-4-{[(2-dimethylaminoethyl)methylamino]methyl}benzamide,
N-[5-(N'-Methyl-N'-phenylhydrazinocarbonyl)-1H-thieno[2,3-c]pyrazol-3-yl]-4-perhydro-1,4-oxazepin-4-ylmethylbenzamide,
N-{5-[N'-(4-Chlorophenyl)-N'-methylhydrazinocarbonyl]-1H-thieno[2,3-c]pyrazol-3-yl}-4-perhydro-1,4-oxazepin-4-ylmethylbenzamide,
N-[5-(N'-Methyl-N'-phenylhydrazinocarbonyl)-1H-thieno[2,3-c]pyrazol-3-yl]-3-morpholin-4-ylmethylbenzamide,
N-{5-[N'-(4-Chlorophenyl)-N'-methylhydrazinocarbonyl]-1H-thieno[2,3-c]pyrazol-3-yl}-3-morpholin-4-ylmethylbenzamide,
4-[(2-Diethylaminoethylamino)methyl]-N-[5-(N'-methyl-N'-phenylhydrazinocarbonyl)-1H-thieno[2,3-c]pyrazol-3-yl]benzamide,
N-{5-[N'-(4-Chlorophenyl)-N'-methylhydrazinocarbonyl]-1H-thieno[2,3-c]pyrazol-3-yl}-4-[(2-diethylaminoethylamino)methyl]benzamide,
4-[(Methylphenylamino)methyl]-N-[5-(N'-methyl-N'-phenylhydrazinocarbonyl)-1H-thieno[2,3-c]pyrazol-3-yl]benzamide,
4-[(Diisopropylamino)methyl]-N-[5-(N'-methyl-N'-phenyl-hydrazinocarbonyl)-1H-thieno[2,3-c]pyrazol-3-yl]benzamide trifluoroacetate,
N-[5-(N'-Benzylhydrazinocarbonyl)-1H-thieno[2,3-c]-pyrazol-3-yl]-4-methoxybenzamide,
4-Methoxy-N-[5-(N'-pyrid-2-ylhydrazinocarbonyl)-1H-thieno[2,3-c]pyrazol-3-yl]benzamide,
N-{5-[N'-(4-Chlorophenyl)-N'-methylhydrazinocarbonyl]-1H-thieno[2,3-c]pyrazol-3-yl}-4-(4-methylpiperazin-1-yl)benzamide,
N-[5-(N'-Benzyl-N'-methylhydrazinocarbonyl)-1H-thieno[2,3-c]pyrazol-3-yl]-4-(4-methylpiperazin-1-yl)benzamide,
N-(Piperid-1-yl)-3-[4-(4-methylpiperazin-1-yl)benzoylamino]-1H-thieno[2,3-c]pyrazole-5-carboxamide,
N-[5-(N'-Benzylhydrazinocarbonyl)-1H-thieno[2,3-c]-pyrazol-3-yl]-4-(4-methylpiperazin-1-yl)benzamide,
N-[5-(N'-Ethyl-N'-phenylhydrazinocarbonyl)-1H-thieno[2,3-c]pyrazol-3-yl]-4-(4-methylpiperazin-1-yl)benzamide,
N-[5-(N'-Benzyl-N'-phenylhydrazinocarbonyl)-1H-thieno[2,3-c]pyrazol-3-yl]-4-(4-methylpiperazin-1-yl)benzamide,
N-[5-(N'-Methyl-N'-phenylhydrazinocarbonyl)-1H-thieno[2,3-c]pyrazol-3-yl]-4-(4-methylpiperazin-1-yl)benzamide,
N-{5-[N'-(4-Chlorophenyl)-N'-cyclobutylmethylhydrazino-carbonyl]-1H-thieno[2,3-c]pyrazol-3-yl}-4-(4-methyl-piperazin-1-yl)benzamide,
N-{5-[N'-Ethyl-N'-(4-fluorophenyl)hydrazinocarbonyl]-1H-thieno[2,3-clpyrazol-3-yll-4-(4-methylpiperazin-1-yl)benzamide,
N-{5-[N'-(4-Fluorophenyl)-N'-(2-methoxyethyl)hydrazinocarbonyl]-1H-thieno[2,3-c]pyrazol-3-yl}-4-(4-methyl-piperazin-1-yl)benzamide,
N-[5-(N'-Ethyl-N'-o-tolylhydrazinocarbonyl)-1H-thieno[2,3-c]pyrazol-3-yl]-4-(4-methylpiperazin-1-yl)benzamide,
N-[5-(N'-Ethyl-N'-m-tolylhydrazinocarbonyl)-1H-thieno[2,3-c]pyrazol-3-yl]-4-(4-methylpiperazin-1-yl)benzamide,
N-[5-(N'-Ethyl-N'-m-tolylhydrazinocarbonyl)-1H-thieno[2,3-c]pyrazol-3-yl]-4-(4-methylpiperazin-1-yl)benzamide,
N-{5-[N'-(4-Fluorophenyl)-N'-isobutylhydrazino-carbonyl]-1H-thieno[2,3-c]pyrazol-3-yl}-4-(4-methyl-piperazin-1-yl)benzamide,
N-{5-[N'-(3-Bromophenyl)-N'-ethylhydrazinocarbonyl]-1H-thieno[2,3-c]pyrazol-3-yl}-4-(4-methylpiperazin-1-yl)benzamide,
6-(4-Methyl[1,4]diazepan-1-yl)-N-[5-(N'-methyl-N'-phenylhydrazinocarbonyl)-1H-thieno[2,3-c]pyrazol-3-yl]nicotinamide,
N-[5-(Benzylidenehydrazinocarbonyl)-1H-thieno[2,3-c]-pyrazol-3-yl]-4-(4-methylpiperazin-1-yl)benzamide,
N-[5-(N'-Methyl-N'-phenylhydrazinocarbonyl)-1H-thieno[2,3-c]pyrazol-3-yl]-4-(2-morpholin-4-ylethoxy)benzamide,
4-(4-Methyl[1,4]diazepan-1-yl)-N-[5-(N'-methyl-N'-phenylhydrazinocarbonyl)-1H-thieno[2,3-c]pyrazol-3-yl]benzamide.

13. Product according to any one of the preceding claims, **characterized in that** it is:
1) in achiral form, or
2) in racemic form, or
3) enriched in one stereoisomer, or
4) enriched in one enantiomer;
and **in that** it is optionally salified.

14. Process for preparing a product of general formula
(I) below: 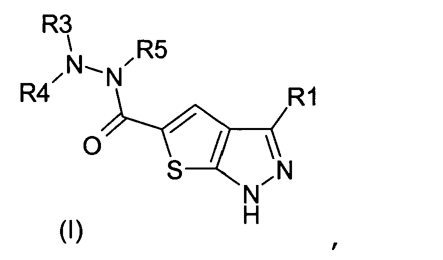
in which R1 is NHCO(R2), and in which R3, R4 and R5 are as defined above, said product of general formula (I) being obtained by:
(i) coupling between (i-a) an acid of general formula (X) below: 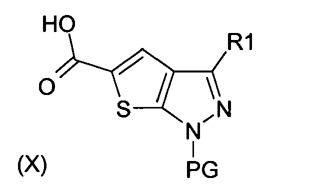 in which R1 is as defined above, and in which PG is a protecting group for the free endocyclic NH function of the thieno[2,3-c]pyrazole nucleus, and
(i-b) a hydrazine (R3)(R4)N-NH(R5) in which R3, R4 and R5 are as defined above, in the presence of a coupling agent and in the presence of a base such as a tertiary amine or an alkali metal carbonate; followed by
(ii) cleavage of PG.

15. Process for preparing a product of general formula (X) according to Claim 14, **characterized in that** it is obtained by saponification of the ester function of the thiophene nucleus of a product of general formula (IX): 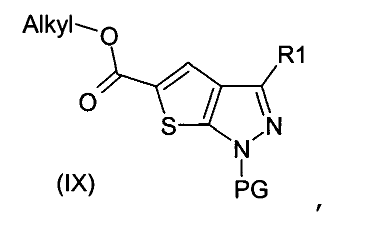 in which R1 is as defined above.

16. Process for preparing a product of general formula (IX) according to Claim 15, **characterized in that** it is obtained by coupling between:
(i) a product of general formula (IIa) below: 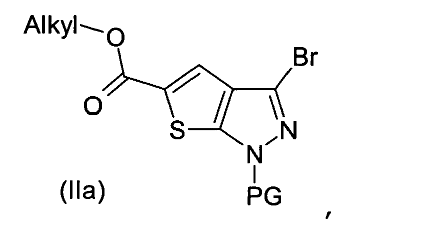 in which Alkyl is as defined above, and in which PG is a protecting group for the free endocyclic NH function of the thieno[2,3-c]pyrazole nucleus, and
(ii) a product of general formula (R2)CONH₂, in the presence of:
- a catalyst such as copper (I) iodide,
- an amine such as trans-1,2-diaminocyclohexane, *trans*-1,2-bis(methylamino)cyclohexane or, preferably, N,N'-dimethyl-1,2-diaminoethane, and
- a base such as tripotassium phosphate or cesium carbonate.

17. Process for preparing a product of general formula (IIa) according to Claim 16, **characterized in that** it is obtained by reaction between an alkyl mercaptoacetate Alkyl-OCO-CH₂-SH, in the presence of a base such as sodium carbonate and a compound (IIIa): 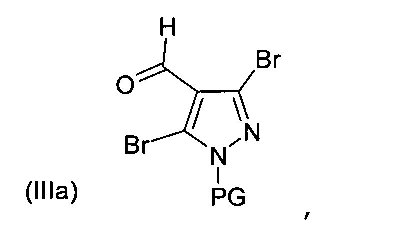 in which PG is as defined above.

18. Process for preparing a product of general formula (IIIa) according to Claim 17, **characterized in that** it is obtained by (i) formylation of 3,4,5-tribromo-pyrazole to obtain 3,5-dibromo-4-formylpyrazole (III), followed by (ii) protection of the endocyclic amine function of (III) via introduction of the protecting group PG.

19. Process according to Claim 18, **characterized in that** the protecting group PG is introduced via:
(i) reaction with ethyl vinyl ether in the presence of an acid such as hydrochloric acid, to obtain a group PG = 1-ethoxyethyl, in an inert solvent such as toluene, or;
(ii) reaction with di-tert-butyl dicarbonate in the presence of a base such as triethylamine, pyridine or N,N-dimethylaminopyridine to obtain a group PG = tert-butyloxycarbonyl, in an inert solvent such as dichloromethane.

20. Pharmaceutical composition comprising a product according to any one of Claims 1 to 13, in combination with a pharmaceutically acceptable excipient.

21. Use of a product according to any one of Claims 1 to 13, for the manufacture of a medicament that is useful for treating a pathological condition, in particular cancer.

22. Use of a product according to any one of Claims 1 to 13, for the manufacture of a medicament that is useful for treating a pathological condition chosen from psoriasis, glaucoma, leukemias, diseases associated with the central nervous system, inflammations, and diseases associated with deregulation of the JNK proteins.

23. Product, **characterized in that** it corresponds to the general formula (XVI) below: 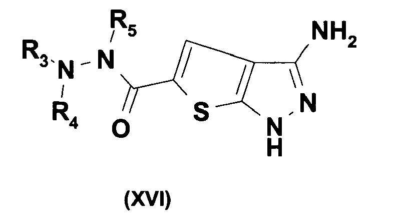 in which R3, R4 and R5 are as defined in one of Claims 1 to 7.

24. Product, **characterized in that** it corresponds to the general formula (XVII) below: 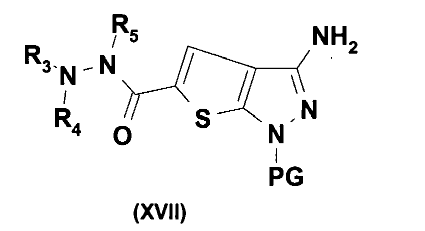 in which R3, R4 and R5 are as defined in one of Claims 1 to 7 and PG is a protecting group.

25. Product, **characterized in that** it corresponds to the general formula (XVIII) below: 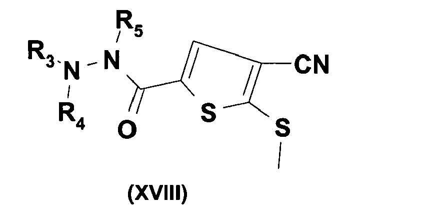 in which R3, R4 and R5 are as defined in one of Claims 1 to 7.

26. Product, **characterized in that** it corresponds to the general formula (XIX) below: 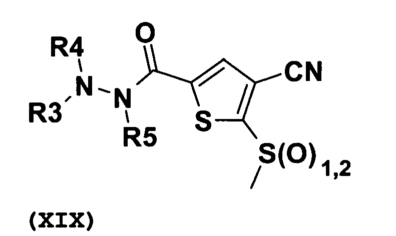 in which R3, R4 and R5 are as defined in one of Claims 1 to 7.

27. Product, **characterized in that** it corresponds to the general formula (XX) below: 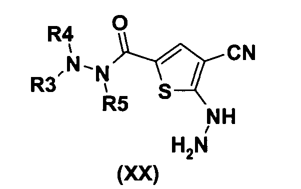 in which R3, R4 and R5 are as defined in one of Claims 1 to 7.

28. Product, **characterized in that** it corresponds to the general formula (IVb) below: 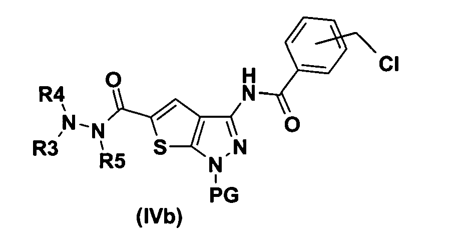 in which R3, R4 and R5 are as defined in one of Claims 1 to 7 and PG is a protecting group.

## Patentansprüche

1. Produkt, **dadurch gekennzeichnet, daß** es der folgenden allgemeinen Formel (I) entspricht: 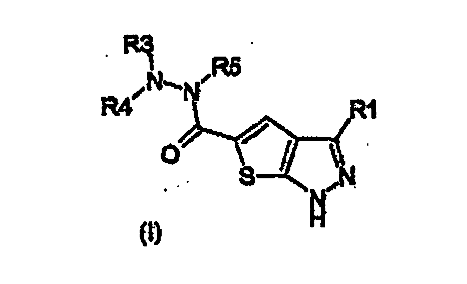 worin:
(i) R1 unabhängig aus der Gruppe bestehend aus -NHCO(R2), -NHCONH(R2) und -NHCOO(R2), worin R2 unabhängig aus der Gruppe bestehend aus -H, -(C₁-C₂₄)-Alkyl, -(C₃-C₉)-Cycloalkyl, -(C₃-C₉)-Cycloalkylen, Heterocycloalkyl, Heterocycloalkylen, Aryl, Heteroaryl, -(C₁-C₆)-Alkyl-aryl, -(C₁-C₆)-Alkyl-heteroaryl, -Aryl-(C₁-C₆) -alkyl und Heteroaryl- (C₁-C₆) -alkyl, gegebenenfalls substituiert, ausgewählt ist, ausgewählt ist;
(ii) jede der Gruppen R3, R4 und R5 unabhängig aus der Gruppe bestehend aus -H, -(C₁-C₆)-Alkyl, - (C₁-C₆) -Alkyl-aryl, - (C₁-C₆) -Alkyl-heteroaryl, -Aryl und -Heteroaryl, gegebenenfalls substituiert, ausgewählt ist oder auch [(R3 und R4) oder (R3 und R5)] unter Bildung eines gegebenenfalls substituierten gesättigten oder ungesättigten monocyclischen oder bicyclischen Heterocyclus mit 2 bis 10 Kohlenstoffgliedern und 1 bis 5 unter N, O und S ausgewählten Heteroatomen, oder unter Bildung einer N=CH-Aryl-Gruppe, wobei das Aryl gegebenenfalls substituiert ist, miteinander verbunden sind.

2. Produkt nach Anspruch 1, **dadurch gekennzeichnet, daß** R5 für H steht.

3. Produkt nach Anspruch 1, **dadurch gekennzeichnet, daß** R4 für H steht.

4. Produkt nach Anspruch 1, **dadurch gekennzeichnet, daß** R4 für Methyl oder Ethyl steht.

5. Produkt nach Anspruch 1, **dadurch gekennzeichnet, daß** R3 und R4 mit dem Stickstoff, an den sie gebunden sind, einen gegebenenfalls substituierten 5- bis 6-gliedrigen Heterocyclus, der gegebenenfalls außerdem ein N-, O- oder S-Atom enthält, bilden.

6. Produkt nach Anspruch 1, **dadurch gekennzeichnet, daß** R1 für NHCO(R2) steht, wobei R2 die in Anspruch 1 angegebene Bedeutung besitzt.

7. Produkt nach Anspruch 1, **dadurch gekennzeichnet, daß** R3 für Aryl oder Heteroaryl, , -(C₁-C₆)-Alkyl-aryl oder -(C₁-C₆)Alkyl-heteroaryl, gegebenenfalls substituiert, steht.

8. Produkt nach einem der Ansprüche 1 bis 7, **dadurch gekennzeichnet, daß** es der Formel (I') entspricht: 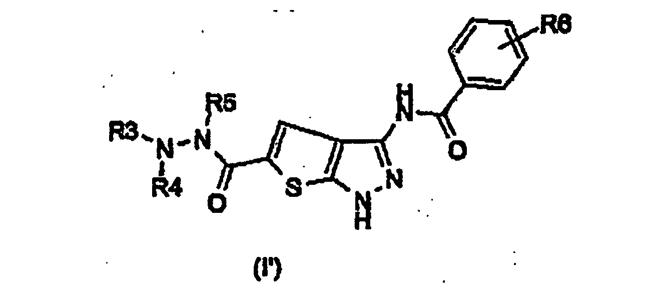 worin:
- jede der Gruppen R3, R4 und R5 die in den Ansprüchen 1 bis 7 definierte Bedeutung besitzt,
- R6 aus der Gruppe bestehend aus Halogen, (C₁-C₃)-Alkyl-NR7R8, (C₁-C₆) -Alkoxy, (C₀-C₃) -Alkyl-heterocyclus, (C₀-C₃) -Alkyl-aryl, (C₀-C₃)-Alkyl-heteroaryl und (C₀-C₃)-Alkyl-cycloalkyl, worin die Ringe gegebenenfalls durch einen oder mehrere (C₁-C₃)-Alkyl, Halogen- oder Alkoxysubstituenten substituiert sind und R7 und R8 unabhängig aus der Gruppe bestehend aus -H, (C₀-C₃)-Alkyl, Aryl und (C₁-C₃)-Alkyl-N-[(C₀-_{C}3)-alkyl]₂ ausgewählt sind, ausgewählt ist.

9. Produkt nach Anspruch 8, **dadurch gekennzeichnet, daß** R₆ in 3- oder 4-Position steht.

10. Produkt nach Anspruch 9, **dadurch gekennzeichnet, daß** R6 für (C₀-C₃)-Alkyl-heterocyclus steht, wobei der Heterocyclus gegebenenfalls durch einen oder mehrere (C₁-C₃) -Alkyl, Halogen- oder Alkoxysubstituenten substituiert ist.

11. Produkt nach einem der Ansprüche 1 bis 9, **dadurch gekennzeichnet, daß** Aryl und Heteroaryl jeweils unabhängig unter Phenyl, Pyridyl, Indolyl, Benzimidazolyl, Pyrazolyl, Thienyl und Pyrrolyl, gegebenenfalls substituiert, ausgewählt sind.

12. Produkt nach Anspruch 1, **dadurch gekennzeichnet, daß** es ausgewählt ist unter:
5-(N'-Phenylhydrazinocarbonyl)-1H-thieno[2,3-c]-pyrazol-3-yl]-4-methoxybenzamid
5-(N'-Methyl-N'-phenylhydrazinocarbonyl)-1H-thieno[2,3-c]pyrazol-3-yl]-4-methoxybenzamid,
N-[5-(N'-Cyclohexylhydrazinocarbonyl)-1H-thieno[2,3-c]pyrazol-3-yl]-4-methoxybenzamid,
N-[5-(N'-Benzyl-N'-phenylhydrazinocarbonyl)-1H-thieno[2,3-c]pyrazol-3-yl]-4-methoxybenzamid,
N-{5-[N'-(2-Ethylphenyl)hydrazinocarbonyl]-1H-thieno[2,3-c]pyrazol-3-yl}-4-methoxybenzamid,
N-{5-[N'-(2-Fluorphenyl)hydrazinocarbonyl]-1H-thieno[2,3-c]pyrazol-3-yl}-4-methoxybenzamid,
4-Methoxy-N-[5-(N'-o-tolylhydrazinocarbonyl)-1H-thieno[2,3-c]pyrazol-3-yl]benzamid.
N-[5-(N'-Methyl-N'-phenylhydrazinocarbonyl)-1H-thieno[2,3-c]pyrazol-3-yl]-4-morpholin-4-ylmethylbenzamid,
4-Brom-N-[5-(N'-methyl-N'-phenylhydrazino-carbonyl)-1H-thieno[2,3-c]pyrazol-3-yl]benzamid,
N-[5-(N'-Methyl-N'-phenylhydrazinocarbonyl)-1H-thieno[2,3-c]pyrazol-3-yl]-4-(4-methylpiperazin-1-ylmethyl)benzamid,
4-(3,5-Dimethylpiperazin-1-ylmethyl)-N-[5-(N'-methyl-N'-phenylhydrazinocarbonyl)-1H-thieno[2,3-c]pyrazol-3-yl]benzamid,
4-(3,5-Dimethylpiperazin-1-ylmethyl)-N-[5-(N'-(4-chlorphenyl)-N'-methylhydrazinocarbonyl)-1H-thieno[2,3-c]pyrazol-3-yl]benzamid,
4-(4-Methylperhydro-1,4-diazepin-1-ylmethyl)-N-[5-(N'-methyl-N'-phenylhydrazinocarbonyl)-1H-thieno[2,3-c]pyrazol-3-yl]benzamid,
4-(4-Methylperhydro-1,4-diazepin-1-ylmethyl)-N-[5-(N'-(4-chlorphenyl)-N'-methylhydrazinocarbonyl)-1H-thieno[2,3-c]pyrazol-3-yl]benzamid,
N-[5-(N'-Methyl-N'-phenylhydrazinocarbonyl)-1H-thieno[2,3-c]pyrazol-3-yl]-4-piperazin-1-ylmethylbenzamid,
N-{5-[N'-(4-Chlorphenyl)-N'-methylhydrazino-carbonyl]-1H-thieno[2,3-c]pyrazol-3-yl}-4-piperazin-1-ylmethylbenzamid,
N-[5-(N'-Methyl-N'-phenylhydrazinocarbonyl)-1H-thieno[2,3-c]pyrazol-3-yl]-4-(3-methylpiperazin-1-ylmethyl)benzamid,
N-{5-[N'-(4-Chlorphenyl)-N'-methylhydrazino-carbonyl]-1H-thieno[2,3-c]pyrazol-3-yl}-4-(3-methylpiperazin-1-ylmethyl)benzamid,
4-Diethylaminomethyl-N-[5-(N'-methyl-N'-phenyl-hydrazinocarbonyl)-1H-thieno[2,3-c]pyrazol-3-yl]benzamid trifluoracetate,
N-{5-[N'-(4-Chlorphenyl)-N'-methylhydrazino-carbonyl]-1H-thieno[2,3-c]pyrazol-3-yl}-4-diethylaminomethylbenzamid,
N-[5-(N'-Methyl-N'-phenylhydrazinocarbonyl)-1H-thieno[2,3-c]pyrazol-3-yl]-3-(4-methylpiperazin-1-ylmethyl)benzamid,
N-[5-(N'-(4-Chlorphenyl)-N'-methylhydrazino-carbonyl)-1H-thieno[2,3-c]pyrazol-3-yl]-3-(4-methylpiperazin-1-ylmethyl)benzamid,
N-[5-(N'-Methyl-N'-phenylhydrazinocarbonyl)-1H-thieno[2,3-c]pyrazol-3-yl]-4-piperidin-1-ylmethylbenzamid,
N-[5-(N'-(4-Chlorphenyl)-N'-methylhydrazino-carbonyl)-1H-thieno[2,3-c]pyrazol-3-yl]-4-piperidin-1-ylmethylbenzamid,
N-[5-(N'-Methyl-N'-phenylhydrazinocarbonyl)-1H-thieno[2,3-c]pyrazol-3-yl]-4-pyrrolidin-1-ylmethylbenzamid,
N-[5-(N'-(4-Chlorphenyl)-N'-methylhydrazino-carbonyl)-1H-thieno[2,3-c]pyrazol-3-yl]-4-pyrrolidin-1-ylmethylbenzamid,
4-Azetidin-1-ylmethyl-N-[5-(N'-methyl-N'-phenyl-hydrazinocarbonyl)-1H-thieno[2,3-c]pyrazol-3-yl]-benzamid,
4-{[(2-Dimethylaminoethyl)methylamino]methyl}-N-[5-(N'-methyl-N'-phenylhydrazinocarbonyl)-1H-thieno[2,3-c]pyrazol-3-yl]benzamid,
N-{5-[N'-(4-Chlorphenyl)-N'-methylhydrazino-carbonyl]-1H-thieno[2,3-c]pyrazol-3-yl}-4-{[(2-dimethylaminoethyl)methylamino]methyl}benzamid,
N-[5-(N'-Methyl-N'-phenylhydrazinocarbonyl)-1H-thieno[2,3-c]pyrazol-3-yl]-4-perhydro-1,4-oxazepin-4-ylmethylbenzamid,
N-{5-[N'-(4-Chlorphenyl)-N'-methylhydrazino-carbonyl]-1H-thieno[2,3-c]pyrazol-3-yl}-4-perhydro-1,4-oxazepin-4-ylmethylbenzamid,
N-[5-(N'-Methyl-N'-phenylhydrazinocarbonyl)-1H-thieno[2,3-c]pyrazol-3-yl]-3-morpholin-4-ylmethylbenzamid,
N-{5-[N'-(4-Chlorphenyl)-N'-methylhydrazino-carbonyl]-1H-thieno[2,3-c]pyrazol-3-yl}-3-morpholin-4-ylmethylbenzamid,
4-[(2-Diethylaminoethylamino)methyl]-N-[5-(N'-methyl-N'-phenylhydrazinocarbonyl)-1H-thieno[2,3-c]pyrazol-3-yl]benzamid,
N-{5-[N'-(4-Chlorphenyl)-N'-methylhydrazino-carbonyl]-1H-thieno[2,3-c]pyrazol-3-yl}-4-[(2-diethylaminoethylamino)methyl]benzamid,
4-[(Methylphenylamino)methyl]-N-[5-(N'-methyl-N'-phenylhydrazinocarbonyl)-1H-thieno[2,3-c]pyrazol-3-yl]benzamid,
4-[(Diisopropylamino)methyl]-N-[5-(N'-methyl-N'-phenylhydrazinocarbonyl)-1H-thieno[2,3-c]pyrazol-3-yl]benzamid trifluoracetate,
N-[5-(N'-Benzylhydrazinocarbonyl)-1H-thieno[2,3-c]pyrazol-3-yl]-4-methoxybenzamid,
4-Methoxy-N-[5-(N'-pyridin-2-ylhydrazinocarbonyl)-1H-thieno[2,3-c]pyrazol-3-yl]benzamid,
N-{5-[N'-(4-Chlorphenyl)-N'-methylhydrazino-carbonyl]-1H-thieno[2,3-c]pyrazol-3-yl}-4-(4-methylpiperazin-1-yl)benzamid,
N-[5-(N'-Benzyl-N'-methylhydrazinocarbonyl)-1H-thieno[2,3-c]pyrazol-3-yl]-4-(4-methylpiperazin-1-yl)benzamid,
N-(Piperidin-1-yl)-3-[4-(4-methylpiperazin-1-yl)-benzoylamino]-1H-thieno[2,3-c]pyrazole-5-carboxamid,
N-[5-(N'-Benzylhydrazinocarbonyl)-1H-thieno[2,3-c]pyrazol-3-yl]-4-(4-methylpiperazin-1-yl)benzamid,
N-[5-(N'-Ethyl-N'-phenylhydrazinocarbonyl)-1H-thieno[2,3-c]pyrazol-3-yl]-4-(4-methylpiperazin-1-yl)benzamid,
N-[5-(N'-Benzyl-N'-phenylhydrazinocarbonyl)-1H-thieno[2,3-c]pyrazol-3-yl]-4-(4-methylpiperazin-1-yl)benzamid,
N-[5-(N'-Methyl-N'-phenylhydrazinocarbonyl)-1H-thieno[2,3-c]pyrazol-3-yl]-4-(4-methylpiperazin-1-yl)benzamid,
N-{5-[N'-(4-Chlorphenyl)-N'-cyclobutylmethyl-hydrazinocarbonyl]-1H-thieno[2,3-c]pyrazol-3-yl}-4-(4-methylpiperazin-1-yl)benzamid,
N-{5-[N'-Ethyl-N'-(4-fluorphenyl)hydrazinocarbonyl]-1H-thieno[2,3-c]pyrazol-3-yl}-4-(4-methylpiperazin-1-yl)benzamid,
N-{5-[N'-(4-Fluorphenyl)-N'-(2-methoxyethyl)-hydrazinocarbonyl]-1H-thieno[2,3-c]pyrazol-3-yl}-4-(4-methylpiperazin-1-yl)benzamid,
N-[5-(N'-Ethyl-N'-o-tolylhydrazinocarbonyl)-1H-thieno[2,3-c]pyrazol-3-yl]-4-(4-methylpiperazin-1-yl)benzamid,
N-[5-(N'-Ethyl-N'-m-tolylhydrazinocarbonyl)-1H-thieno[2,3-c]pyrazol-3-yl]-4-(4-methylpiperazin-1-yl)benzamid,
N-[5-(N'-Ethyl-N'-m-tolylhydrazinocarbonyl)-1H-thieno[2,3-c]pyrazol-3-yl]-4-(4-methylpiperazin-1-yl)benzamid,
N-{5-[N'-(4-Fluorphenyl)-N'-isobutylhydrazino-carbonyl]-1H-thieno[2,3-c]pyrazol-3-yl}-4-(4-methylpiperazin-1-yl)benzamid,
N-{5-[N'-(3-Bromphenyl)-N'-ethylhydrazino-carbonyl]-1H-thieno[2,3-c]pyrazol-3-yl}-4-(4-methylpiperazin-1-yl)benzamid,
6-(4-Methyl[1,4]diazepan-1-yl)-N-[5-(N'-methyl-N'-phenylhydrazinocarbonyl)-1H-thieno[2,3-c]pyrazol-3-yl]nicotinamid,
N-[5-(Benzylidenhydrazinocarbonyl)-1H-thieno[2,3-c]pyrazol-3-yl]-4-(4-methylpiperazin-1-yl)benzamid,
N-[5-(N'-Methyl-N'-phenylhydrazinocarbonyl)-1H-thieno[2,3-c]pyrazol-3-yl]-4-(2-morpholin-4-yl-ethoxy)benzamid,
4-(4-Methyl[1,4]diazepan-1-yl)-N-[5-(N'-methyl-N'-phenylhydrazinocarbonyl)-1H-thieno[2,3-c]pyrazol-3-yl]benzamid.

13. Produkt nach einem der vorhergehenden Ansprüche, **dadurch gekennzeichnet, daß** es in
1) achiraler Form oder
2) racemischer Form oder
3) in mit einem Stereoisomer angereicherter Form oder
4) in mit einem Enantiomer angereicherter Form vorliegt und gegebenenfalls versalzt ist.

14. Verfahren zur Herstellung eines Produkts der folgenden allgemeinen Formel (I): 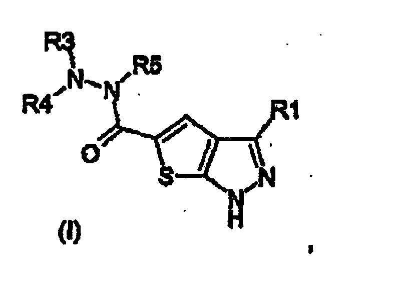 worin R1 für NHCO(R2) steht und R3, R4 und R5 die oben angegebene Bedeutung besitzen, wobei das Produkt der allgemeinen Formel (I) erhalten wird durch:
(i) Kupplung zwischen (i-a) einer Säure der folgenden allgemeinen Formel (X): 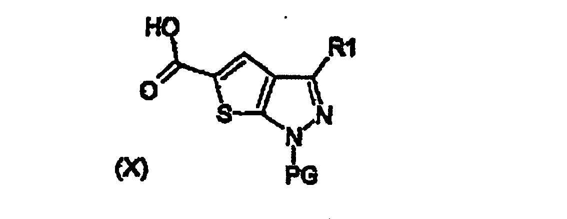 worin R1 die oben angegebene Bedeutung besitzt und PG für eine Schutzgruppe der intracyclischen freien NH-Funktion des Thieno[2,3-c]pyrazolkerns steht, und (i-b) einem Hydrazin (R3)(R4)N-NH(R5), worin R3, R4 und R5 die oben angegebene Bedeutung besitzen, in Gegenwart eines Kupplungsmittels und in Gegenwart einer Base wie eines tertiären Amins oder eines Alkalimetallcarbonats und dann
(ii) Abspaltung von PG.

15. Verfahren zur Herstellung eines Produkts der allgemeinen Formel (X) gemäß Anspruch 14, **dadurch gekennzeichnet, daß** es erhalten wird durch Verseifung der Esterfunktion des Thiophenkerns eines Produkts der allgemeinen Formel (IX): 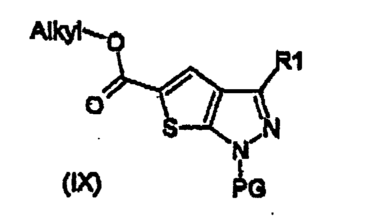 worin R1 die oben angegebene Bedeutung besitzt.

16. Verfahren zur Herstellung eines Produkts der allgemeinen Formel (IX) gemäß Anspruch 15, **dadurch gekennzeichnet, daß** es erhalten wird durch Kupplung zwischen:
(i) einem Produkt der folgenden allgemeinen Formel (IIa): 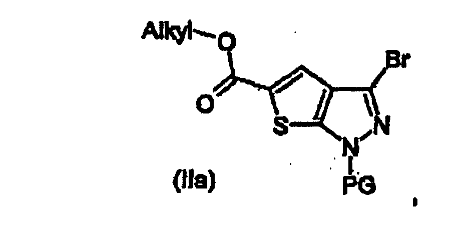 worin Alkyl die oben angegebene Bedeutung besitzt und PG für eine Schutzgruppe der intracyclischen freien NH-Funktion des Thieno[2,3-c]pyrazolkerns steht, und
(ii) einem Produkt der allgemeinen Formel (R2)CONH₂ in Gegenwart von:
- einem Katalysator wie Kupfer(I)-iodid,
- einem Amin wie *trans*-1,2-Diaminocyclohexan, trans-1,2-Bis(methylamino)cyclohexan oder vorzugsweise N,N'-Dimethyl-1,2-diaminoethan und
- einer Base wie Trikaliumphosphat oder Caesiumcarbonat.

17. Verfahren zur Herstellung eines Produkts der allgemeinen Formel (IIa) gemäß Anspruch 16, **dadurch gekennzeichnet, daß** es erhalten wird durch Umsetzung zwischen einem Mercaptoessigsäurealkylester Alkyl-OCO-CH₂-SH in Gegenwart einer Base wie Natriumcarbonat und einer Verbindung (IIIa): 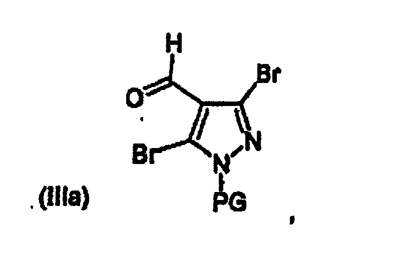 worin PG die oben angegebene Bedeutung besitzt.

18. Verfahren zur Herstellung eines Produkts der allgemeinen Formel (IIIa) gemäß Anspruch 17,
**dadurch gekennzeichnet, daß** es erhalten wird durch
(i) Formylierung von 3,4,5-Tribrompyrazol zu 3,5-Dibrom-4-formylpyrazol (III) und dann (ii) Schützung der intracyclischen Aminfunktion von (III) durch Einführung der Schutzgruppe PG.

19. Verfahren nach Anspruch 18, **dadurch gekennzeichnet, daß** man die Schutzgruppe PG einführt durch:
(i) Umsetzung mit Ethylvinylether in Gegenwart einer Säure wie Salzsäure zum Erhalt einer Gruppe PG = 1-Ethoxyethyl in einem inerten Lösungsmittel wie Toluol oder
(ii) Umsetzung mit Di-tert-butyldicarbonat in Gegenwart einer Base wie Triethylamin, Pyridin oder N,N-Dimethylaminopyridin zum Erhalt einer Gruppe PG = tert-Butyloxycarbonyl in einem inerten Lösungsmittel wie Dichlormethan.

20. Pharmazeutische Zusammensetzung, enthaltend ein Produkt nach einem der Ansprüche 1 bis 13 in Kombination mit einem pharmazeutisch annehmbaren Hilfsstoff.

21. Verwendung eines Produkts nach einem der Ansprüche 1 bis 13 zur Herstellung eines Arzneimittels, das zur Behandlung eines pathologischen Zustands, insbesondere Krebs, verwendet werden kann.

22. Verwendung eines Produkts nach einem der Ansprüche 1 bis 13 zur Herstellung eines Arzneimittels, das zur Behandlung eines pathologischen Zustands, der unter Psoriasis, Glaukom, Leukämien, mit dem Zentralnervensystem verbundenen Erkrankungen, Entzündungen und mit Deregulation der JNK-Proteine verbundenen Erkrankungen ausgewählt ist, verwendet werden kann.

23. Produkt, **dadurch gekennzeichnet, daß** es der folgenden allgemeinen Formel (XVI) entspricht: 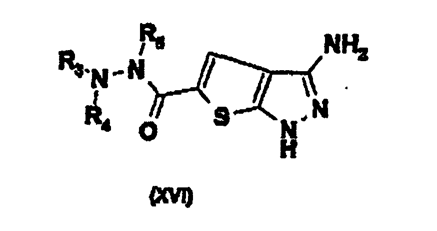 worin R3, R4 und R5 die in einem der Ansprüche 1 bis 7 angegebene Bedeutung besitzen.

24. Produkt, **dadurch gekennzeichnet, daß** es der folgenden allgemeinen Formel (XVII) entspricht: 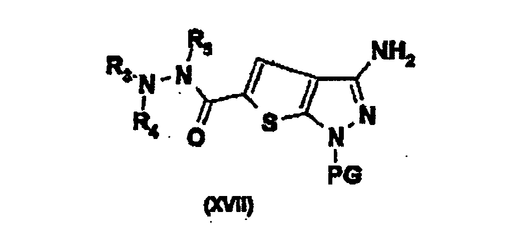 worin R3, R4 und R5 die in einem der Ansprüche 1 bis 7 angegebene Bedeutung besitzen und PG für eine Schutzgruppe steht.

25. Produkt, **dadurch gekennzeichnet, daß** es der folgenden allgemeinen Formel (XVIII) entspricht: 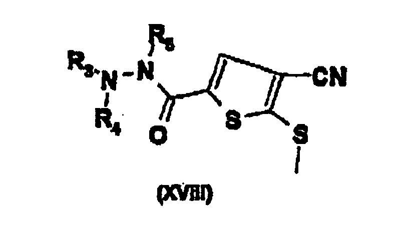 worin R3, R4 und R5 die in einem der Ansprüche 1 bis 7 angegebene Bedeutung besitzen.

26. Produkt, **dadurch gekennzeichnet, daß** es der folgenden allgemeinen Formel (XIX) entspricht: 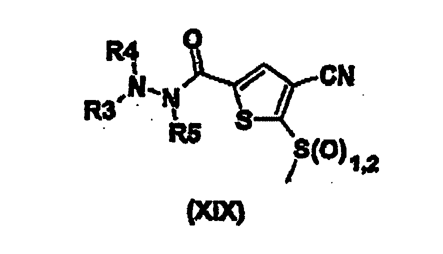 worin R3, R4 und R5 die in einem der Ansprüche 1 bis 7 angegebene Bedeutung besitzen.

27. Produkt, **dadurch gekennzeichnet, daß** es der folgenden allgemeinen Formel (XX) entspricht: 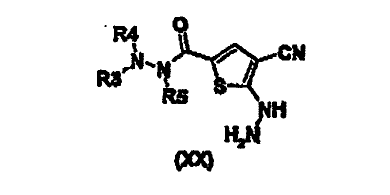 worin R3, R4 und R5 die in einem der Ansprüche 1 bis 7 angegebene Bedeutung besitzen.

28. Produkt, **dadurch gekennzeichnet, daß** es der folgenden allgemeinen Formel (IVb) entspricht: 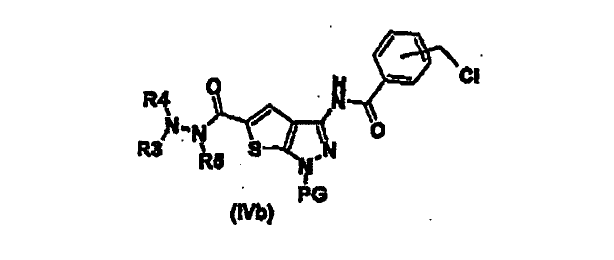 worin R3, R4 und R5 die in einem der Ansprüche 1 bis 7 angegebene Bedeutung besitzen und PG für eine Schutzgruppe steht.
